# EUROPEAN PATENT SPECIFICATION

(11) **EP 3 806 652 B1**
(45) Date of publication and mention of the grant of the patent: **04.11.2026**
(21) Application number: 19800382.4
(22) Date of filing: 08.05.2019
(51) Int. Cl.: A23K 10/33, A61K 36/899, A23K 20/111, A61K 31/192, A61K 31/352

(54) **SUGAR CANE EXTRACTS FOR USE IN ANIMAL FEEDS**
ZUCKERROHREXTRAKTE ZUR VERWENDUNG IN TIERFUTTERMITTELN
EXTRAITS DE CANNE À SUCRE DESTINÉS À ÊTRE UTILISÉS DANS DES ALIMENTS POUR ANIMAUX

(30) Priority: 11.05.2018 AU 2018901631
(43) Date of publication of application: 21.04.2021
(73) Proprietor: Poly Gain Pte Ltd, Singapore 408555 (SG)
(72) Inventor: MITCHELL, Shane, Keysborough, Victoria 3173 (AU); KITCHEN, Barry, Keysborough, Victoria 3173 (AU); MACNAB, Gregor, Keysborough, Victoria 3173 (AU); NEOH, Julian, Keysborough, Victoria 3173 (AU); FLAVEL, Matthew, Keysborough, Victoria 3173 (AU)
(74) Representative: Roukounas, Dimitrios
(86) International application number: PCT/AU2019/050422
(87) International publication number: WO 2019/213703

(56) References cited:
- WO-A1-2005/117608
- WO-A1-2006/128259
- WO-A1-2012/106761
- WO-A1-2015/021512
- WO-A1-2018/079641
- WO-A1-2018/079641
- WO-A1-2019/028506
- JP-A- 2003 063 975
- US-A1- 2003 147 978
- US-A1- 2016 263 177
- YANGCHUN XIA ET AL: "Effect of dietary supplementation with sugar cane extract on meat quality and oxidative stability in finishing pigs", ANIMAL NUTRITION, vol. 3, no. 3, 19 March 2017 (2017-03-19), pages 295 - 299, XP055651248, ISSN: 2405-6545, DOI: 10.1016/j.aninu.2017.05.002
- RAMLI, M.N. ET AL.: "Growth, Feed Efficiency, Behaviour, Carcass Characteristics and meat Quality of Goats Fed Fermented Bagasse Feed", ASIAM- AUST J. ANIMAL SCI, vol. 18, no. 11, 2005, pages 1594 - 1599, XP055651247
- XIA, Y. ET AL.: "Effect of Dietary supplementation with sugar Cane Extract on Meat Quality and Oxidative Stability in Finishing Pigs", ANIMAL NUTRITION, vol. 3, 2017, pages 295 - 299, XP055651248
- KOGE, K. ET AL.: "Antioxidants and Other Functional Extracts from Sugarcane", ASIAN FUNCTIONAL FOODS, XP055651257

## Description

### Technical Field

This disclosure relates to animal supplements and feeds comprising an extract derived from sugar cane, in particular, animal supplements and feeds comprising a polyphenolic extract derived from sugar cane. The disclosure also relates to animal supplements, animal feeds, methods and uses for improving or maintaining the health of animals to the benefit of improved food production and food quality.

Throughout this disclosure, various publications are referred to by first author and year of publication. Full citations for these publications, in the order they appear in the application, are presented in a References section immediately before the claims.

### Background

Antibiotic use has been a staple in animal production worldwide. Antibiotics have been used as additives or supplements in animal feed to not only control infections, but also to promote growth. Antibiotics are considered to aid animals in digesting food more efficiently, improving both the quality and yield of food products leading to economic benefits for farmers.

Antimicrobial resistance (AMR) is a natural process whereby microbes evolve to be able to resist the action of drugs, making them ineffective. This leads to antibiotics becoming less effective over time and in extreme cases, ultimately useless. AMR has increasingly become a problem because the pace at which new antibiotics are discovered has slowed dramatically and consequently there are a very limited number of new drugs. Meanwhile, antibiotic use has risen due in part to the adoption of intensive farming methods. AMR threatens the effective prevention and treatment of an everincreasing range of infections caused by bacteria, parasites, viruses and fungi and is becoming an increasingly serious threat to global public health. The wide and overuse of antibiotics has given rise to life-threatening "superbugs" that are resistant to several classes of antibiotics.

In a new "post-antibiotic era", major government agencies (European Union, US FDA, Australia's Department of Agriculture and Health) have responded to AMR by implementing directives and legislation to control the use of antibiotics in food. Major companies in the food industries, such as McDonalds and Wal-Mart, are proposing their own initiatives to reduce antibiotic use.

However, the phasing out of antibiotic use has the consequence that, without the use of growth promoting antibiotics, food production levels will drop resulting in greater economic burden for the farming industry. The reduction of antibiotics has also resulted in some animal diseases becoming more widespread and prevalent.

It is therefore important to increase and develop the armamentarium of agents that have the potential to act as alternatives to antibiotics. There is a need for medicated animal feeds that may be used to alleviate the problems associated with AMR and the reduction of antibiotics in the "post-antibiotic" era.

Extracts comprising polyphenolic compounds and their uses in feed and disclosed in e.g., US 2016/263177 A1; WO 2018/079641 A1; and Yangchun et al, Animal Nutrition 2017; 3 (3): 295-299.

### Summary

Sugar cane waste and sugar cane extracts can provide various benefits to human beings and animals: some sugar cane extracts containing phytochemicals may be used as nutritional supplements and other sugar cane extracts containing phytochemicals have the ability to lower the glycaemic index (GI) of foods and beverages. The present disclosure is based on the finding that a polyphenolic extract derived from sugar cane has surprising and favourable properties for use in improving or maintaining the health of animals.

In one aspect of the disclosure there is provided a non-human animal formulated supplement comprising an extract derived from sugar cane, the extract comprising from about 20 catechin equivalent (CE) g/L to about 50 CE g/L of polyphenols or from about 200 CE mg/g to about 500 CE mg/g of polyphenols.

In another aspect of the disclosure there is provided a non-human animal feed comprising the supplement as described herein.

In another aspect of the disclosure there is provided a non-therapeutic method for improving growth performance in a non-human animal subject, the method comprising the step of administering an effective amount of the supplement as described herein or the feed as described herein.

In another aspect of the disclosure there is provided a non-therapeutic method for reducing body fat content in a non-human animal subject, the method comprising the step of administering an effective amount of the supplement as described herein or the feed as described herein.

In another aspect of the disclosure there is provided a non-therapeutic method for improving nutrient digestibility in a non-human animal subject, the method comprising the step of administering to the subject an effective amount of the supplement as described herein or the feed as described herein.

In another aspect of the disclosure there is provided a non-therapeutic method for reducing feed conversion ratio (FCR) in a non-human animal subject, the method comprising administering to the subject an effective amount of the supplement as described herein or the feed as described herein.

In another aspect of the disclosure there is provided a non-therapeutic method for improving meat quality in a non-human animal subject, the method comprising administering to the subject an effective amount of the supplement as described herein or the feed as described herein.

In another aspect of the disclosure there is provided a non-therapeutic method for improving or maintaining muscle condition in a non-human animal subject, wherein the method comprises the step of administering an effective amount of the supplement as described herein or the feed as described herein.

In another aspect of the disclosure there is provided a non-therapeutic method for stimulating or sustaining appetite in a non-human animal subject, wherein the method comprises the step of administering an effective amount of the supplement as described herein or the feed as described herein.

### Brief Description of Drawings

Whilst it will be appreciated that a variety of embodiments of the disclosure may be utilised, in the following, a number of examples of the disclosure are described with reference to the following drawings/figures:
Figure 1 exhibits a process for the preparation of extracts derived from molasses.
Figure 2 exhibits a process for the preparation of extracts derived from dunder.
Figure 3 exhibits a process for the preparation of extracts derived from dunder and molasses.
Figure 4 exhibits base peak chromatograms (FTMS negative) of three extracts from molasses produced by the process of Figure 1 analysed by LCMS. A) resin bound sample, B) resin unbound sample, and C) 74 Brix sample.
Figure 5 exhibits a LC-MS spectrum of a representative extract derived from sugar cane molasses prepared according to Example 3.
Figure 6 exhibits LC-MS spectra for sugar cane dunder starting material (A) and an extract of sugar cane dunder according to the present invention (B).
Figure 7 exhibits a representative binding curve for an extract derived from sugar cane of the disclosure against nuclear factor κB (NF-KB).
Figure 8 exhibits a representative response curve for an extract derived from sugar cane of the disclosure against nuclear factor erythroid 2-related factor (Nrf2).
Figure 9 exhibits a representative binding curve for an extract derived from sugar cane of the disclosure against tumor necrosis factor (TNF-α).
Figure 10 exhibits a representative inhibition curve for an extract derived from sugar cane of the disclosure against prostaglandin E2 (PGE 2).
Figures 11 A and B exhibit representative inhibition curves for an extract derived from sugar cane of the disclosure against cyclooxygenase-1 (COX-1) and cyclooxygenase-2 (COX-2).
Figure 12 exhibits the average growth chart by weight (g) for the pangus studied in Example 13.
Figure 13 exhibits the average growth chart by length (cm) for the pangus studied in Example 13.
Figure 14 exhibits average weight gain chart (g) for the pangus studied in Example 13.
Figure 15 exhibits the average length gain chart (cm) for the pangus studied in Example 13.
Figure 16 exhibits photographs of the pangus studied in Example 13 by treatment group: A. T₀ - control group; B. T₁ - treatment group (0.2% extract); C. T₂ - treatment group (0.4% extract); D. T₃ - treatment group (0.6% extract).
Figure 17 exhibits a size comparison between the T₀, T₁, T₂ and T₃ treatment groups for the pangus studied in Example 13.
Figure 18 exhibits the average growth chart by weight (g) for the tilapia studied in Example 13.
Figure 19 exhibits average growth chart by length (cm) for the tilapia studied in Example 13.
Figure 20 exhibits average weight gain chart (g) for the tilapia studied in Example 13.
Figure 21 exhibits the average growth chart by length (cm) for the tilapia studied in Example 13.
Figure 22 exhibits photographs of the tilapia studied in Example 13 by treatment group: A. T₀ - control group; B. T₁ - treatment group (0.2% extract); C. T₂ - treatment group (0.4% extract); D. T₃ - treatment group (0.6% extract).
Figure 23 exhibits a size comparison between the T₀, T₁, T₂ and T₃ treatment groups for the tilapia studied in Example 13.
Figure 24 exhibits the average growth chart by weight (g) for the prawn studied in Example 13.
Figure 25 exhibits the average growth chart by length (cm) for the prawn studied in Example 13.
Figure 26 exhibits the average weight gain chart (g) for the prawn studied in Example 13. The chart shows bar graphs depicting the average weight gain for the T₀, T₁, T₂ and T₃ treatment groups: the left bar graph within a treatment group is the average weight gain (sampling 9) and the right bar graph within a treatment group is the average weight gain (sampling 10).
Figure 27 exhibits the average length gain chart (cm) for the prawn studied in Example 13.
Figure 28 exhibits photographs of the prawn studied in Example 13 by treatment group: A. T₀ - control group; B. T₁ - treatment group (0.2% extract); C. T₂ - treatment group (0.4% extract); D. T₃ - treatment group (0.6% extract).
Figure 29 exhibits a size comparison between the T₀, T₁, T₂ and T₃ treatment groups for the prawn studied in Example 13.
Figure 30 exhibits graphs of the average daily gain for the chicken study of Example 14: A. days 10-17 (starter period) and B. days 17-24 (grower period).
Figure 31 exhibits graphs of weight gain for the chicken study of Example 14: A. average daily gain over days 24-38 and B. body weight gain at day 38; and at C. a graph of average daily feed intake over days 24-38.
Figure 32 exhibits the structure of the treatment groups in the cat study of Example 15. Compound X refers to a sugar cane extract of Example 3.
Figure 33 exhibits the effects of an extract of the present disclosure on blood health parameters observed in the cat study of Example 9. Abbreviations: MCH (mean corpuscular haemoglobin); MCHC (mean corpuscular haemoglobin concentration); HCT (haematocrit). The left hand side bar of the bar graphs relates to data for the control whereas the right hand side bar relates to data for the sugar cane extract of Example 3.
Figure 34 exhibits the effects of an extract of the present disclosure (extract of Example 3) on further blood health parameters observed in the cat study of Example 15. Abbreviation: Seg Neut (segmented neutrophil). The left hand side bar of the bar graphs relates to data for the Control whereas the right hand side bar relates to data for the sugar cane extract of Example 3 (referred to as "Compound" in the Figure).
Figure 35 exhibits the effect of an extract of the present disclosure (extract of Example 3) on urinary health parameters observed in the cat study of Example 15: y-axis values are the reference (normal) range for each component. Values are reported as means and SED. The left hand side bar of the bar graphs relates to data for the Control whereas the right hand side bar relates to data for the sugar cane extract of Example 3 (referred to as "Compound" in the Figure).
Figure 36 exhibits the effect of an extract of the present disclosure (extract of Example 3) on the digestibility of macronutrients observed in the cat study of Example 15. Values are reported as means and SED. The left hand side bar of the bar graphs relates to data for the Control whereas the right hand side bar relates to data for the sugar cane extract of Example 3 (referred to as "Compound" in the Figure).
Figure 37 exhibits the effect of an extract of the present disclosure (extract of Example 3) on A. body composition and B. bodyweight observed in the cat study of Example 15 over 30 weeks where Panel A exhibits lean mass (kg) and Panel B exhibits body weight (kg). Body composition was determined using deuterated water injection (D₂O) or dual energy X-ray absorptiometry (DEXA). Values are reported as means and SED. The left hand side bar of the bar graphs relates to data for the Control whereas the right hand side bar relates to data for the sugar cane extract of Example 3 (referred to as "Compound" in the Figure).
Figure 38 exhibits a graph of a cross-over trial for the cat study of Example 9. C-C denotes a control diet administered over 31 weeks; C-X denotes a control diet for 18 weeks that is then crossed over to a sugar cane extract diet for the remainder of the trial; X-C denotes a sugar cane extract diet for 18 weeks that is then crossed over to a control diet for the remainder of the trial; X-X denotes a sugar cane extract diet administered over 31 weeks.
Figure 39 exhibits the effect of an extract of the present disclosure (extract of Example 3) on body fat composition observed in the cat study of Example 15 where Panel A exhibits % fat and Panel B exhibits fat mass (kg). Body composition was determined using deuterated water injection (D₂O) or dual energy X-ray absorptiometry (DEXA). Values are reported as means and SED. The left hand side bar of the bar graphs relates to data for the Control whereas the right hand side bar relates to data for the sugar cane extract of Example 3 (referred to as "Compound" in the Figure).
Figure 40 exhibits a line graph of energy intake over 18 weeks for the cat study of Example 15. The upper line marked "C" shows the trend for a control diet; the lower line marked "X" shows the trend for a sugar cane extract diet based on the extract of Example 3.
Figure 41 exhibits % fat body content as measured by DEXA for the cat study of Example 15. A line graph is presented showing the trend in % fat body content over 18 weeks.. The upper line "C" shows the trend for a control diet; the lower line "X" shows the trend for a sugar cane extract diet based on the extract of Example 3.
Figure 42 exhibits before (Panel A) and after (Panel B) photographs for Horse A of Example 11.
Figure 43 exhibits before and after photographs for Horse B of Example 11.
Figure 44 exhibits before and after photographs for Horse C of Example 11.
Figure 45 exhibits before and after photographs for Horse D of Example 11.
Figure 46 exhibits before and after photographs for Horse E of Example 11.
Figure 47 exhibits a graph showing an overview of temperature conditions and base diets used in Example 18.
Figure 48 exhibits a graph of body weight (g) of broilers of Example 18 with an increasing sugar cane extract diet, based on the extract of Example 3, with dotted lines showing the positive trends.
Figure 49 exhibits a graph of feed conversion ratio (FCR) with increasing sugar cane extract diet, based on the extract of Example 3, with dotted lines showing the negative trends.
Figure 50 exhibits a graph of Warner Bratzler Shear Force (WBSF, kg/cm²) with sugar cane extract diet based on the extract of Example 3 inclusion at 0, 2, 4, 6 and 10 g/kg.
Figure 51a exhibits a graph of Thiobarbituric acid reactive substances (TBARS) assay with sugar cane extract diet based on the extract of Example 3 inclusion at 0, 2, 4, 6 and 10 g/kg at 24 hour and 72 hour time periods in the thermoneutral (TN) group of broilers.
Figure 51b exhibits a graph of Thiobarbituric acid reactive substances (TBARS) assay with sugar cane extract diet based on the extract of Example 3 inclusion at 0, 2, 4, 6 and 10 g/kg at 24 hour and 72 hour time periods in the heat stress (HS) group of broilers.

### Description of Embodiments

### Definitions

Unless specifically defined otherwise, all technical and scientific terms used herein shall be taken to have the same meaning as commonly understood by one of ordinary skill in the art (e.g., chemistry, biochemistry, formulation science, food and nutritional science, animal science and animal husbandry, cell culture, and molecular biology). Further, unless otherwise required by context, singular terms shall include pluralities and plural terms shall include the singular. Thus, as used in this specification and the appended claims, the singular forms "a", "an" and "the" include plural referents unless the context clearly indicates otherwise. Thus, the term "an animal subject" means "one or more animal subjects" unless the context clearly indicates otherwise.

The term "about" as used herein refers to a range of +/-5% of the specified value.

"Administering" as used herein is to be construed broadly and includes administering an extract or animal supplement or animal feed as described herein to an animal subject. The term encompasses the normal consumption of food and water by the animal subject and oral administration (including buccal or sublingual). The term "administering" as used herein also encompasses administration by nasal administration.

The term "animal feed" as used herein refers to any compound, preparation, or mixture suitable for, or intended for intake by an animal.

"Animal supplement" as used herein refers to a substance which is added to the feed for purposes including but not limited to enhancing the digestibility of the feed, completing the nutritional value of the feed, improving or maintaining the health of the recipient such as improving the immune defence or improving or maintaining gastrointestinal health.

The term "animal subject" as used herein refers to any animal except humans. Thus, the disclosure relates to non-human animals. The non-human animals may be mammals. Examples of non-human animals are aquatic animals, insects, amphibians, reptiles, gastropods, birds, monogastric animals, ruminants and pseudo-ruminants.

The term "aquatic animal(s)" as used herein includes fish including but not limited to finfish and shellfish. Finfish include but are not limited to pangus and tilapia. Further examples of finfish are barramundi, bass, bream, carp, catfish, cod, crappie, drum, eel, goby, goldfish, grouper, halibut, java, labeo, lai, loach, mackerel, milkfish, mojarra, mudfish, mullet, paco, pearlspot, pejerrey, perch, pike, pompano, roach, salmon, sampa, sauger, sea bass, seabream, shiner, sleeper, snakehead, snapper, snook, sole, spinefoot, sturgeon, sunfish, sweetfish, tench, terror, trout, tuna, turbot, vendace, walleye and whitefish. Shellfish include but not limited to a crustacean (e.g. crabs, crayfish, lobsters, prawns and shrimp) and a mollusc (e.g. clams, mussels, oysters, scallops and winkles).

Insects include, for example, cicadas, grasshoppers, beetles, bees, wasps, butterflies, moths, ants, flies, crickets, aphids, bugs and dragonflies.

Amphibians include, for example, frogs, toads and salamanders.

Reptiles include, for example, snakes, lizards, iguanas, turtles and crocodiles.

Gastropods include, for example, snails and slugs, including sea snails and sea slugs, as well as freshwater snails, freshwater limpets, land snails and land slugs.

Birds include, for example, poultry such as chickens, ducks, geese, turkeys, quail, guinea fowl, pigeons (including squabs) and birds of prey (including hawks, eagles, kites, falcons, vultures, harriers, ospreys, and owls). Chickens include, for example, broiler chickens (broilers), chicks, roosters and layer hens (layers).

Monogastric animals include but not limited to pigs or swine, such as piglets, growing pigs and sows, cats and dogs, rodents (rats, mice).

Ruminant animals include, for example, animals such as cattle, sheep, goats, deer, yak, camel, llama and kangaroo. Cattle include but are not limited to beef cattle, dairy cattle, cows and young calves.

Pseudo-ruminant animals include, for example, horses, camels, rabbits and guinea pigs.

The term "animal subject" encompasses companion animals and food-producing animals as defined herein and aquarium and zoo animals.

As used herein, the term "composition" is intended to encompass a product comprising the specified ingredients, as well as any product which results, directly or indirectly, from combination of the specified ingredients.

As used herein the terms "improvement", "improve", "improving", "treatment", "treat", "treating" and the like refer to the control, healing or amelioration of a disease, disorder or condition, or a decrease in the rate of advancement of a disease, disorder or condition, or defending against or inhibiting a symptom or side effect, reducing the severity of the development of a symptom or side effect, and/or reducing the number or type of symptoms or side effects suffered by an animal subject, as compared to not administering a veterinary composition, animal supplement or animal feed comprising an extract derived from sugar cane of the present disclosure. The term "ameliorate" encompasses relieving of adverse symptoms, inducing a state of comfort or wellbeing or removing or reducing biochemical, physiological or clinical markers of the condition, disease or disorder. As used herein, the terms "prevention", "prevent", "preventing" and the like refer to avoiding, delaying, reducing or slowing down the onset of a specified condition, disease or disorder or to avoid at least one symptom or side effect of the condition, disease or disorder. As would be understood by those skilled in the art, the term "preventing" includes that, for example, anemia is completely prevented, however, it does not necessarily mean that the anemia is completely prevented. Likewise, it would be recognised that the term "improvement" or "treatment" includes that, for example, anemia is cured, however, it does not necessarily mean that the anemia is completely cured. The terms "maintenance", "maintain", "maintaining" and the like when used in the phrase "maintenance, maintain, maintaining [of] gastrointestinal health" refer to causing or enabling gastrointestinal health to continue whereby gastrointestinal health is retained.

With regard to "improvement", "maintenance", "prevention" and "treatment", the term "effective amount", as used herein, refers to an amount (of a sugar cane extract or a composition, a non-human animal formulated supplement or a non-human animal feed comprising that extract) when administered to an animal which is sufficient to elicit the biological or medical response of a tissue, system, animal or human that is being sought by a practitioner in the field of animal husbandry e.g. a farmer, researcher or veterinarian. Undesirable effects, e.g. side effects, are sometimes manifested along with the desired effect; hence, a practitioner balances the potential benefits against the potential risks in determining what an appropriate "effective amount" is. The exact "effective amount" required varies from subject to subject, depending on the species, age and general condition of the subject, mode of administration, severity of the disease and the like. Thus, it may not be possible to specify an exact "effective amount". However, an appropriate "effective amount" in any individual case may be determined by one of ordinary skill in the art using routine experimentation. The terms "improvement", "maintenance", "prevention" and "treatment" encompass use in a palliative setting.

As used herein the term Feed Conversion Ratio (FCR) refers to a measure of an animal's efficiency in converting feed mass into increases of the desired output. For food-producing animals which are farmed for meat *e.g.* fish, poultry, cattle and swine the output is the mass gained by the animal. Specifically, unless otherwise explicitly stated in the disclosure, FCR is calculated as feed intake divided by weight gain, all over a specified period. Improvement in FCR means reduction of the FCR value. A FCR improvement of 2% means that the FCR was reduced by 2%.

The term "fiber" as used herein refers to indigestible portion of food derived from plants. The fiber may be soluble or insoluble fiber. Non-limiting examples of fiber include, sugar cane fiber, oat bran, flour (including, for example, soy, rice, wheat, bran, rye, corn, sorghum, potato), modified starch, gelatin, non-starch polysaccharides such as arabinoxylans, cellulose, and many other plant components such as resistant starch, resistant dextrins, inulin, lignin, chitins, pectins, beta-glucans, and oligosaccharides.

The term "food producing animal" as used herein refers to an animal that is farmed for the production of food for consumption by another animal, for example, a human. It would be understood that the term "food-producing animal" encompasses a food producing animal that is an aquatic animal; a food producing animal that is a bird; a food producing animal that is a monogastric animal; a food producing animal that is a ruminant; and a food producing animal that is a pseudo ruminant. It would be understood that the term "food producing animal" includes, for example, finfish, shellfish, poultry, such as chickens, geese and turkeys, pigs, cattle, sheep, goats and horses.

The term "growth performance" as used herein refers to the response of an animal subject to an extract derived from sugar cane, animal supplement or animal feed of the present disclosure. Growth performance may be assessed by methods well known in the art and may be characterised by any one or more of the following: feed conversion ratio, feed intake, weight gain, gain in size e.g. gain in length. It may also be characterised by food production including meat yield or milk yield.

It would be appreciated that the size of an animal may be measured with respect to any physical dimension such as body length, width, thickness and circumference and head length, width, thickness and circumference. It would be appreciated that such measurements have been standardized to facilitate comparison between, for example, different animals of the same species. As an illustration, in measuring finfish "standard length" as used herein refers to a measurement from the snout of the finfish to the last vertebrate. Where it is difficult to identify the last vertebrate an alternative measurement may be used. In this regard, "fork length" refers to a measurement from the snout to the intersection of the caudal tail fins.

The term "CE", or "catechin equivalent" and the term "GAE", or "gallic acid equivalent" as used herein are measures of total polyphenolic content. The term "CE", or "catechin equivalent" as used herein is expressed as mg catechin equivalents/g crude material or g catechin equivalents/L crude material. The term "GAE", or "gallic acid equivalent" as used herein is expressed as mg gallic acid equivalents/g extract derived from sugar cane or g gallic acid equivalents/L extract derived from sugar cane. As a measure of total polyphenolic content, the terms "CE", "catechin equivalent", "GAE" and "gallic acid equivalent" are equivalent and are used interchangeably herein.

The term "free amino acids" as used herein refers to amino acids which are singular molecules and structurally not attached to peptide bonds which are attached to other amino acids.

The term "sugar cane derived product" as used herein refers to products of the sugar cane milling and refining processes including, but not limited to, sugar, molasses, massecuite, bagasse, first expressed juice, mill mud, clarified sugar cane juice, clarified syrup, treacle, golden syrup, field trash, cane strippings, leaves, growing tips, pulp and dunder and combinations thereof. Dunder is the residue produced when a product such as sugar or molasses is fermented to give, for example, ethanol. Sugar cane dunder is also referred to as biodunder, stillage or vinasse. As used herein , the terms "dunder", "bio-dunder", "stillage" and "vinasse" are equivalent and used interchangeably.

The terms "an extract derived from sugar cane of the (present) disclosure", "an extract of the (present) disclosure", "a sugar cane extract of the (present) disclosure", "sugar cane extract" and "the extract" are used interchangeably herein.

Throughout this specification the word "comprise", or variations such as "comprises" or "comprising", will be understood to imply the inclusion of a stated element, integer or step, or group of elements, integers or steps, but not the exclusion of any other element, integer or step, or group of elements, integers or steps.

### General Techniques

### Exemplary process for producing extracts derived from sugar cane

A suitable process for producing the extract derived from sugar cane may be determined by a skilled person.

### Feedstock for the extraction process

After being mechanically harvested, sugar cane is transported to a mill and crushed between serrated rollers. The crushed sugar cane is then pressed to extract raw sugar juice and leaves fibrous material known as bagasse (typically used as fuel). The raw juice is then heated to its boiling point to extract any impurities, then lime and bleaching agents are added and mill mud is removed. The raw juice is further heated under vacuum to concentrate and increase the Brix value. The concentrated syrup is seeded to produce bulk sugar crystals and a thick syrup known as molasses. The two are separated by a centrifuge and typically the molasses waste stream is collected for use as a low-grade animal feedstock.

The extracts produced according to the process of the disclosure may be extracts of sugar cane or extracts from any sugar cane derived product, including those produced during the sugar cane milling process, the sugar cane refining process and other processes using sugar cane products.

As defined above, the term "sugar cane derived product" refers to products of the sugar cane milling and refining processes including, but not limited to, molasses, massecuite, bagasse, first expressed juice, mill mud, clarified sugar cane juice, clarified syrup, treacle, golden syrup, field trash, cane strippings, leaves, growing tips, pulp and dunder and combinations thereof. In one embodiment, the sugar cane derived product is molasses or dunder. In one embodiment, the sugar cane derived product is a combination of molasses and dunder. In another embodiment, the sugar cane derived product is molasses. In another embodiment, the sugar cane derived product is massecuite. In another embodiment, the sugar cane derived product is dunder. In another embodiment, the sugar cane derived product is a combination of molasses and dunder. In another embodiment, the sugar cane derived product is bagasse. In another embodiment, the sugar cane derived product is first expressed juice. In another embodiment, the sugar cane derived product is mill mud. In another embodiment, the sugar cane derived product is clarified sugar cane juice. In another embodiment, the sugar cane derived product is clarified syrup. In another embodiment, the sugar cane derived product is treacle. In another embodiment, the sugar cane derived product is golden syrup. In another embodiment, the sugar cane derived product is field trash. In another embodiment, the sugar cane derived product is cane strippings. In another embodiment, the sugar cane derived product is leaves. In another embodiment, the sugar cane derived product is growing tips. In another embodiment, the sugar cane derived product is pulp.

Sugar cane derived products generally comprise complex mixtures of substances including, but not limited to, polyphenols, phytosterols, monosaccharides, disaccharides, oligosaccharides, polysaccharides, organic acids, amino acids, peptides, proteins, vitamins, and minerals.

As would be understood by a skilled person, polyphenols are compounds characterized by the presence of multiple phenol structural units. Polyphenols may be classified into sub-groups by their chemical structure. Examples of sub-groups of polyphenols include, but are not limited to, flavonoids (including flavones, flavanols, flavonols), hydroxybenzoic acids, hydroxycinamic acids, catechins, proanthocyanidins, anthocyanidins, stilbenes, lignans, and phenolic acids. The polyphenols of sugar cane derived products also include conjugates such as, for example, glycosides, glucosides, galactosides, galacturonides, ethers, esters, arabinosides, sulphates, phosphates, aldopentoses (xylose, arabinose) and aldohexoses.

An exemplary process for producing an extract according to the disclosure is provided below. This exemplary process involves an extraction step. This exemplary process with molasses as the sugar cane derived product is depicted in Figure 1.

In one process for producing extracts of the disclosure, the sugar cane derived product is used as a feedstock and mixed with a suitable solvent such as ethanol to form an extraction mixture.

The skilled person will understand that in order to facilitate mixing of the sugar cane derived product with a suitable solvent such as ethanol, the sugar cane derived product may need to be mixed with a liquid, for example but not limited to water, and/or heated in order to achieve a desired viscosity. In one embodiment of the disclosure in which the sugar cane derived product is molasses, for example, the molasses may be mixed with a liquid, for example, water to achieve a desired viscosity. The sugar cane derived product, either mixed with a liquid or not, may be heated to decrease viscosity.

For sugar cane derived products comprising solid material such as bagasse, field trash and cane shippings, it is desirable that the product is first blended or homogenised with a liquid, for example but not limited to water, prior to mixing with ethanol to form the extraction mixture. The amount of a liquid with which the sugar cane derived product is blended or homogenised can be readily determined by the skilled person in order to achieve a sugar cane derived product having a suitable viscosity for mixing with ethanol to form an extraction mixture.

In one embodiment, the sugar cane derived product will have a viscosity less than or equal to about 100 centipoise. In another embodiment, the sugar cane derived product will have a viscosity of between about 50 to about 100 centipoise. In another embodiment, the sugar cane derived product will have a viscosity of between about 50 to about 80 centipoise.

The high viscosity of molasses is as a result of the high total solids (particularly soluble carbohydrates) and this is typically measured by determination of Brix degrees. In one embodiment, the sugar cane derived product may have about 10° to about 80° Brix. In another embodiment, the sugar cane derived product may have about 20° to about 70° Brix. In another embodiment, the sugar cane derived product may have about 20° to about 50° Brix. In another embodiment, the sugar cane derived product may have about 30° to about 60° Brix. In another embodiment, the sugar cane derived product may have about 40° to about 50° Brix.

To extract compounds such as polyphenols, the sugar cane derived product is mixed with ethanol to form an extraction mixture. In one embodiment, the extraction mixture comprises at least about 50% v/v ethanol. In another embodiment, the extraction mixture comprises at least about 75%, 76%, 77%, 78%, 79%, 80%, 81%, 82%, 83%, 84% or 85% v/v ethanol.

The optimal concentration of ethanol in the extraction mixture for removing colour in the supernatant while minimising reduction in polyphenols is about 70% to about 85% v/v. In one embodiment, the extraction mixture comprises about 65% to about 75% v/v ethanol. In one embodiment, the extraction mixture comprises about 70% to about 80% v/v ethanol. In one embodiment, the extraction mixture comprises about 70% to about 75% v/v ethanol. In one embodiment, the extraction mixture comprises about 75% to about 80% v/v ethanol. In one embodiment, the extraction mixture comprises about 80% to about 85% v/v ethanol. In one embodiment, the extraction mixture comprises about 80% to about 83% v/v ethanol. In one embodiment, the extraction mixture comprises about 65% v/v ethanol. In another embodiment, the extraction mixture comprises about 70% v/v ethanol. In another embodiment, the extraction mixture comprises about 75% v/v ethanol. In another embodiment, the extraction mixture comprises about 80% v/v ethanol. In another embodiment, the extraction mixture comprises about 83% v/v ethanol. In another embodiment, the extraction mixture comprises about 85% v/v ethanol.

In the process of the disclosure, it may be desirable that pH extremes be avoided in the extraction mixture as they can have a deleterious effect on the components of the extraction mixture. Accordingly, in one embodiment the extraction mixture has a pH of about pH 4 to about pH 7.5. In another embodiment, the extraction mixture has a pH of about pH 4 to about pH 6. In another embodiment, the extraction mixture has a pH of about pH 4 to about pH 5.

Following the formation of precipitate in the extraction mixture, the precipitate may be removed from the mixture by any suitable method known in the art. For example the precipitate may be removed by centrifugation and the supernatant may be obtained. Alternatively, the precipitate may be allowed to settle for a time sufficient to allow the supernatant to be obtained while leaving precipitate behind, such as, for example, by sedimentation under gravity. The skilled person will understand that other techniques such as filtration can be used alone or in combination with centrifugation or sedimentation in order to produce the extract derived from sugar cane.

Once the supernatant has been obtained the ethanol is removed using techniques known in the art. By way of non-limiting example, the ethanol may be removed from the supernatant by evaporation, such as by using a rotary evaporator with a heating bath at approximately 45° C or higher. In some instances it may be desirable to further remove water from the supernatant to increase the Brix value of the supernatant. In one embodiment the process provides an extract having at least about 60°Bx (degrees Brix). In some instances the Bx value of the extract derived from sugar cane is at least about 65°Bx. In some instances the Bx value of the extract derived from sugar cane is at least about 70°Bx. In some instances the Bx value of the extract derived from sugar cane is about 60 - 65 °Bx. In some instances the Bx value of the extract derived from sugar cane is about 65 - 70 °Bx. In some instances the Bx value of the extract derived from sugar cane is about 64 - 65 °Bx. In some instances the Bx value of the extract derived from sugar cane is about 70 - 75 °Bx.

In one embodiment of the process of the disclosure, the supernatant comprising ethanol, or the extract derived from sugar cane from which ethanol has been removed may be used without further processing. Optionally the supernatant comprising ethanol, or the extract derived from sugar cane from which ethanol has been removed may be subjected to purification or fractionation.

A purification step may remove impurities, such as pigments that contribute to the colour of the extract derived from sugar cane. By way of non-limiting example, the supernatant or the extract derived from sugar cane may be subject to a purification step which includes, one or more or of, membrane filtration, size exclusion chromatography, ion exchange chromatography, and/or hydrophobic interaction chromatography. In one embodiment, the supernatant or extract may be subjected to hydrophobic interaction chromatography.

There are several techniques known in the art for separating compounds based on size. For example, it is known in the art that components of a supernatant or extract falling within a specific molecular weight range may be separated by size exclusion processing methods such as gel permeation chromatography or ultrafiltration.

Separation of components in the supernatant and/or the extract derived from sugar cane may also be achieved using chromatographic techniques or combinations of techniques. In one embodiment, chromatographic techniques include, but are not limited to, ion exchange chromatography, hydrophobic interaction chromatography, liquid chromatography-mass spectrometry (LCMS) and/or HPLC. Appropriate stationary and mobile phases of any chromatographic technique used will be readily determined by a skilled person. Appropriate elution techniques will also be readily determined by a skilled person. Chromatographic techniques may utilise fractional elution by stepwise increase in pH or with suitable solvents.

In one embodiment, the supernatant and/or the extract derived from sugar cane is subjected to one or more chromatographic techniques. In one embodiment, the supernatant and/or the extract derived from sugar cane is subjected to hydrophobic interaction chromatography. In one embodiment, the supernatant and/or the extract derived from sugar cane is subjected to hydrophobic interaction chromatography with an sephadex LH-20, XAD or FPX66 resin. In one embodiment, the supernatant and/or the extract derived from sugar cane is subjected to sephadex LH-20 resin. In one embodiment, the supernatant and/or the extract derived from sugar cane is subjected to XAD resin. In one embodiment, the supernatant and/or the extract derived from sugar cane is subjected to FPX66 resin.

The supernatant and/or the extract derived from sugar cane may also be processed by standard techniques such as, but not limited to, microfiltration, reverse osmosis, gel permeation, vacuum evaporation and freeze drying, spray drying and/or tunnel drying.

Another exemplary process for producing an extract according to the disclosure is provided below. This exemplary process involves multiple filtration steps. This exemplary process with dunder as the sugar cane derived product is depicted in Figure 2.

Sugar cane dunder is allowed to settled overnight (typically eight hours) in a V - bottom tank. The supernatant is then subjected to a number of filtration steps. The skilled person will understand that a variety of filtration steps (such as, for example, microfiltration or ultrafiltration) may be performed and the appropriate filtration steps will be readily determined by the skilled person.

In one embodiment, the supernatant is subjected to sequential microfiltration. In one embodiment the supernatant is sequentially filtered through: (i) a 5 micron filter; (ii) a 1 micron filter; (iii) a 0.5 micron filter; and (iv) a 0.1 micron filter. The skilled person would understand that a variety of filters could be used in the process to remove the desired sediment and undissolved matter. Exemplary filters are stainless steel filters, ceramic filters and cellulose filters.

The filtered supernatant is subsequently concentrated to remove water providing the extract. Any method for removing the water may be employed, including for example, heat exchange and evaporation. In one embodiment, the filtered supernatant is concentrated in a heat exchanger to remove water until the desired Brix level of the extract is achieved. In one embodiment, the process provides an extract having at least about 40°Bx. In one embodiment, the Bx value of the extract is at least about 50°Bx. In one embodiment, the Bx value of the extract is at least about 55°Bx. In one embodiment, the Bx value of the extract is at least about 60°Bx. In one embodiment, the Bx value of the extract is at least about 70°Bx. In one embodiment, the Bx value of the extract is about 45 - 55 °Bx. In one embodiment, the Bx value of the extract is about 50 °Bx. In one embodiment, the Bx value of the extract is about 50 - 55 °Bx. In one embodiment, the Bx value of the extract is about 55 - 60 °Bx. In one embodiment, the Bx value of the extract is about 50 - 70 °Bx. Another exemplary process for producing an extract according to the disclosure is provided below. This exemplary process with a combination of dunder and molasses as the sugar cane derived product is depicted in Figure 3.

Sugar cane mill molasses is mixed with settled sugar cane dunder (as described above) and stirred well to provide a mixture with the desired Brix level. The skilled person will understand that in order to facilitate mixing of the molasses and dunder, a liquid, for example but not limited to water, may be added. The liquid may be added to the molasses and/or the dunder prior to combining the two or the liquid may be added to the combined molasses and dunder. Additionally, heat may be applied to achieve a desired viscosity. In one embodiment, the combined mixture of molasses and dunder is about 50 - 55 °Bx. In one embodiment, the combined mixture of molasses and dunder is about 50 °Bx. In one embodiment, the combined mixture of molasses and dunder is about 55 °Bx. In one embodiment, the combined mixture of molasses and dunder is at least about 50 °Bx. In one embodiment, the combined mixture of molasses and dunder is at least about 60 °Bx. In one embodiment, the combined mixture of molasses and dunder is at least about 70 °Bx.

The combined mixture of molasses and dunder is maintained at a constant temperature (for example between 20-25 °C) and ethanol (for example 95% food grade ethanol) is added and stirred to ensure that the ethanol is evenly and quickly dispersed. Ethanol is added until the desired ethanol level is reached. The desired ethanol content can be from about 50% v/v to about 90% v/v. The desired ethanol content can be about 50, 51, 52, 53, 54, 55, 56, 57, 58, 59, 60, 61, 62,63, 64, 65, 66, 67, 68, 69, 70, 71, 72, 73, 74, 75, 76, 77, 78, 79, 80, 81, 82, 83, 84, 85, 86, 87, 88, 89, 90 % v/v. In one embodiment, the desired ethanol level is at least about 60% v/v. In one embodiment, the desired ethanol level is at least about 70% v/v. In one embodiment, the desired ethanol level is at least about 80% v/v. In one embodiment, the desired ethanol level is about 60 - 70% v/v. In one embodiment, the desired ethanol level is about 70 - 80% v/v. In one embodiment, the desired ethanol level is about 75% v/v. In one embodiment, the desired ethanol level is about 76% v/v

The addition and mixing of ethanol may lead to the formation of a gelatinous precipitate. The precipitate in the mixture is allowed to settle and the supernatant is removed, by, for example decantation and/or filtration. In one embodiment, the supernatant is decanted. In one embodiment, the supernatant is filtered. In one embodiment, the supernatant is decanted and filtered.

The ethanol is removed from the supernatant to provide the extract. Any method for removing the ethanol may be employed, including for example, heat exchange and evaporation. In one embodiment, the ethanol is removed by evaporation until the desired Brix level of the extract is achieved. In one embodiment, the process provides an extract having at least about 50°Bx. In one embodiment, the Bx value of the extract is at least about 60°Bx. In one embodiment, the Bx value of the extract is at least about 70°Bx. In one embodiment, the Bx value of the extract is at least about 80°Bx. In one embodiment, the Bx value of the extract is about 50 - 60 °Bx. In one embodiment, the Bx value of the extract is about 60 - 70 °Bx. In one embodiment, the Bx value of the extract is about 70 - 80 °Bx. In one embodiment, the Bx value of the extract is about 65 - 75°Bx. In one embodiment, the Bx value of the extract is about 75°Bx. In one embodiment, the Bx value of the extract is about 70°Bx.

### Extracts derived from sugar cane

As described above, extracts derived from sugar cane generally comprise complex mixtures of substances including, but not limited to, polyphenols, phytosterols, oligosaccharides, polysaccharides, monosaccharide, disaccharides, organic acids, amino acids, peptides, proteins, vitamins, and minerals.

In one embodiment, the extract derived from sugar cane of the present disclosure comprises at least about 20 g/L of polyphenols or at least about 200 mg CE/g of polyphenols. As explained above, the term "CE", or "catechin equivalent" is a measure of total polyphenolic content, expressed as catechin equivalents mg/g extract derived from sugar cane or catechin equivalents g/L extract derived from sugar cane.

In one embodiment, the extract derived from sugar cane of the present disclosure comprises at least about 20, 25, 30, 35, 40, 45, and up to 50 CE g/L of polyphenols.

In one embodiment, the extract derived from sugar cane of the present disclosure comprises at least about 200, 210, 220, 230, 240, 250, 250, 275, 300, 325, 350, 375, 400, 425, 450, and up to 500 mg CE/g of polyphenols.

In one embodiment, the extract derived from sugar cane of the present disclosure comprises from about 20 CE g/L to about 30 CE g/L of polyphenols or from about 200 CE mg/g to about 300 CE mg/g of polyphenols.

In one embodiment, the extract derived from sugar cane of the present disclosure comprises from about 20 CE g/L to about 27 g CE/L of polyphenols or from about 200 CE mg/g to about 270 CE mg/g of polyphenols.

In one embodiment, the extract derived from sugar cane of the present disclosure comprises from about 27 CE g/L to about 35 g CE/L of polyphenols or about 270 CE mg/g to about 350 CE mg/g of polyphenols.

In one embodiment, the extract derived from sugar cane of the present disclosure comprises from about 35 CE g/L to about 40 g CE/L of polyphenols or from about 350 CE mg/g to about 400 CE mg/g of polyphenols.

In one embodiment, the extract derived from sugar cane of the present disclosure comprises from about 40 CE g/L to about 50 g CE/L of polyphenols or from about 400 CE mg/g to about 500 CE mg/g of polyphenols.

In one embodiment, the extract derived from sugar cane of the present disclosure comprises from about 45 CE g/L to about 50 g CE/L of polyphenols or about 450 CE mg/g to about 500 CE mg/g of polyphenols.

The extract derived from sugar cane of the present disclosure contains the flavonoid class of polyphenols in an amount of about 1 CE g/L to about 15 CH g/L.

In one embodiment, the extract derived from sugar cane of the present disclosure comprises from about 1 CE g/L to about 15 CE g/L of flavonoids or from about 10 CE mg/g to about 150 CE mg/g of flavonoids. In one embodiment, the extract derived from sugar cane of the present disclosure comprises from about 3 CE g/L to about 10 CE g/L of flavonoids or about 30 CE mg/g to about 100 CE mg/g of flavonoids. In one embodiment, the extract derived from sugar cane of the present disclosure comprises about 5 CE g/L to about 8 CE g/L of flavonoids or about 50 CE mg/g to about 80 CE mg/g of flavonoids. In one embodiment, the extract derived from sugar cane of the present disclosure comprises about 6 CE g/L to about 8 CE g/L of flavonoids or about 60 CE mg/g to about 80 CE mg/g of flavonoids. In one embodiment, the extract derived from sugar cane of the present disclosure comprises about 6.5 CE g/L to about 7.5 CE g/L of flavonoids or about 65 CE mg/g to about 75 CE mg/g of flavonoids.

The extract derived from sugar cane of the present disclosure may contain the proanthocyanidin class of polyphenols. The extract derived from sugar cane may contain proanthocyandins in any amount. In one embodiment, the extract derived from sugar cane of the present disclosure comprises at least about 1.5 CE g/L of proanthocyanidins or at least about 15 CE mg/g of proanthocyanidins. In one embodiment, the extract derived from sugar cane of the disclosure comprises at least about 1.8 CE g/L of proanthocyanidins or at least about 18 CE mg/g of proanthocyanidins. In one embodiment, the extract derived from sugar cane of the disclosure comprises about 1.5 CE g/L to about 2.5 CE g/L of proanthocyanidins or about 15 CE mg/g to about 25 CE mg/g of proanthocyanidins. In one embodiment, the extract derived from sugar cane of the disclosure comprises about 1.8 CE g/L to about 2.2 CE g/L of proanthocyanidins or about 18 CE mg/g to about 22 CE mg/g of proanthocyanidins.

The polyphenols of the extract derived from sugar cane of the present disclosure include, but are not limited to, one or more of syringic acid, chlorogenic acid, caffeic acid, vanillin, sinapic acid, p-coumaric acid, ferulic acid, gallic acid, vanillic acid, diosmin, diosmetin, apigenin, vitexin, orientin, homoorientin, swertisin, tricin, (+)-catechin, (-)-catechin gallate, (-) epicatechin, quercetin, kaempherol, myricetin, rutin, schaftoside, isoschaftoside, luteolin, scoparin and/or derivatives thereof. The polyphenols of the extract derived from sugar cane of the present disclosure may also include, but are not limited to, one or more of hydroxycinnamic acid, isoorientin, swertiajaponin, neocarlinoside, isovitexin, vicenin, and/or derivatives thereof.

The polyphenols of the extract derived from sugar cane also include conjugates, such as, for example, glycosides, glucosides, galactosides, galacturonides, ethers, esters, arabinosides, sulphates, phosphates, aldopentoses (xylose, arabinose) and aldohexoses.

In one embodiment, the extract derived from sugar cane of the present disclosure comprises syringic acid, chlorogenic acid, caffeic acid, vanillin, sinapic acid, diosmin, diosmetin, apigenin, vitexin, orientin, homoorientin, swertisin, and tricin and/or derivatives thereof.

In one embodiment, the extract derived from sugar cane of the present disclosure comprises syringic acid, chlorogenic acid and diosmin and/or derivatives thereof.

In one embodiment, the extract derived from sugar cane of the present disclosure comprises syringic acid. In one embodiment, the extract derived from sugar cane of the present disclosure comprises chlorogenic acid. In one embodiment, the extract derived from sugar cane of the present disclosure comprises diosmin. In one embodiment, the extract derived from sugar cane of the present disclosure comprises caffeic acid. In one embodiment, the extract derived from sugar cane of the present disclosure comprises vanillin. In one embodiment, the extract derived from sugar cane of the present disclosure comprises sinapic acid. In one embodiment, the extract derived from sugar cane of the present disclosure comprises vitexin. In one embodiment, the extract derived from sugar cane of the present disclosure comprises p-coumaric acid. In one embodiment, the extract derived from sugar cane of the present disclosure comprises ferulic acid. In one embodiment, the extract derived from sugar cane of the present disclosure comprises gallic acid. In one embodiment, the extract derived from sugar cane of the present disclosure comprises vanillic acid. In one embodiment, the extract derived from sugar cane of the present disclosure comprises diosmetin. In one embodiment, the extract derived from sugar cane of the present disclosure comprises apigenin. In one embodiment, the extract derived from sugar cane of the present disclosure comprises orientin. In one embodiment, the extract derived from sugar cane of the present disclosure comprises homoorientin. In one embodiment, the extract derived from sugar cane of the present disclosure comprises swertisin. In one embodiment, the extract derived from sugar cane of the present disclosure comprises tricin. In one embodiment, the extract derived from sugar cane of the present disclosure comprises (+)-catechin. In one embodiment, the extract derived from sugar cane of the present disclosure comprises (-)-catechin gallate. In one embodiment, the extract derived from sugar cane of the present disclosure comprises (-)-epicatechin. In one embodiment, the extract derived from sugar cane of the present disclosure comprises quercetin. In one embodiment, the extract derived from sugar cane of the present disclosure comprises kaempherol. In one embodiment, the extract derived from sugar cane of the present disclosure comprises myricetin. In one embodiment, the extract derived from sugar cane of the present disclosure comprises rutin. In one embodiment, the extract derived from sugar cane of the present disclosure comprises schaftoside. In one embodiment, the extract derived from sugar cane of the present disclosure comprises isoschaftoside. In one embodiment, the extract derived from sugar cane of the present disclosure comprises luteolin. In one embodiment, the extract derived from sugar cane of the present disclosure comprises scoparin. In one embodiment, the extract derived from sugar cane of the present disclosure comprises hydroxycinnamic acid. In one embodiment, the extract derived from sugar cane of the present disclosure comprises isoorientin. In one embodiment, the extract derived from sugar cane of the present disclosure comprises swertiajaponin. In one embodiment, the extract derived from sugar cane of the present disclosure comprises neocarlinoside. In one embodiment, the extract derived from sugar cane of the present disclosure comprises isovitexin. In one embodiment, the extract derived from sugar cane of the present disclosure comprises vicenin.

In one embodiment, syringic acid, chlorogenic acid and diosmin are the three most abundant polyphenols of the extract derived from sugar cane of the present disclosure.

In one embodiment, the extract derived from sugar cane of the disclosure comprises about 5 - 20 µg/g dry weight of syringic acid. In one embodiment, the extract derived from sugar cane of the disclosure comprises about 7 - 15 µg/g dry weight of syringic acid. In one embodiment, the extract derived from sugar cane of the disclosure comprises about 10 - 12 µg/g dry weight of syringic acid. In one embodiment, the extract derived from sugar cane of the disclosure, when present, comprises about 10.9 µg/g dry weight of syringic acid. The extract derived from sugar cane may be in a syrup form.

In one embodiment, the extract derived from sugar cane of the disclosure comprises about 50 - 200 µg/g dry weight of syringic acid. In one embodiment, the extract derived from sugar cane of the disclosure comprises about 90 - 130 µg/g dry weight of syringic acid. In one embodiment, the extract derived from sugar cane of the disclosure comprises about 100 - 120 µg/g dry weight of syringic acid. In one embodiment, the extract derived from sugar cane of the disclosure comprises about 107 µg/g dry weight of syringic acid. The extract derived from sugar cane may be in a powder form.

In one embodiment, the extract derived from sugar cane of the disclosure comprises about 1 - 15 µg/g dry weight of chlorogenic acid. In one embodiment, the extract derived from sugar cane of the disclosure comprises about 3 - 10 µg/g dry weight of chlorogenic acid. In one embodiment, the extract derived from sugar cane of the disclosure comprises about 5 - 8 µg/g dry weight of chlorogenic acid. In one embodiment, the extract derived from sugar cane of the disclosure comprises about 6.53 µg/g dry weight of chlorogenic acid. The extract derived from sugar cane may be in a syrup form.

In one embodiment, the extract derived from sugar cane of the disclosure comprises about 30 - 150 µg/g dry weight of chlorogenic acid. In one embodiment, the extract derived from sugar cane of the disclosure comprises about 60 - 90 µg/g dry weight of chlorogenic acid. In one embodiment, the extract derived from sugar cane of the disclosure comprises about 70 - 80 µg/g dry weight of chlorogenic acid. In one embodiment, the extract derived from sugar cane of the disclosure comprises about 74 µg/g dry weight of chlorogenic acid. The extract derived from sugar cane may be in a powder form.

In one embodiment, the extract derived from sugar cane of the disclosure comprises about 10 - 30 µg/g dry weight of diosmin. In one embodiment, the extract derived from sugar cane of the disclosure comprises about 15 - 25 µg/g dry weight of diosmin. In one embodiment, the extract derived from sugar cane of the disclosure comprises about 18 - 21 µg/g dry weight of diosmin. In one embodiment, the extract derived from sugar cane of the disclosure comprises about 19 - 45 µg/g dry weight of diosmin. The extract derived from sugar cane may be in a syrup form.

In one embodiment, the extract derived from sugar cane of the disclosure comprises about 100 - 300 µg/g dry weight of diosmin. In one embodiment, the extract derived from sugar cane of the disclosure comprises about 190 - 260 µg/g dry weight of diosmin. In one embodiment, the extract derived from sugar cane of the disclosure comprises about 210 - 240 µg/g dry weight of diosmin. In one embodiment, the extract derived from sugar cane of the disclosure comprises about 227 µg/g dry weight of diosmin. The extract derived from sugar cane may be in a powder form.

In one embodiment, the extract derived from sugar cane of the present disclosure comprises about 7 - 15 µg/g dry weight of syringic acid, and/or about 4 - 9 µg/g dry weight of chlorogenic acid, and/or about 0.1 - 0.5 µg/g dry weight of caffeic acid, about 0.05 - 0.3 µg/g dry weight of vanillin, and/or about 0.1 - 0.3 µg/g dry weight of sinapic acid, and/or about 15 - 25 µg/g dry weight of diosmin, and/or about 0.1 - 0.4 µg/g dry weight of orientin, and/or about 0.4-0.9 µg/g dry weight of swertisin, and/or about 0.05 - 0.3 µg/g dry weight of disomentin. The extract derived from sugar cane may be in a syrup form.

In one embodiment, the extract derived from sugar cane of the present disclosure comprises about 10 - 12 µg/g dry weight of syringic acid, and/or about 5 - 8 µg/g dry weight of chlorogenic acid, and/or about 0.2 - 0.4 µg/g dry weight of caffeic acid, and/or about 0.1 - 0.2 µg/g dry weight of vanillin, and/or about 0.1 - 0.25 µg/g dry weight of sinapic acid, and/or about 18 - 21 µg/g dry weight of diosmin, and/or about 0.2 - 0.3 µg/g dry weight of orientin, and/or about 0.5-0.8 µg/g dry weight of swertisin, and/or about 0.1 - 0.2 µg/g dry weight of disomentin. The extract derived from sugar cane may be in a syrup form.

In one embodiment, the extract derived from sugar cane of the present disclosure comprises about 10.9 µg/g dry weight of syringic acid, and/or about 6.53 µg/g dry weight of chlorogenic acid, and/or about 0.29 µg/g dry weight of caffeic acid, and/or about 0.153 µg/g dry weight of vanillin, and/or about 0.18 µg/g dry weight of sinapic acid, and/or about 19.45 µg/g dry weight of diosmin, and/or about 0.245 µg/g dry weight of orientin, and/or about 0.69 µg/g dry weight of swertisin, and/or about 0.15 µg/g dry weight of disomentin. The extract derived from sugar cane may be in a syrup form.

In one embodiment, the extract derived from sugar cane of the present disclosure comprises about 90 - 130 µg/g dry weight of syringic acid, and/or about 60 - 90 µg/g dry weight of chlorogenic acid, and/or about 4 - 10 µg/g dry weight of caffeic acid, and/or about 1 - 4 µg/g dry weight of vanillin, about 1 - 3 µg/g dry weight of sinapic acid, and/or about 190 - 260 µg/g dry weight of diosmin, and/or about 3 - 7 µg/g dry weight of orientin, and/or 3 - 8 µg/g dry weight of swertisin, and/or about 0.05 - 0.3 µg/g dry weight of disomentin. The extract derived from sugar cane may be in a powder form.

In one embodiment, the extract derived from sugar cane of the present disclosure comprises about 100 - 120 µg/g dry weight of syringic acid, and/or about 70 - 80 µg/g dry weight of chlorogenic acid, and/or about 6 - 8 µg/g dry weight of caffeic acid, about 2 - 3 µg/g dry weight of vanillin, and/or about 1.5 - 2.5 µg/g dry weight of sinapic acid, and/or about 210 - 240 µg/g dry weight of diosmin, about 4 - 5 µg/g dry weight of orientin, 4-6 µg/g dry weight of swertisin, and/or about 0.1 - 0.2 µg/g dry weight of disomentin. The extract derived from sugar cane may be in a powder form.

In one embodiment, the extract derived from sugar cane of the present disclosure comprises about 107 µg/g dry weight of syringic acid, and/or about 74 µg/g dry weight of chlorogenic acid, and/or about 7.5 µg/g dry weight of caffeic acid, and/or about 2 µg/g dry weight of vanillin, and/or about 1.7 µg/g dry weight of sinapic acid, and/or about 227 µg/g dry weight of diosmin, and/or about 4.5 µg/g dry weight of orientin, 5.2 µg/g dry weight of swertisin, and/or about 0.16 µg/g dry weight of disomentin. The extract derived from sugar cane may be in a powder form.

The extract derived from sugar cane of the present disclosure may contain a range of organic acids that are found naturally in sugar cane. These organic acids may include, but are not limited to, aconitic (*cis-* and *trans-*)*,* oxalic, citric, lactic, tartaric, glycolic, succinic, citric, malic, fumaric and shikimic acids. In one embodiment, the extract derived from sugar cane contains higher levels of citric and malic acids than other organic acids. In another embodiment, the extract derived from sugar cane contains low to trace amounts of oxalic, citric, tartaric, glycolic, succinic and citric acids. In another embodiment, the two most abundant organic acids in the extract derived from sugar cane are *trans-* and cis-aconitic acids.

The extract derived from sugar cane of the present disclosure may contain *trans-* and/or cis-aconitic acids. In one embodiment, the extract derived from sugar cane of the present disclosure comprises *trans*-aconitic in amount of about 10,000 - 40,000 mg per kg and/or cis-aconitic in amount of about 3,000 - 7,000 mg/kg. In one embodiment, the extract derived from sugar cane of the present disclosure contains *trans*-aconitic in an amount of about 17,000 - 30,000 mg per kg and/or cis-aconitic in amount of about 4,000 - 6,500 mg/kg. In one embodiment, the extract derived from sugar cane of the present disclosure may contain *trans*-aconitic in amount of about 20,000-25,000 mg per kg and/or cis-aconitic in amount of about 5,000 - 5,500 mg/kg.

The extract derived from sugar cane of the present disclosure may contain amino acids. In one embodiment, the total amino acids levels of the extract derived from sugar cane of the present disclosure is about 50,000 - 80,000 µg per gram, or about 60,000 - 70,000 µg per gram, or about 65,000 µg per gram. In one embodiment, about 10 - 40% of these total amino acids are essential amino acids. In one embodiment, about 15 - 30% of these total amino acids are essential amino acids. In one embodiment, about 20 - 25% of these total amino acids are essential amino acids.

The extract derived from sugar cane of the present disclosure may contain free amino acids. In one embodiment, the extract derived from sugar cane of the present disclosure comprises about 10,000 - 50,000 µg of free amino acids per gram. In one embodiment, the extract derived from sugar cane of the present disclosure may contain about 20,000 - 35,000 µg of free amino acids per gram. The extract derived from sugar cane of the present disclosure may contain about 25,000 - 30,000 µg of free amino acids per gram.

As defined above, the term "free amino acids" as used herein refers to amino acids which are singular molecules and structurally not attached to peptide bonds which are attached to other amino acids.

The extract derived from sugar cane of the present disclosure may contain leucine, a branched chain essential amino acid. In one embodiment, the concentration of leucine in the extract derived from sugar cane, is about 1 - 5 mM, or about 1.5 - 4 mM, or about 2 - 3 mM. In one embodiment, the amount of leucine in the extract derived from sugar cane is about 1,000 - 20,000 µg per gram, or about 1,000 - 10,000 µg per gram, or about 1,000 - 5,000 µg per gram, or about 1,000 - 2,000 µg per gram, or about 5,000 - 10,000 µg per gram, or about 10,000 - 20,000 µg per gram.

The extract derived from sugar cane of the present disclosure may contain minerals. In one embodiment, the extract derived from sugar cane contains minerals that are found naturally in sugar cane. In one embodiment, the extract derived from sugar cane contains one or more minerals including, but not limited to, potassium, sodium, calcium, magnesium, iron, zinc, selenium and chromium.

In one embodiment, the extract derived from sugar cane contains minerals bound to the polyphenols. In one embodiment, the extract derived from sugar cane contains divalent ions bound to the polyphenols. In one embodiment, the extract derived from sugar cane contains calcium, magnesium and/or iron bound to the polyphenols. In one embodiment, the extract derived from sugar cane contains iron bound to the polyphenols.

In one embodiment, the extract derived from sugar cane of the present disclosure comprises about 20,000 - 32,000 mg of potassium per kilogram, and/or about 300 - 600 mg of sodium per kilogram, and/or about 800 - 1,300 mg of calcium per kilogram, and/or about 3,000 - 6,000 mg of magnesium per kilogram, and/or about 40 - 90 mg of iron per kilogram, and/or about 3 - 10 mg of zinc per kilogram, and/or about 500 - 900 µg of selenium per kilogram and/or about 1,000 - 1,600 µg of chromium per kilogram. The extract derived from sugar cane may be in a syrup form.

In one embodiment, the extract derived from sugar cane of the present disclosure comprises about 25,000 - 27,000 mg of potassium per kilogram, and/or about 400 - 500 mg of sodium per kilogram, and/or about 1,000 - 1,200 mg of calcium per kilogram, and/or about 4,000 - 5,500 mg of magnesium per kilogram, and/or about 55 - 75 mg of iron per kilogram, and/or about 5.5 - 7.5 mg of zinc per kilogram, and/or about 700 - 850 µg of selenium per kilogram, and/or about 1,200 - 1,400 µg of chromium per kilogram. The extract derived from sugar cane may be in a syrup form.

In one embodiment, the extract derived from sugar cane of the present disclosure comprises about 26,000 mg of potassium per kilogram, and/or about 450 mg of sodium per kilogram, and/or about 1,090 mg of calcium per kilogram, and/or about 4,700 mg of magnesium per kilogram, and/or about 65 mg of iron per kilogram, about 6.6 mg of zinc per kilogram, and/or about 786 µg of selenium per kilogram and/or about 1,300 µg of chromium per kilogram. The extract derived from sugar cane may be in a syrup form.

In one embodiment, the extract derived from sugar cane of the present disclosure comprises about 50 - 350 mg of potassium per kilogram, and/or about 5 - 70 mg of sodium per kilogram, and/or about 7,000 - 10,000 mg of calcium per kilogram, and/or about 1,000 - 3,000 mg of magnesium per kilogram, and/or about 500 - 1,300 mg of iron per kilogram. The extract derived from sugar cane may be in a powder form.

In one embodiment, the extract derived from sugar cane of the present disclosure comprises about 100 - 250 mg of potassium per kilogram, and/or about 10 - 50 mg of sodium per kilogram, and/or about 8,000 - 9,000 mg of calcium per kilogram, and/or about 1,500 - 2,500 mg of magnesium per kilogram, and/or about 800 - 1,000 mg of iron per kilogram. The extract derived from sugar cane may be in a powder form.

In one embodiment, the extract derived from sugar cane of the present disclosure comprises about 190 mg of potassium per kilogram, and/or about 30 mg of sodium per kilogram, and/or about 8,800 mg of calcium per kilogram, and/or about 2,000 mg of magnesium per kilogram, and/or about 890 mg of iron per kilogram. The extract derived from sugar cane may be in a powder form.

The extract derived from sugar cane of the present disclosure may contain monosaccharides, disaccharides, oligosaccharides and/or polysaccharides. Examples of these include, but are not limited to, sucrose, glucose, galactose, xylose, ribose, mannose, rhamnose, fructose, maltose, lactose, maltotriose, xylopyranose, raffinose, 1-kestose, theanderose, 6-kestose, panose, neo-kestose, nystose, glucans and xylans.

The extract derived from sugar cane of the present disclosure may contain fiber. The fiber may be present in the extract as obtained by the process or fiber may be added to the extract. The term "fiber" as used herein refers to indigestible portion of food derived from plants. The fiber may be soluble or insoluble fiber. Non-limiting examples of fiber include, sugar cane fiber, oat bran, flour (including, for example, soy, rice, wheat, bran, rye, corn, sorghum, potato), modified starch, gelatin, non-starch polysaccharides such as arabinoxylans, cellulose, chia fiber, psyllium fiber, fenugreek fiber and many other plant components such as resistant starch, resistant dextrins, inulin, lignin, chitins, pectins, beta-glucans, and oligosaccharides. In one embodiment, the extract derived from sugar cane of the present disclosure contains sugar cane fiber. In one embodiment, the extract derived from sugar cane of the present disclosure contains flour. In one embodiment, the extract derived from sugar cane of the present disclosure contains modified starch. In one embodiment, the extract derived from sugar cane of the present disclosure contains cellulose. In one embodiment, the extract derived from sugar cane of the present disclosure contains chia fiber. In one embodiment, the extract derived from sugar cane of the present disclosure contains pysillium fiber. In one embodiment, the extract derived from sugar cane of the present disclosure contains fenugreek fiber.

In one embodiment, the fiber is present in the extract of the present disclosure. In one embodiment, the fiber is added to the extract of the present disclosure.

The extract derived sugar cane of the present disclosure may be a mash, crumble, pellet, syrup, liquid or powder. In one embodiment, the extract may be a mash, crumble, or pellet. In one embodiment, the extract may be a mash. In one embodiment, the extract may be a crumble. In one embodiment, the extract may be a pellet. In one embodiment, the extract may be a liquid. In one embodiment, the extract may be a syrup.

The extract derived from sugar cane of the present disclosure may be in a powder form. In one embodiment, the powder form is a freeze dried powder form, or a dehydrated powder form or a spray dried powder form. The extract derived from sugar cane of the present disclosure may be in an encapsulated form.

It may be desirable that extremes of pH of the extract derived from sugar cane of the present disclosure be avoided. In one embodiment the pH of the extract derived from sugar cane of the present disclosure is in the range of about 3 to about 7, or about 3 to about 6, or about 4 to about 5.5, or about 4.5 to about 5,or about 4.6 to about 4.8.

The Brix value of the extract derived from sugar cane of the present disclosure may vary. In some instances the Bx value of the extract is at least about 40°Bx (degrees Brix). In some instances the Bx value of the extract is at least about 50°Bx. In some instances the Bx value of the extract is at least about 60°Bx. In some instances the Bx value of the extract is at least about 65°Bx. In some instances the Bx value of the extract is at least about 70°Bx. In some instances the Bx value of the extract is about 50 - 75 °Bx. In some instances the Bx value of the extract is about 50 - 70 °Bx. In some instances the Bx value of the extract is about 60 - 65 °Bx. In some instances the Bx value of the extract is about 50 - 60 °Bx. In some instances the Bx value of the extract is about 55 °Bx. In some instances the Bx value of the extract is about 60 - 65 °Bx. In some instances the Bx value of the extract is about 64 - 65 °Bx. In some instances the Bx value of the extract is about 65 -70 °Bx. In some instances the Bx value of the extract is about 70 - 75 °Bx. In some instances the Bx value of the extract is about 75 - 80 °Bx.

### Compositions, methods and uses of the extracts derived from sugar cane

### Compositions, animal feed supplements and animal feeds

The extracts derived from sugar cane of the present disclosure may be included in veterinary compositions for administration to an animal or included in animal supplements or animal feeds intended for intake by an animal. The veterinary compositions, animal supplements or animal feeds may have application in various uses and methods.

The extracts derived from sugar cane of the present disclosure, together with a conventional adjuvant, carrier or diluent, may be placed into the form of a veterinary composition and unit dosages thereof, and in such form may be employed as solids, such as tablets, powders or filled capsules, liquids as solutions, suspensions, emulsions (including microemulsions), syrups, elixirs or capsules filled with the same, creams, serums, gels, and oils. Extracts derived from sugar cane of the present disclosure, together with other conventional additives may be placed in animal feed supplements or animal feeds.

The veterinary compositions of the disclosure may also contain other ingredients. For example, but not limited to, the compositions of the disclosure may also contain the components as listed hereafter. A binder such as gum, acacia, corn starch or gelatin; excipients such as dicalcium phosphate which may be used as a diluting agent; a disintegrating agent such as corn starch, potato starch, alginic acid and the like; a lubricant such as magnesium stearate; a sweetening agent such as sucrose, lactose or saccharin; and a liquid carrier, may be added. Various other ingredients may be present as coatings or to otherwise modify the physical form of the veterinary composition. The veterinary compositions may contain methyl and propylparabens as preservatives, a dye and flavouring agents such as cherry or orange flavour. Information on additives and excipients that are suitable for veterinary applications may be found, for example, in the Merck Veterinary Manual (online at www.merckvetmanual.com).

Veterinary compositions of the present disclosure may be formulated for oral administration (including buccal or sublingual) or nasal administration (including buccal and sublingual). Therefore, the veterinary compositions of the invention may be formulated, for example, as tablets, capsules, powders, granules, lozenges, creams or liquid preparations such as oral solutions or suspensions. Such formulations may be prepared by any method known in the art, for example by bringing into association an active ingredient, or combination of active ingredients, of with acceptable excipient(s). Such formulations may be prepared as enterically coated granules, tablets or capsules suitable for oral administration and delayed release formulations. The combinations of active ingredients are proposed for both liquid delivery as well as in solids for mixing through animal feeds.

The veterinary compositions of the disclosure may be presented in a single unit form or in a bulk form and may be prepared by any of the methods well known in the art. All methods include the step of bringing the extract derived from sugar cane of the present disclosure, into association with one or more accessory ingredients. In general, the compositions are prepared by uniformly and intimately bringing the extract derived from sugar cane of the present disclosure, into association with a liquid carrier or a finely divided solid carrier or both, and then, if necessary, shaping the product into the desired formulation. As used herein, the term "composition" is intended to encompass a product comprising the specified ingredients, as well as any product which results, directly or indirectly, from combination of the specified ingredients.

Veterinary compositions include those for oral administration. The compositions include solutions, syrups, powders, tablets and capsules. In one embodiment, the composition is in a dry form or a liquid form. In one embodiment, the composition is in a dry form. In one embodiment, the composition is in a liquid form. In one embodiment, the composition is in a syrup form. In one embodiment, the composition is in a tablet or capsule form. In one embodiment, the composition is in a tablet form. In one embodiment, the composition is in a capsule form. Such forms are conveniently stable under the conditions of manufacture and storage and are generally preserved against the contaminating action of microorganisms such as bacteria and fungi.

The extracts derived from sugar cane of the present disclosure may be included in animal supplements or animal feeds intended for intake by an animal. Animal supplements and animal feeds of the present disclosure may be formulated following well known methods in the art. Guidance on feed formulation is provided by, for example, the Food and Agriculture Organization of the United Nations at (www.fao.org). It would be recognised that formulation of feeds is dependent on the animal subject. For example, animal feed for a monogastric animal, such as a pig, typically comprises concentrates as well as supplements whereas animal feed for ruminants generally comprises forage (including roughage and silage) and may further comprise concentrates as well as supplements. It would be further recognised that feed formulation depends on the availability, quality and expense of ingredients which can vary from season to season and geographic location. For example, fishmeal has a high quality of protein to meet the essential amino acid (EAA) requirements in fish feeds but is expensive. In its place, plant protein sources such as soya-bean meal or a combination of fishmeal and plant protein may be used.

Supplements may include vitamins, minerals (e.g. calcium, phosphorus, trace elements such as zinc, selenium and chromium, sodium), enzymes (e.g. phytases to improve nutrient digestibility), essential oils, direct fed microbial (to maintain gastrointestinal microbiota balance and health), organic acids, amino acids (e.g, methionine, lysine and threonine) which may be provided in a premix.

Other additives include auxiliary components and excipients as described above for veterinary compositions including: binders, anti-oxidants, preservatives, coloring agents, pigments and dyes, flavouring agents, such as sweeteners, which may be used to mask the bitterness of feed ingredients to improve feed palatability, vehicles, diluting agents, emulsifying and suspending agents, attractants, and medications including growth enhancers, immunostimulants, hormones and antimicrobials. In addition, excipients are chosen for their suitability in preparing feed forms such as mash, granules, crumbles, pellets, powders and lickblocks. For example, cornstarch or polyvinylpyrollidone (PVP) are suitable for forming a granular feed product. Guidance on animal feed additives, excipients and supplements is also provided by the Food and Agriculture Organization of the United Nations at (www.fao.org) and other resources, for example, the Merck Veterinary Manual (online at www.merckvetmanual.com) and the CRC Handbook of Food, Drug and Cosmetic Excipients, 2005.

The animal supplements or animal feeds of the present disclosure may have a Brix value of at least about 40°Bx. In some instances the Bx value of the animal supplement or animal feed is at least about 50°Bx. In some instances the Bx value of the animal supplement or animal feed is at least about 60°Bx. In some instances the Bx value of the animal supplement or animal feed is at least about 65°Bx. In some instances the Bx value of the animal supplement or animal feed is at least about 70°Bx. In some instances the Bx value of the animal supplement or animal feed is about 50 - 75 °Bx. In some instances the Bx value of the animal supplement or animal feed is about 50 - 70 °Bx. In some instances the Bx value of the animal supplement or animal feed is about 60 - 65 °Bx. In some instances the Bx value of the animal supplement or animal feed is about 50 - 60 °Bx. In some instances the Bx value of the animal supplement or animal feed is about 55 °Bx. In some instances the Bx value of the animal supplement or animal feed is about 60 - 65 °Bx. In some instances the Bx value of the animal supplement or animal feed is about 64 - 65 °Bx. In some instances the Bx value of the animal supplement or animal feed is about 65 -70 °Bx. In some instances the Bx value of the animal supplement or animal feed is about 70 - 75 °Bx. In some instances the Bx value of the animal supplement or animal feed is about 75 - 80 °Bx.

The animal supplements or animal feeds of the present disclosure may contain fiber. The term "fiber" as used herein refers to indigestible portion of food derived from plants. The fiber may be soluble or insoluble fiber. The fiber may be mixed with the extract of the present disclosure to provide the animal supplement or feed or the fiber may be coated onto the extract of the present disclosure to provide the animal supplement or feed. In one embodiment, the fiber is mixed with the extract of the present disclosure to provide the animal supplement or feed. In one embodiment, the fiber is coated onto the extract of the present disclosure to provide the animal supplement or feed.

The fiber may be present in the animal supplement or animal feed of the present disclosure in an amount up to about 20 wt%. In one embodiment, the fiber is present in the animal supplement or animal feed in an amount up to about 0.5, 1, 1.5, 2, 2.5, 3, 3.5, 4, 4.5, 5, 5.5, 6, 6.5, 7, 7.5, 8, 8.5, 9, 9.5, 10, 11, 12, 13, 14, 15, 16, 17, 18, 19 or 20 wt%. In one embodiment, the fiber is present in the animal supplement or animal feed in an amount up to about 0.5 wt%. In one embodiment, the fiber is present in the animal supplement or animal feed in an amount up to about 1 wt%. In one embodiment, the fiber is present in the animal supplement or animal feed in an amount up to about 1.5 wt%. In one embodiment, the fiber is present in the animal supplement or animal feed in an amount up to about 2 wt%. In one embodiment, the fiber is present in the animal supplement or animal feed in an amount up to about 3 wt%. In one embodiment, the fiber is present in the animal supplement or animal feed in an amount up to about 4 wt%. In one embodiment, the fiber is present in the animal supplement or animal feed in an amount up to about 5 wt%. In one embodiment, the fiber is present in the animal supplement or animal feed in an amount up to about 10 wt%. In one embodiment, the fiber is present in the animal supplement or animal feed in an amount up to about 15 wt%. In one embodiment, the fiber is present in the animal supplement or animal feed in an amount up to about 20 wt%.

Non-limiting examples of fiber include, sugar cane fiber, oat bran, flour, modified starch, gelatin, non-starch polysaccharides such as arabinoxylans, cellulose, chia fiber, psyillium fiber, fenugreek fiber and many other plant components such as resistant starch, resistant dextrins, inulin, lignin, chitins, pectins, beta-glucans, and oligosaccharides. In one embodiment, the animal supplement or animal feed of the present disclosure contains sugar cane fiber. In one embodiment, the animal supplement or animal feed of the present disclosure contains modified starch. In one embodiment, the animal supplement or animal feed of the present disclosure contains cellulose. In one embodiment, the animal supplement or animal feed contains chia fiber. In one embodiment, the animal supplement or animal feed of the present disclosure contains pysillium fiber. In one embodiment, the animal supplement or animal feed of the present disclosure contains fenugreek fiber.

The compositions, animal supplements or animal feeds of the present disclosure may also comprise other compounds which can be applied in the improvement or maintenance of the health of an animal. Selection of the appropriate active compounds for use in combination therapy may be made by one of ordinary skill in the art, according to conventional veterinary principles. The combination of active compounds may act synergistically to effect the improvement or maintenance of the health of an animal. Using this approach, one may be able to achieve efficacy with lower dosages of each active compound, thus reducing the potential for adverse side effects.

In one embodiment, the active compound(s) for use in combination therapy is one or more plant bioactives. In one embodiment, the active compound(s) for use in combination therapy is one or more marine bioactives.

The compositions, animal supplements or animal feeds of the present disclosure may comprise the extracts derived from sugar cane of the present disclosure in an amount of up to about 5.0 wt % based upon the total weight of the composition, animal supplement or animal feed. In one embodiment, the compositions, animal supplements or animal feeds of the present disclosure comprise the extracts derived from sugar cane of the present disclosure in an amount of about 0.1, 0.2, 0.3, 0.4, 0.5, 0.6, 0.7, 0.8, 0.9, 1.0, 1.1, 1.2, 1.3, 1.4, 1.5, 1.6, 1.8, 1.8, 1.9, 2.0, 2.1, 2.2, 2.3, 2.4, 2.5, 2.6, 2.7, 2.8, 2.9, 3.0, 3.1, 3.2, 3.3, 3.4, 3.5, 3.6, 3.7, 3.8, 3.9, 4.0, 4.1, 4.2, 4.3, 4.4, 4.5, 4.6, 4.7, 4.8, 4.9, 5.0, 6.0, 7.0, 8.0, 9.0, 10.0, 15.0 or 20.0 wt % based upon the total weight of the composition, animal supplement or animal feed. In one embodiment, the compositions, animal supplements or animal feeds comprise the extracts derived from sugar cane of the present disclosure in an amount of 0.1 wt % to 5 wt % based upon the total weight of the composition, animal supplement or animal feed. In one embodiment, the compositions, animal supplements or animal feeds comprise the extracts derived from sugar cane of the present disclosure in an amount of 0.1 wt % to 0.5 wt % based upon the total weight of the composition, animal supplement or animal feed. In one embodiment, the compositions, animal supplements or animal feeds comprise the extracts derived from sugar cane of the present disclosure in an amount of 0.1 wt % to 1 wt % based upon the total weight of the composition, animal supplement or animal feed. In one embodiment, the compositions, animal supplements or animal feeds comprise the extracts derived from sugar cane of the present disclosure in an amount of 0.1 wt % to 2 wt % based upon the total weight of the composition, animal supplement or animal feed. In one embodiment, the compositions, animal supplements or animal feeds comprise the extracts derived from sugar cane of the present disclosure in an amount of 0.01 wt % to 1 wt % based upon the total weight of the composition, animal supplement or animal feed. In one embodiment, the compositions, animal supplements or animal feeds comprise the extracts derived from sugar cane of the present disclosure in an amount of 0.01 wt % to 0.05 wt % based upon the total weight of the composition, animal supplement or animal feed. In one embodiment, the compositions, animal supplements or animal feeds comprise the extracts derived from sugar cane of the present disclosure in an amount of 0.01 wt % to 2 wt % based upon the total weight of the composition, animal supplement or animal feed.

In one aspect of the disclosure there is provided an animal feed comprising an animal supplement as described herein. In one embodiment, the supplement is present in the animal feed in an amount up to about 20 wt%. In one embodiment, the supplement is present in the animal feed in an amount up to about 0.5, 1, 1.5, 2, 2.5, 3, 3.5, 4, 4.5, 5, 5.5, 6, 6.5, 7, 7.5, 8, 8.5, 9, 9.5, 10, 11, 12, 13, 14, 15, 16, 17, 18, 19 or 20 wt%. In one embodiment, the supplement is present in the animal feed in an amount up to about 0.5 wt%. In one embodiment, the supplement is present in the animal feed in an amount up to about 1 wt%. In one embodiment, the supplement is present in the animal feed in an amount up to about 2 wt%. In one embodiment, the supplement is present in the animal feed in an amount up to about 5 wt%. In one embodiment, the supplement is present in the animal feed in an amount up to about 10 wt%. In one embodiment, the supplement is present in the animal feed in an amount up to about 15 wt%. In one embodiment, the supplement is present in the animal feed in an amount up to about 20 wt%.

In one aspect of the disclosure there is provided an non-human animal formulated supplement comprising an extract derived from sugar cane. In one embodiment, the extract comprises from about 20 catechin equivalent (CE) g/L to about 50 CE g/L of polyphenols or from about 100 CE mg/g to about 500 CE mg/g of polyphenols.

In one embodiment, the extract comprises at least 20, 25, 30, 35, 40, 45, up to 50 CE g/L of polyphenols. In one embodiment, the extract comprises at least about 200, 210, 220, 230, 240, 250, 250, 275, 300, 325, 350, 375, 400, 425, 450, up to 500 mg CE/g of polyphenols.

In one embodiment, the extract comprises from about 20 CE g/L to about 30 CE g/L of polyphenols or from about 200 CE mg/g to about 300 CE mg/g of polyphenols. In one embodiment, the extract comprises from about 20 CE g/L to about 27 g CE/L of polyphenols or from about 200 CE mg/g to about 270 CE mg/g of polyphenols. In one embodiment, the extract comprises from about 27 CE g/L to about 35 g CE/L of polyphenols or about 270 CE mg/g to about 350 CE mg/g of polyphenols. In one embodiment, the extract comprises from about 35 CE g/L to about 40 g CE/L of polyphenols or from about 350 CE mg/g to about 400 CE mg/g of polyphenols. In one embodiment, the extract comprises from about 40 CE g/L to about 50 g CE/L of polyphenols or from about 400 CE mg/g to about 500 CE mg/g of polyphenols. In one embodiment, the extract comprises from about 45 CE g/L to about 50 g CE/L of polyphenols or about 450 CE mg/g to about 500 CE mg/g of polyphenols.

### Methods and uses

The extracts derived from sugar cane of this disclosure comprise a complex mixture of plant primary and secondary metabolites, including polyphenols. The collective variety and number of plant primary and secondary metabolites (including polyphenols) of the extracts drastically exceed what is typical in normal animal diets. When an extract is consumed by an animal subject, the plant metabolites stimulate a variety of biological mechanisms (including for example, anti-oxidative pathways, anti-inflammatory pathways and immunomodulatory pathways) in the animal resulting in numerous beneficial health effects. The results described in the present disclosure demonstrate what is currently understood about the impact of the extracts on a number of biological mechanisms and the resultant health effects. However, due to the complex nature of the extracts and the current state of the art, further biological mechanisms may be present, but not yet described.

The compositions, supplements and feeds comprising the extracts of the present disclosure can be used for improving or maintaining health in an animal subject. It is noted that such uses of improving or maintaining health are not part of the present invention as defined by the claims. These are therefore disclosed for information. The present inventors have surprisingly found that the extracts derived from sugar cane of the present disclosure have properties making them favourable for use in improving or maintaining the health of animals. Representative properties include: a beneficial immunomodulatory effect, wherein the local or systemic immune response is beneficially stimulated or modulated; an anti-inflammatory effect; an anti-oxidant effect; a cytoprotective effect; and an anti-microbial effect, wherein gastrointestinal microbiota function is improved or maintained. Further favourable properties include anti-viral activity; anti-bacterial activity; anti-carcinogenic activity; cardio-vascular benefits; anti-ulcer activity; vasodilatory properties; gene regulating properties; anticariogenic and analgesic properties.

In one aspect of the disclosure there is provided a non-therapeutic use of the supplement according to the present invention for improving growth performance in a non-human animal subject, the method comprising the step of administering an effective amount of the supplement or the animal feed described herein. In one embodiment, the size of the subject is increased. In one embodiment, the weight gain of the subject is increased. In one embodiment, the average weight gain of the subject is increased. In one embodiment, the daily weight gain of the subject is increased. In one embodiment, the average daily weight gain of the subject is increased. In one embodiment, the weight gain is the live weight gain. In one embodiment, the length of the subject is increased. In one embodiment, the standard length of the subject is increased. In one embodiment, the average standard length of the subject is increased. In one embodiment, the fork length of the subject is increased, wherein the subject is a finfish. In one embodiment, the average fork length of the subject is increased, wherein the subject is a finfish. In one embodiment, the total length of the subject is increased. In one embodiment, the average total length of the subject is increased. In one embodiment, the body length of the subject is increased. In one embodiment, the average body length of the subject is increased. In one embodiment, the head length of the subject is increased. In one embodiment, the average head length of the subject is increased. In one embodiment, feed conversion ratio (FCR) is reduced.

In one aspect of the disclosure there is provided a non-therapeutic use of the supplement of the present invention for reducing body fat content in a non-human animal subject, the method comprising the step of administering an effective amount of an the supplement or the feed described herein. In one embodiment, there is a concomitant reduction in body weight of the subject. In one embodiment, peripheral and/or visceral fat is reduced.

In one embodiment, there is no adverse effect on blood health. In one embodiment, there is no adverse effect on blood health as assessed by complete blood count (CBC) analysis. In one embodiment, there is no adverse effect on blood health as assessed by a count of the number of red blood cells (RBC) and/or the number of white blood cells (WBC). In one embodiment, there is no adverse effect on blood health as assessed by an analysis of the total haemoglobin in blood. In one embodiment, there is no adverse effect on blood health as assessed by an analysis of the fraction of the blood composed of RBC (haematocrit).

In one embodiment, there is no adverse effect on urinary health. In one embodiment, there is no adverse effect on urine pH. In one embodiment, there is no adverse effect on urine specific gravity.

In one aspect of the disclosure there is provided a non-therapeutic use of the supplement of the present invention for improving nutrient digestibility in a non-human animal subject, the method comprising the step of administering to the subject an effective amount of an the supplement or the animal feed described herein. In one embodiment, there is negligible digestible food remaining in the faeces of the subject.

In one aspect of the disclosure there is provided a non-therapeutic use of the supplement of the present invention for reducing feed conversion ratio (FCR) in a non-human animal subject, the method comprising administering to the subject an effective amount of the supplement or the feed described herein.

In one aspect of the disclosure there is provided a non-therapeutic method for improving food production and quality. In one embodiment, there is an improvement in milk yield. In one aspect of the disclosure there is provided a method for improving meat quality in a non-human animal subject, the method comprising administering to the subject an effective amount of the supplement or the feed described herein. In one embodiment, the toughness of meat is improved. In one embodiment, the toughness of meat is improved as assessed by shear force measurement. In one embodiment, the taste of the meat is improved. In one embodiment, the flavour of the meat is improved. In one embodiment, the odour of the meat is reduced. In one embodiment, the protein percentage of the meat is increased. In one embodiment, the shelf life of the meat is extended. As would be understood by a skilled person, shelf life is the recommended maximum time for which products or fresh (harvested) produce can be stored, during which the defined quality of a specified proportion of the goods remains acceptable under expected (or specified) conditions of distribution, storage and display. In one embodiment, the onset of rancidity of the meat is delayed or slowed. As would be understood by a skilled person, rancidity is the process which causes a substance to become rancid, that is, having a rank, unpleasant smell or taste. Specifically, it is the hydrolysis and/or autoxidation of fats into short-chain aldehydes and ketones which are objectionable in taste and odour.

In one aspect of the disclosure, which is not part of the invention as defined by the claims, there is provided a method for preventing and/or treating anemia in a non-human animal subject, wherein the method comprises the step of administering an effective amount of the supplement or the feed described herein. In one embodiment, anemia is a vitamin deficiency anemia. In one embodiment, the anemia is an iron deficiency anemia. In one embodiment, the extract further comprises iron bound to the polyphenols.

In the method for preventing and/or treating anemia in a non-human animal subject, the frequency and amount of non-human animal formulated supplement or non-human animal feed administered may be varied as required to prevent and/or treat the anemia in the animal subject. In one aspect, , the dosage is in the range of about 100 mg to 300 mg at birth followed by about 100 mg to 300 mg at 14 days. In one embodiment, the dosage is in the range of about 150 mg to 250 mg at birth followed by about 150 mg to 250 mg at 14 days. In one aspect, the dosage is in the range of about 100 mg to 200 mg at birth followed by about 100 mg to 200 mg at 14 days. In one aspect, , the dosage is about 300 mg at birth followed by about 300 mg at 14 days. In one embodiment, the dosage is about 300 mg at birth followed by about 300 mg at 14 days. In one aspect, , the supplement is administered at a fixed dose of about 250 mg at birth followed by a fixed dose of about 250 mg at 14 days. In one aspect, , the supplement is administered at a fixed dose of about 200 mg at birth followed by a fixed dose of about 200 mg at 14 days. In one aspect, , the supplement is administered at a fixed dose of about 150 mg at birth followed by a fixed dose of about 150 mg at 14 days.

In one aspect, , the subject is a pig. In one embodiment, the white blood cell count in a blood sample withdrawn from the pig subject is increased. In one embodiment, the red blood cell count in a blood sample withdrawn from the pig subject is increased. In one aspect, the concentration of haemoglobin in a blood sample withdrawn from the pig subject is increased. In one embodiment, growth performance of the pig subject is improved. In one embodiment, weight gain of the pig is increased. In one embodiment, the weight gain is the live weight gain.

In one aspect of the disclosure there is provided a non-therapeutic use of the supplement of the present invention for improving or maintaining muscle condition in a non-human animal subject, wherein the method comprises the step of administering an effective amount of the supplement or the feed described herein. In one embodiment, muscle build is improved. In one embodiment, muscle shape is improved.

In one aspect of the disclosure there is provided a non-therapeutic use of the supplement of the present invention for stimulating or sustaining appetite in a non-human animal subject, wherein the method comprises the step of administering an effective amount of the supplement or the feed described herein.

In one aspect of the disclosure, which is not part of the invention as defined by the claims, there is provided a method for preventing, reducing and/or treating gastric ulcers in a non-human animal subject, the method comprising the step of administering an effective amount of a non-human animal formulated supplement or a non-human animal feed described herein. In one aspect, , the gastric ulcers are prevented. In one embodiment, the ulcers are treated. In one embodiment, the gastric ulcers are reduced. In one embodiment, the animal subject is a horse.

The compositions, non-human animal formulated supplements and non-human animal feeds comprising the extracts of the present disclosure can be administered or fed to a non-human animal subject. The term "animal subject" as used herein refers to any animal except humans. Thus, the disclosure relates to non-human animals. The non-human animals may be mammals. Examples of non-human animals are aquatic animals, insects, amphibians, reptiles, gastropods, birds, monogastric animals, ruminants and pseudo-ruminants.

In one embodiment, the animal is an aquatic animal. In one embodiment, the aquatic animal is finfish and shellfish. In one embodiment, the aquatic animal is finfish. In one embodiment, the aquatic animal is shellfish. In one embodiment, the finfish is pangus. In one embodiment, the finfish is tilapia. In one embodiment, the finfish is salmon. In one embodiment, the finfish is barramundi. In one embodiment, the finfish is selected from barramundi, bass, bream, carp, catfish, cod, crappie, drum, eel, goby, goldfish, grouper, halibut, java, labeo, lai, loach, mackerel, milkfish, mojarra, mudfish, mullet, paco, pearlspot, pejerrey, perch, pike, pompano, roach, salmon, sampa, sauger, sea bass, seabream, shiner, sleeper, snakehead, snapper, snook, sole, spinefoot, sturgeon, sunfish, sweetfish, tench, terror, trout, tuna, turbot, vendace, walleye and whitefish.

In one embodiment, the aquatic animal is shellfish. In one embodiment, the shellfish is a crustacean. In one embodiment, the shellfish is a mollusc. In one embodiment, the shellfish is crustacean is selected from crabs, crayfish, lobsters, prawns and shrimp. In one embodiment, the shellfish is prawns. In one embodiment, the shellfish is shrimp. In one embodiment, the shellfish is a mollusc selected from clams, mussels, oysters, scallops and winkles. In one embodiment, the shellfish is mussels. In one embodiment, the shellfish is oysters. In one embodiment, the shellfish is scallops.

In one embodiment, the animal is an insect. In one embodiment, the insect is selected from cicadas, grasshoppers, beetles, bees, wasps, butterflies, moths, ants, flies, crickets, aphids, bugs and dragonflies. In one embodiment, the insect is grasshoppers. In one embodiment, the insect is bees. In one embodiment, the insect is crickets. In one embodiment, the insect is butterflies.

In one embodiment, the animal is an amphibian. In one embodiment, the amphibian is selected from frogs, toads and salamanders. In one embodiment, the amphibian is a frog. In one embodiment, the amphibian is a toad. In one embodiment, the amphibian is a salamander.

In one embodiment, the animal is a reptile. In one embodiment, the reptile is selected from snakes, lizards, iguanas, turtles and crocodiles. In one embodiment, the reptile is a snake. In one embodiment, the reptile is a lizard. In one embodiment, the reptile is a turtle. In one embodiment, the reptile is a crocodile.

In one embodiment, the animal is a bird. In one embodiment, the bird is selected from poultry such as chickens, ducks, geese, turkeys, quail, guinea fowl, pigeons (including squabs) and birds of prey (including hawks, eagles, kites, falcons, vultures, harriers, ospreys, and owls). In one embodiment, the bird is selected from chickens, ducks, geese and turkeys. In one embodiment, the bird is poultry. In one embodiment, the bird is a chicken. In one embodiment, the bird is a broiler chicken. In one embodiment, the bird is a layer hen. In one embodiment, the bird is a duck. In one embodiment, the bird is a goose. In one embodiment, the bird is a turkey. In one embodiment, the bird is a quail. In one embodiment, the bird is a guinea fowl. In one embodiment, the bird is a pigeon. In one embodiment, the bird is a bird of prey.

In one embodiment, the animal is a monogastric animal. In one embodiment, the monogastric animal is selected from pigs or swine, such as piglets, growing pigs and sows, cats and dogs and rodents (rats, mice). In one embodiment, the monogastric animal is a pig. In one embodiment, the monogastric animal is a cat. In one embodiment, the monogastric animal is a dog. In one embodiment, the monogastric animal is rodent. In one embodiment, the monogastric animal is a mouse. In one embodiment, the monogastric animal is a rat.

In one embodiment, the animal is a ruminant. In one embodiment, the animal is a ruminant selected from cattle, sheep, goats, deer, yak, llama and kangaroo. Cattle include but are not limited to beef cattle, dairy cattle, cows and young calves. In one embodiment, the ruminant is selected from cattle, sheep, goats and deer. In one embodiment, the ruminant is a cow. In one embodiment, the ruminant is a beef cow. In one embodiment, the ruminant is a dairy cow. In one embodiment, the ruminant is a calf. In one embodiment, the ruminant is a sheep. In one embodiment, the ruminant is a goat. In one embodiment, the ruminant is a deer. In one embodiment, the ruminant is a llama. In one embodiment, the ruminant is a kangaroo.

In one embodiment, the animal is a pseudo-ruminant. In one embodiment, the pseudo-ruminant is selected from horses, camels, rabbits and guinea pigs. In one embodiment, the pseudo-ruminant is selected from horses, rabbits and guinea pigs. In one embodiment, the pseudo-ruminant is a horse. In one embodiment, the pseudo-ruminant is a rabbit. In one embodiment, the pseudo-ruminant is a camel. In one embodiment, the pseudo-ruminant is a guinea pig.

In the methods of the present disclosure, the administration may be by oral administration.

The frequency of administration of the extract derived from sugar cane or a composition, non-human animal formulated supplement or animal feed comprising the extract derived from sugar cane, may be as required to provide the desired improvement, maintenance, prevention and/or treatment. As would be understood by one of ordinary skill in the art, the frequency of administration of the extract derived from sugar cane or a composition, non-human animal formulated supplement or animal feed comprising the extract derived from sugar cane, may depend on the amount or dosage of the extract. A higher amount or dosage of the extract derived from sugar cane may result in less frequent administration being required. A lower amount or dosage of the extract derived from sugar cane may result in more frequent administration being required. The administration of the extract derived from sugar cane or a composition, non-human animal formulated supplement or animal feed comprising the extract derived from sugar cane, may be for a short period or for an extended or continuous period.

The frequency of administration may be daily, twice daily, thrice daily, every 1-3 days, every 1-5 days, weekly, fortnightly, monthly, bi-monthly, every 1-3 months, every 1-6 months, every 6 months, or yearly. In one embodiment, the frequency of administration is daily. In one embodiment, the frequency of administration is twice daily. In one embodiment, the frequency of administration is weekly. In one embodiment, the frequency of administration is fortnightly. In one embodiment, the frequency of administration is monthly. In one embodiment, the frequency of administration is bi-monthly. In one embodiment, the frequency of administration is every 1-3 months. In one embodiment, the frequency of administration is every 1-6 months. In one embodiment, the frequency of administration is every 6 months. In one embodiment, the frequency of administration is yearly.

It will be understood, however, that the specific dosage level and frequency of dosage for any particular subject may be varied and will depend upon a variety of factors including the activity of the specific extract derived from sugar cane employed, the activity of a veterinary composition, an non-human animal formulated supplement or animal feed comprising that extract, the metabolic stability and length of action of that extract derived from sugar cane, veterinary composition, non-human animal formulated supplement or animal feed, the age, body weight, general health, sex, diet, mode and time of administration, rate of excretion, drug combination, the severity of the particular condition, and the animal subject undergoing therapy.

The compositions, non-human animal formulated supplements, non-human animal feeds, methods or uses of the present disclosure may further comprise other active agents or compounds which improve or maintain animal health. Selection of the appropriate agents or compounds for use in combination may be made by one of ordinary skill in the art.

### EXAMPLES

**Example** 1 provides illustrative and non-limiting examples of characterisation of the extracts derived from sugar cane of the present disclosure.

### Example 1. Characterisation of extracts derived from sugar cane

In order to characterise the types and quantity of polyphenols in extracts derived from sugar cane, some extracts were analysed by Liquid Chromatography-Mass Spectrometry (LCMS) and by NMR spectroscopy.

The three sample extracts A, B, and C were fractions from molasses (Figure 1). All the samples were stored at -20°C.

**Table 1. Extract fractions from molasses**

| **Code** | **Sample Name** | **Description** |
|---|---|---|
| A | FPX66 bound fraction | Brown syrup |
| B | FPX66 unbound fraction | Light yellow syrup |
| C | 74 Brix | Dark brown syrup |

One mL of each of the samples were transferred into pre-weighed vials in duplicate and then freeze-dried for 3 days to obtain dry mass (Table 2). One replicate of each of the samples was analysed by NMR spectroscopy and the other replicate of each of the samples was used for quantitative analysis of polyphenols by LCMS.

**Table 2. Moisture content of samples**

| **Sample** | **Replicate** | **Wt. of 1.0 mL, g** | **Loss in wt., g** | **Wt. of dried extract, g** | **% Moisture** | **Analysis** |
|---|---|---|---|---|---|---|
| A: Bound Fraction | a | 1.0568 | 0.8471 | 0.2097 | 80.16 | NMR |
| | b | 1.0683 | 0.8559 | 0.2124 | 80.12 | LCMS |
| B: Unbound Fraction | a | 1.1324 | 0.7761 | 0.3563 | 68.54 | NMR |
| | b | 1.1288 | 0.7730 | 0.3558 | 68.48 | LCMS |
| C: 74 Brix Fraction | a | 1.0300 | 0.2491 | 0.7809 | 24.18 | NMR |
| | b | 1.1690 | 0.2751 | 0.8939 | 23.53 | LCMS |

The 74 Brix sample was fractionated by C18 solid phase extraction (SPE) to remove the sugars and obtain more concentrated phenolic components. One mL was diluted in Milli-Q water and eluted through a Waters 3 mL SPE Vac C18 cartridge that was initially activated with MeOH and then conditioned with Milli-Q water. The polar components were eluted with 6 mL Milli-Q water which was discarded. The remaining metabolites on the SPE cartridge were then eluted with 2 x 3 mL MeOH into a pre-weighed vial and the solvent was evaporated to dryness under nitrogen gas. The 74 Brix SPE-MeOH fraction was further dried overnight in the freeze dryer and then weighed to obtain the dry weight of fraction (55.6 mg). The extract was reconstituted in 200 µL 80:20 McOH-H₂O (concentration = 278 mg/mL) and analysed on the LCMS.

### Reference Standards

Table 3 lists the reference standards used for the qualitative analysis of phenolic compounds by LCMS. Standard solutions were prepared either in MeOH or 1:1 McOH-H₂O. Fourteen of the standards were used for quantitative analysis of phenolic compounds by LCMS and a range concentrations was prepared from stock solutions indicated in Table 3 using 80:20 MeOH-H₂O as diluent.

**Table 3. List of reference standards used for LCMS analysis**

| **Code** | **Compound** | **Molecular. Formula** | **Molecular Wt., g/mol** | **Stock Concentration, µg/mL** |
|---|---|---|---|---|
| ***S01*** | ***Syringic acid*** | C₉H₁₀O₅ | 198.17 | 6,000 |
| ***S02*** | ***Caffeic acid*** | C₉H₈O₄ | 180.16 | 600 |
| ***S03*** | ***Vanillin*** | C₈H₈O₃ | 152.15 | 60 |
| **S4** | ***Sinapic acid*** | C₁₁H₁₂O₅ | 224.21 | 115 |
| ***S5*** | ***Tricin*** | C₁₇H₁₄O₇ | 330.29 | 100 |
| ***S6*** | ***Chlorogenic acid*** | C₁₆H₁₈O₉ | 354.31 | 1,900 |
| ***S7*** | ***Diosmin*** | C₂₈H₃₂O₁₅ | 608.54 | 1,000 |
| ***S8*** | ***Diosmetin*** | C₁₆H₁₂O₆ | 300.26 | 100 |
| ***S9*** | ***Apigenin*** | C₁₅H_{1O}O₅ | 270.24 | 10 |
| ***S10*** | ***Vitexin*** | C₂₁H₂₀O₁₀ | 432.38 | 100 |
| ***S11*** | ***Orientin*** | C₂₁H₂₀O₁₁ | 448.38 | 90 |
| ***S12*** | ***Homoorientin*** | C₂₁H₂₀O₁₁ | 448.38 | 40 |
| ***S13*** | ***Swertisin*** | C₂₂H₂₂O₁₀ | 446.40 | 21 |
| ***S14*** | ***Myricetin*** | C₁₅H₁₀O₈ | 318.24 | 400 |

### Qualitative Analysis by Liquid Chromatography-Mass Spectroscopy (LCMS)

The samples were analysed by LCMS. The negative MS data was analysed using Genedata software and after pre-processing (RT restriction to exclude sugars, noise removal, cluster identification, *etc*.)*.* 4,250 features were identified across all samples. There were 4,196 features identified in sample A (FPX66 bound fraction), 1,127 in sample B (FPX66 unbound fraction), and 178 in C (74 Brix sample) (Figures 4 to 6).

A number of phenolic compounds were identified in the extracts by comparison to the standards analysed: vanillin, apigenin, orientin, vitexin, caffeic acid, chlorogenic acid, syringic acid, diosmin, swertisin, homoorientin, diosmetin, sinapic acid (trace amount), myricetin (trace amount), tricin (trace amount).

Table 4 exhibits polyphenol amounts in an extract derived from sugar cane molasses from LCMS analysis in µg/gram dry weight basis.

**Table 4. Polyphenol amounts in sample extracts derived from sugar cane molasses of the present disclosure.**

| **Polyphenol** | **74 Brix sample (C) in µg/g** | **FPX66 bound sample (A) in µg/g** |
|---|---|---|
| Syringic Acid | 10.9 | 107.57 |
| Caffeic Acid | 0.29 | 7.54 |
| Vanillin | 0.153 | 2.13 |
| Sinapic Acid | 0.18 | 1.73 |
| Tricin | 0.03 | 0.4 |
| Chlorogenic Acid | 6.53 | 74.29 |
| Diosmin | 19.45 | 227 |
| Diosmetin | 0.15 | 0.16 |
| Apigenin | 0.001 | 0.01 |
| Vitexin | 0.084 | 1.62 |
| Orientin | 0.245 | 4.5 |
| Homoorientin | 0.041 | 0.58 |
| Swertisin | 0.69 | 5.25 |

Table 5 exhibits characteristics and components of various extracts derived from sugar cane.

**Table 5. Two example extracts derived from sugar cane molasses of the present disclosure Extract 1 is comparative.**

| | **Extract 1** | **Extract 2** |
|---|---|---|
| Brix | 65-70 Brix | 68-70 Brix |
| pH | 4-5 | 4.5-4.7 |
| Density | 1.25-1.35 | 1.35 |
| Colour Absorbance 420 | 69.1 | 65 |
| Absorbance 270 | 708 | 1506 |
| Ratio A270/A420 | 10 | 23 |
| Total Polyphenol (mg/L gallic acid equivalent) | 16,500 | 24000-28,000 |
| Total Flavonoid (mg/L catechin equivalent) | 2800 | 5900 |
| Conductivity (uS/cm) | 138,800 | 57,200 |
| Calcium (mg/kg) | 5100 | 1614 |
| Iron (mg/kg) | 110 | 52 |
| Magnesium (mg/kg) | 1800 | 2250 |
| Potassium (mg/kg) | 26,000 | 21,000 |
| Sodium (mg/100g) | 23 | 47 |

Table 6 exhibits a component comparison between molasses and extracts derived from sugar cane of the present disclosure.

**Table 6. Comparison between molasses and sugar cane extracts of the present disclosure**

| **Components** | **Molasses** | **Sugar cane extracts** |
|---|---|---|
| Total solids (g/L) | 80.5 | 70 |
| Fructose (g/L) | 131.9 | 74.5 |
| Glucose (g/L) | 107.1 | 52.8 |
| Sucrose (g/L) | 473.8 | 343 |
| Total sugars (g/L) | 722.8 | 470 |
| Ratio (Fructose+Glucose/Sucrose) | 0.50 | 0.41 |
| Total polyphenol (mg GAE/L ) | 20,000 | 25,000-28,000 |
| Antioxidants (ABTS mg GAE/L) | 7,000-8,500 | 10,500-11,500 |
| Calcium (mg/L) | 5746 | 2145 |
| Magnesium (mg/L) | 2374 | 3003 |
| Sodium(mg/L) | 303 | 605 |
| Potassium (mg/L) | 20,794 | 27170 |

Table 7 exhibits the mineral concentration of extracts derived from sugar cane of the present disclosure prepared according to the process of Figure 1 in mg/kg on a dry weight basis. The concentration of selenium and chromium is shown in µg/kg on a dry weight basis.

**Table 7. Mineral composition of representative sample extracts derived from sugar cane molasses of the present disclosure**

| **Anions** | **74 Brix Sample (C)** | **FPX66 bound sample (A)** | **FPX66 bound sample (A)** |
|---|---|---|---|
| Potassium | 26,000 mg/kg | 100-250 mg/kg | 190 mg/kg |
| Sodium | 450 mg/kg | 10-50 mg/kg | 30 mg/kg |
| Calcium | 1,090 mg/kg | 8,000-9,000 mg/kg | 8,800 mg/kg |
| Magnesium | 4,700 mg/kg | 1,500-2,500 mg/kg | 2,000 mg/kg |
| Iron | 65 mg/kg | 800-1000 mg/kg | 890 mg/kg |
| Zinc | 6.6 mg/kg | Not detected | Not detected |
| Selenium (µg/kg) | 786 µg/kg | Not detected | Not detected |
| Chromium (µg/kg) | 1,300 µg/kg | Not detected | Not detected |

**Example 2** to **Example 12** provide illustrative and non-limiting examples of the preparation and characterisation of extracts derived from sugar cane of the present disclosure.

### Example 2. Sugar cane extracts derived from molasses

Example sugar cane extracts of the present disclosure were prepared from molasses as follows.

Sugar cane molasses was diluted with de-ionised water, mixed well to give a final Brix of 50°. This mixture was held between 20-25 °C and 95% food grade ethanol added with overhead stirring to ensure that the ethanolic mixture was evenly and quickly dispersed. This step was continued until the final ethanol content reached 76% v/v. During this time, a gelatinous precipitate formed. The precipitate was allowed to settle and the supernatant was decanted and filtered under vacuum in a Buchner Funnel through a Whatman GFA filter paper grade 1. The ethanol was subsequently removed under reduced pressure in a Buchi Rotary Evaporator at 45 °C. Evaporation was continued under reduced pressure at 50-55 °C to give a syrup with a final Brix of 70 ° with a bitter sweet aroma. Characterisation of exemplary syrups obtained by this method is shown in Table 8.

**Table 8. Properties of sugar cane extracts prepared from molasses Extract 1 is comparative.**

| **Property** | **Extract 1** | **Extract 2** |
|---|---|---|
| Brix (°Bx) | 65-70 | 70° (+/- 2) @ 20°C |
| pH | 4-5 | 4.6 (+/- 0.2) @ 20°C |
| Density (g/mL) | 1.25-1.35 | 1.35 (+/- 0.05 @ 20°C) |
| Colour Absorbance 420 | 69.1 | - |
| Absorbance 270 | 708 | - |
| Ratio A270/A420 | 10 | - |
| Total Polyphenol (mg/L as gallic acid equivalents) | 16,500 | Minimum 20, 000 |
| Total Flavonoids (mg/L as catechin equivalents) | 2800 | Minimum 7,000 |
| ORAC 5.0 | - | Minimum 2.5 mol/kg |
| CAA | - | Minimum 2.5 mol/kg as trolox equivalents |
| Conductivity (µS/cm) | 138,800 | - |
| Calcium | 5100 mg/kg | 400 - 1,300 mg/L |
| Iron | 110 mg/kg | 10 - 100 mg/L |
| Magnesium | 1800 mg/kg | 2,400 - 5,500 mg/L |
| Potassium | 26,000 mg/kg | 20,000 - 40,0000 mg/L |
| Sodium | 23 mg/100 g | 60 - 80 mg/100 mL |
| Zinc | - | 0.3 - 0.8 mg/100 mL |
| Selenium | - | 0.03 - 0.09 mg/100 mL |
| Chromium | - | 0.03 - 0.140 mg/100 mL |

### Example 3. Fractionated sugar cane extracts derived from molasses

In general, the title fractionated sugar cane extracts may be prepared using hydrophobic chromatography procedures. Extracts prepared using the processes described in Example 2 and any sugar cane derived product may be used as feedstocks for chromatography. The hydrophobic resin used for chromatography may be a food grade resin.

In a representative preparation, FPX66 resin (Dow, Amberlite FPX66, food grade)) was pre-treated by washing with de-ionised water, ethanol and then finally with de-ionised water following the manufacturer's instructions. The washed resin was filtered under vacuum through a Buchner Funnel using Whatman filter paper grade 1 (1 µm pore size). The resin granules were then used as is.

De-ionised water was added to sugar cane molasses with constant stirring until the Brix reached 20 °. To a beaker containing 1 litre of the 20 ° Brix feedstock (maintained at 20-25 °C) and mounted on a magnetic stirrer, 500 g of wet weight pre-treated resin was added with gentle stirring to ensure effective mixing of the resin granules with the feedstock. The mixing was continued for 10 min at which point the mixture was filtered under vacuum and the resin was collected.

The collected resin was washed by resuspension in de-ionised water (1 litre). This step was repeated.

The washed resin was then suspended in 1 litre 70% ethanol solution in de-ionised water, stirred for 10 mins and the filtrate was collected by vacuum filtration. This was repeated twice more with 1 litre batches of the 70% ethanolic solution with each filtrate being collected. Finally, the three 70% ethanolic filtrates were combined and the ethanol removed by evaporation under reduced pressure. The aqueous fraction was lyophilised or spray-dried into a free flowing brown powder with a moisture content of 0.3-2.0% w/w.. The properties of the ethanolic fraction are shown below in Table 9.

**Table 9. Properties of extract derived from sugar cane molasses**

| **Properties** | **Ethanol fraction** |
|---|---|
| Colour Absorbance at 420nm | 10 (1% in solution @ 20°C) |
| Absorbance at 270nm | 180 (1% in solution @ 20°C) |
| Ratio A270nm/A420nm | 19 (1% in solution @ 20°C) |
| Total Polyphenol (mg/g gallic acid equivalent) | Minimum 200 |
| Total Flavonoid (mg/g catechin equivalent) | Minimum 50 |
| Calcium (mg/kg) | 840 |
| Iron (mg/kg) | 77 |
| Magnesium (mg/kg) | 2300 |
| Potassium (mg/kg) | 1100 |
| Sodium (mg/g) | 1700 |
| Zinc (mg/kg) | 48 |
| Selenium (mg/kg) | 0.18 |
| Chromium (mg/kg) | 1.8 |

Figure 5 exhibits a LC-MS spectrum of a representative extract derived from sugar cane molasses using this process.

### Example 4. Sugar cane extracts derived from dunder

A scheme for the preparation of the title sugar cane extracts is shown in Figure 2.

Sugar cane dunder was allowed to settled overnight for eight hours in a V - bottom tank. The supernatant was then subjected to sequential microfiltration through: (i) a 5 micron filter; (ii) a 1 micron filter; (iii) a 0.5 micron filter; and (iv) a 0.1 micron filter.

The filtered supernatant was subsequently concentrated in a heat exchanger to remove water to provide the liquid extract with 55 °Bx.

The properties of an extract derived from dunder is shown below in Table 10.

**Table 10. Properties of extract derived from dunder**

| **Properties** | **Sugar cane extract** |
|---|---|
| Brix | 55° (+/- 2) @ 20°C |
| pH | 4.6 (+/- 0.2) @ 20°C |
| Density | 1.28 g/mL (+/- 0.05) @ 20°C |
| Colour Absorbance 420 | 190 - 280 |
| Absorbance 270 | 2300 - 3000 |
| Ratio A270/A420 | 10 - 15 |
| Total Polyphenol (mg/L as gallic acid equivalent) | Minimum 45,000 |
| Total Flavonoid (mg/L as catechin equivalent) | Minimum 10,000 |
| Conductivity (uS/m) | 250,000 - 350,000 |
| Calcium (mg/kg) | 3,000 - 4,000 |
| Iron (mg/kg) | 100 - 150 |
| Magnesium (mg/kg) | 3,000 - 5,000 |
| Potassium (mg/kg) | 30,000 - 40,000 |
| Sodium (mg/kg) | 2,000 - 3,000 |
| Zinc (mg/100 g) | 0.5 - 1.5 |
| Selenium (mg/100 g) | 0.02 - 0.05 |
| Chromium (mg/100 g) | 0.20 - 0.5 |

Figure 6 exhibits example LC-MS spectra for sugar cane dunder starting material (A) and an extract of sugar cane derived dunder (B) in accordance with the above process.

### Example 5. Hybrid sugar cane extracts derived from a combination of sugar cane molasses and dunder.

A scheme for the preparation of the title sugar cane extracts is shown in Figure 3. Sugar cane mill molasses was diluted with water and mixed with settled sugar cane dunder (as described above) and stirred well to provide a mixture with 50 °Bx. The combined mixture of molasses and dunder was maintained at a constant temperature of between 20-25 °C and 95% food grade ethanol added and stirred to ensure that the ethanol was evenly and quickly dispersed. Ethanol was added until the ethanol level was 76% v/v

The addition and mixing of ethanol led to the formation of a gelatinous precipitate. The precipitate in the mixture was allowed to settle and the supernatant was removed by decantation and vacuum filtration in a Buchner funnel through a Whatman GFA filter paper 1.

The ethanol was removed from the supernatant under vacuum in a Buchi rotary evaporator at 45 °C. Evaporation of water from the supernatant was performed under vacuum at 50-55 °C until the final syrup reaches 70 °Bx.

Table 11 shows the properties of the hybrid sugar cane extract obtained.

**Table 11. Properties of hybrid sugar cane extract**

| **Property** | **Hybrid sugar cane extract** |
|---|---|
| Brix | 70° (+/- 2) @ 20°C |
| pH | 4.6 (+/- 0.2) @ 20°C |
| Density | 1.35 (+/- 0.05 @ 20°C) |
| Colour Absorbance 420 | 90 - 120 |
| Absorbance 270 | 1900 - 2300 |
| Ratio A270/A420 | 15 - 30 |
| Total Polyphenol (mg/L as gallic acid equivalent) | Min 30,000 milligrams per litre (as gallic acid equivalents) |
| Total Flavonoid (mg/L as catechin equivalent) | Minimum 10,000 |
| Conductivity (uS/m) | 180,000 - 200,000 |
| Calcium (mg/kg) | 80 - 160 |
| Iron (mg/kg) | 2-8 |
| Magnesium (mg/kg) | 300 - 600 |
| Potassium (mg/kg) | 2000 - 4000 |
| Sodium (mg/kg) | 60 - 180 |
| Zinc (mg/100 g) | 1.5 - 3.0 |
| Selenium (mg/100 g) | 0.04 - 0.09 |
| Chromium (mg/100 g) | 0.015 - 0.50 |

**Example 6** to **Example 12** provide illustrative and non-limiting examples of characterisation of the anti-inflammatory and/or anti-oxidant activity of extracts derived from sugar cane of the present disclosure.

### Example 6. Nuclear factor κB study

### Description

Nuclear Factor κB (NF-κB) is a protein complex that is involved in cellular responses to stimuli such as stress and free radicals, ultraviolet irradiation, oxidized LDL, and bacterial or viral antigens. It plays a key role in regulating the immune response to infection. Suppression of NF-κB limits the production of pro-inflammatory gene expression and reduces the level of inflammation, therefore inhibition of NF-κB is used as an indicator of anti-inflammatory activity.

### Methodology

The assay of NF-κB inhibition follows a procedure where a test material is absorbed into human cells. A proinflammatory cytokine is then introduced to the human cells to mimic cellular stress, which would normally induce NF-κB activation leading to inflammation. If an NF-κB inhibiting material is present in the cellular environment, the material inhibits NF-κB activation and the degree of inhibition can be monitored via NF-κB expression. NF-κB expression level of the human cells, treated with and without the test material, under the stressed condition are therefore monitored and compared to assess the NF-κB inhibition effect of a material.

Human cells were first treated with or without a representative powdered extract derived from sugar cane of the present disclosure (extract of Example 3) to allow for natural absorption of the extract into the cells. Maximum percentage of NF-κB expression inhibition induced by the powdered extract was reported as with the concentration used that induced the maximum inhibition of NF-κB expression. The half maximal inhibitory concentration (EC₅₀) was calculated. Assay results are shown in Table 12.

**Table 12. Nuclear factor κB activation assay results**

| **Sugar cane extract of Example 3** | **Inhibition (%)** | | |
|---|---|---|---|
| Conc. (µg/mL) | Replicate 1 | Replicate 2 | Average |
| 178.13 | 92.04 | 104.19 | 98.12 |
| 89.06 | 66.45 | 76.33 | 71.39 |
| 44.53 | 53.73 | 58.42 | 56.08 |
| 22.27 | 12.37 | 15.49 | 13.93 |
| 11.13 | 7.96 | 14.00 | 10.98 |
| 5.57 | 4.83 | 13.86 | 9.35 |

The response curve for this data is shown in Figure 9. A maximum inhibition of 98.12 % was observed. The concentration that induced the maximum inhibition was 178 µg/mL. The calculated half-maximal response (EC₅₀) was calculated to be 632.1 µg/mL. This data demonstrates that the extract derived from sugar cane of the present disclosure inhibits NF-κB indicating anti-inflammatory activity.

### Example 7. Cellular Nrf2 activation assay

### Description

Cellular Antioxidant Assay (Nrf2) can be used to determine the potential of an extract derived from sugar cane of the present disclosure to stimulate the production of Nrf2 in human cells. Nrf2 serves as a biomarker for anti-oxidation and anti-inflammatory capacity (Maes, M. *et al.,* 2012; Tan, S. M. and de Haan, J. B, 2014).

Nuclear erythroid 2-related factor (Nrf2) is a redox-sensitive sensitive transcription factor that binds to antioxidant response elements (ARE) to regulate the expression of antioxidant enzymes that protect against oxidative damage triggered by injury and inflammation. Activation of the Nrf2 pathway has been found to prevent and treat a large number of chronic inflammatory diseases. A number of natural occurring phytonutrients such as resveratrol, sulforaphane, and curcumin have been reported to activate Nrf2, with more phytonutrient Nrf2 activator discoveries being the focus of investigation. Nrf2 has been investigated as biomarker for regulating *in vivo* anti-oxidation and anti-inflammation response.

### Methodology

Human cells were treated with or without a representative powdered extract derived from sugar cane of the present disclosure (extract of Example 3), and the impact of the extract on Nrf2 activation was monitored. The concentration of the extract that gives half-maximal response (EC₅₀) was calculated. The assay results are shown in Table 13.

**Table 13. Cellular Nrf2 activation assay results**

| **Nrf2 pathway activity** | | | | |
|---|---|---|---|---|
| Conc. (µg/mL) of powdered extract of Example 3 | Replicate 1 | Replicate 2 | Average | Standard deviation |
| 500 | 73715.08 | 53623.08 | 63669.08 | 14207.189 |
| 250 | 20837.08 | 31168.08 | 26002.58 | 7305.120 |
| 125 | 12246.08 | 6352.08 | 9299.08 | 4167.687 |
| 62.5 | 2279.08 | 3046.08 | 2662.58 | 542.351 |
| 31.25 | 1466.08 | 1247.08 | 1356.58 | 154.856 |
| 15.625 | 1220.08 | 1072.08 | 1146.08 | 104.652 |

The response curve for this data is shown in Figure 10. The calculated half-maximal response (EC₅₀) was calculated to be 632.1 µg/mL. This data demonstrates that the extract derived from sugar cane of the present disclosure activates Nrf2 and therefore has anti-oxidation and anti-inflammatory capacity.

### Example 8. TNF-α study

### Description

Tumor necrosis factor (TNF)-α is a pro-inflammatory cytokine (small proteins that impact cell signalling) that triggers downstream cellular feedback loops governing inflammation. TNF-α has been identified as an inflammation trigger and precursor. Thus, TNF-α inhibitors have potential as anti-inflammatory agents.

### Methodology

Human cells are first treated with or without powdered sugar cane extract (extract of Example 3) to allow for natural absorption of the material into the cells. Then, the cells are stressed with an inflammation inducer, which would normally stimulate TNF-α production then further develop into inflammation through series of cellular signalling. If a TNF-α inhibitor presents in the cellular environment, the material inhibits TNF-α production and the degree of inhibition is assessed by level of decreased TNF-α production. TNF-α production level of the human cells, treated with and without a test material, under the stressed condition is monitored and compared to assess the TNF-α inhibition effect of the test material. The maximum percentage of TNF-α expression inhibition induced by the tested sugar cane extracts was reported as was the concentration used that induced the maximum inhibition of TNF-α expression. Assay results are shown in Table 14.

**Table 14. Cellular TNFα inhibition assay results**

| **Sugar cane extract of Example 3** | **Inhibition (%)** | | |
|---|---|---|---|
| Conc. (µg/mL) | Replicate 1 | Replicate 2 | Average |
| 178.13 | 99.20 | 94.80 | 97.00 |
| 89.06 | 92.36 | 92.66 | 92.51 |
| 44.53 | 59.36 | 59.94 | 59.65 |
| 22.27 | 20.00 | 30.35 | 25.18 |
| 11.13 | 13.65 | 10.63 | 12.14 |
| 5.57 | -3.73 | 5.06 | 0.66 |

The response curve for this data is shown in Figure 11. A maximum inhibition of 97 % was observed. The concentration that induced the maximum inhibition was 178 µg/mL. The calculated half-maximal response (IC₅₀) was calculated to be 36.31 µg/mL. This data demonstrates that the extract derived from sugar cane of the present disclosure inhibits TNF-α indicating anti-inflammatory activity.

### Example 9. Prostaglandin E₂ (PGE₂) study

### Description

PGE₂ is a primary product of arachidonic acid metabolism controlled by cyclooxygenase enzymes. It plays a critical role in increasing vascular permeability, fever generation, and tumor growth. Drugs used to inhibit PGE₂ synthesis have shown to control inflammation, pain and fever.

Assaying the inhibition of PGE₂ expression follows a procedure whereby a test material is absorbed into mammalian cells. Cells are stressed with an inflammation inducer, which would normally stimulate PGE₂ production that would further develop into inflammation through series of cellular signalling. However, if a PGE₂ inhibitor is presents in the cellular environment, the material inhibits PGE₂ production and the degree of inhibition is assessed by level of decreased PGE₂ production. PGE₂ production level in cells, treated with and without a test material, under the stressed condition is monitored and compared to assess the PGE₂ inhibition effect of the test material.

### Methodology

Mammalian cells were first treated with or without powdered sugar cane extract (extract of Example 3) to allow for natural absorption of the material into the cells. The maximum percentage of PGE₂ expression inhibition induced by the powdered extract and the concentration used that induced the maximum inhibition of PGE₂ expression were reported. The half maximal effective concentration (EC₅₀) was calculated. The assay results are shown in Table 15.

**Table 15. Cellular PGE₂ inhibition assay results**

| **Conc. of powdered sugar cane extract of Example 3 (µg/mL)** | **Inhibition of PGE2 (%)** |
|---|---|
| 183.13 | 58.29 |
| 91.56 | 44.08 |
| 45.78 | 45.72 |
| 22.89 | 30.97 |
| 11.45 | 24.41 |
| 5.72 | -8.38 |

The response curve for this data is shown in Figure 12. A maximum inhibition of 58.29 % was observed. The concentration that induced the maximum inhibition was 183.13 µg/mL. The calculated half-maximal response (IC₅₀) was calculated to be 91.62 µg/mL. This data demonstrates that the extract derived from sugar cane of the present disclosure inhibits PGE₂ indicating anti-inflammatory activity.

### Example 10. Cyclooxygenases-1 (COX-1) and cyclooxygenases-2 (COX-2) inhibition assays

### Description

Cyclooxygenases-1 (COX-1) inhibitors are among the important targets for treatment of inflammation related diseases. COX has two well-known isoforms, COX-1 and COX-2, which are similar in their amino-acid sequences and identity. COX-2 predominates at sites of inflammation, and COX-1 is constitutively expressed in the gastrointestinal tract. It is reported that selective COX-2 inhibitors can target inflammation and pain with reduced risk of chronic ulceration and acute injury (Hawkey, C. J, 2001).

### Methodology

COX-1 and COX-2 inhibition assays were used to assess the inhibition capability of representative powdered extracts derived from sugar cane of the present disclosure (extract of Example 3) by monitoring the extracts' impact on the activity of a specific COX enzyme. The assays compare the enzymatic activity of the target COX in the presence with and without the material of interest to determine the inhibition potential of the material. The results were expressed as the concentration of the tested material used to achieve 50% of COX inhibition (IC₅₀), if 50% of inhibition has been achieved. If the IC₅₀ value could not be calculated, the maximum percentage of COX inhibition achieved, and the concentration of the material used that induced the maximum inhibition were reported. COX-1 and COX-2 results are shown in Table 16 and Table 17 respectively.

**Table 16. COX-1 assay results**

| **COX-1 inhibition (%)** | | | | |
|---|---|---|---|---|
| Conc. (µg/mL) | Replicate 1 | Replicate 2 | Average | Standard deviation |
| 500.00 | 16.73 | 6.62 | 11.68 | 7.15 |
| 250.00 | -3.67 | 4.83 | 0.58 | 6.01 |
| 125.00 | 5.73 | -5.65 | 0.04 | 8.05 |
| 62.50 | 1.14 | 10.11 | 5.63 | 6.35 |
| 31.25 | 4.83 | 6.62 | 5.72 | 1.27 |
| 15.63 | 8.38 | 3.92 | 6.15 | 3.16 |

The inhibition curve for the COX-1 data is shown in Figure 13 panel A. The maximum inhibition is 11.68 % and the calculated effective concentration at maximum inhibition is 500.00 µg/mL.

**Table 17. COX-2 assay results**

| **COX-2 inhibition (%)** | | | | |
|---|---|---|---|---|
| Conc. (µg/mL) | Replicate 1 | Replicate 2 | Average | Standard deviation |
| 500.00 | 46.52 | 44.98 | 45.70 | 1.02 |
| 250.00 | 25.78 | 30.20 | 27.99 | 3.13 |
| 125.00 | 18.97 | 26.43 | 22.70 | 5.27 |
| 62.50 | 11.56 | 9.99 | 10.78 | 1.10 |
| 31.25 | 13.09 | 9.99 | 11.54 | 2.19 |
| 15.63 | 9.20 | 6.79 | 8.00 | 1.71 |

The inhibition curve for the COX-2 data is shown in Figure 13 panel B. The maximum inhibition was observed to be 47.70 %. The calculated effective concentration at maximum inhibition was 500.00 µg/mL.

This demonstrates that the extract of sugar cane of the present disclosure was selective for COX-2 over COX-1. Further, this data demonstrates that the extract derived from sugar cane of the present disclosure inhibits COX-2 indicating anti-inflammatory activity.

### Example 11. Cellular anti-oxidant assay (CAA)

### Description

CAA analyses the capacity of a material to protect a fluorescent probe (as a marker) from damage by reactive oxygen species (ROS) in intracellular environment. In this assay, peroxyl radical is used as the ROS, and human liver cells are used as the cellular model. Quercetin is used as the standard, and the results are expressed as µmole quercetin equivalency (QE) per gram of the sugar cane extracts tested. The CAA results for 5 extracts derived from sugar cane of the present disclosure are set out in Table 18. Extracts 1, II, III and IV were produced according to Example 2 and extract V was produced according to Example 3.

**Table 18. CAA results for 5 extracts derived from sugar cane of the present disclosure**

| **Sugar cane extract** | **Results (µmol QE/gram)** |
|---|---|
| Extract I | 48.16 |
| Extract II | 56.21 |
| Extract III | 61.37 |
| Extract IV | 67.35 |
| Extract V | 229.12 |

CAA is used to observe the antioxidant capabilities of a substance in a living cellular context, rather than as an abstract chemical reaction. This technique is designed to give a detailed understanding of the mechanisms, bioavailability, uptake, and metabolism of the antioxidant compounds in a cell culture environment that reflects the complexity of a biological system. A high CAA value indicates that an antioxidant compound has been able to enter the cell which indicates bioavailability, without negatively affecting the cell which would indicate toxicity. As a reference, the Kakadu Plum (*Terminalia ferdinandiana*) has been suggested to have the highest Vitamin C concentration of any fruit in the world (Brand *et al.* 1982). Consequently, it is acknowledged to be an extremely efficient antioxidant. Kakadu Plum has been reported to return a CAA value of 71.5 ± 11.3 QE/gram (Tan *et al.* 2011). The sugar cane extracts disclosed returned CAA values slightly lower, within or significantly higher than this range. This demonstrates that the sugar cane extracts of the present disclosure provide powerful antioxidant protection in both *in vitro* and *in vivo* contexts.

### Example 12. Oxygen Radical Absorbance Capacity (ORAC) testing

### Description

ORAC tests are among the most acknowledged methods that measure antioxidant scavenging activity against oxygen radicals that are known to be involved in the pathogenesis of aging and many common diseases (Ou *et al.* 2001A; Huang *et al.* 2002; Ou *et al.* 2002; Dubost *et al.* 2007; Zhang *et al.* 2009; US 7,132,296). ORAC 5.0 consists of five types of ORAC assays that evaluate the antioxidant capacity of a material against five primary reactive oxygen species (ROSs, commonly called "oxygen radicals") found in humans: peroxyl radical, hydroxyl radical, peroxynitrite, superoxide anion and singlet oxygen. The ORAC 6.0 test adds in measurement of anti-oxidant scavenging activity against hypochlorite. Thus, the ORAC 5.0/6.0 tests are comprehensive panels that evaluate anti-oxidant capacity.

The tests work on the principle of measuring an anti-oxidant's capacity to preserve a probe from ROS degradation. A ROS inducer is introduced to the assay system. The ROS inducer triggers the release of a specific ROS, which would degrade the probe and cause its emission wavelength or intensity change. However, when an antioxidant is present in the system, the antioxidant absorbs the ROS and preserves the probe from degradation. The degree of probe preservation indicates the anti-oxidant capacity of the material.

### Methodology

The ORAC 5.0/6.0 tests undertaken evaluated the capacity of four representative sugar cane extracts of the present disclosure (Extracts A to D) to protect a probe (a fluorescent probe or chromagen) from its damage by ROSs. Trolox is used as the reference standard, and the results are expressed as µ mole Trolox equivalency (TE) / gram of the tested sugar cane extract. Extracts A to C were produced according to Example 2 and extract D was produced according to Example 3.

**Table 19. ORAC results for 4 extracts derived from sugar cane of the present disclosure**

| | **Results** | | | |
|---|---|---|---|---|
| **Analysis** | Extract A | Extract B | Extract C | Extract D |
| ORAC against peroxyl radicals | 303 | 258 | 265 | 2,336 |
| ORAC against hydroxyl radicals | 1,902 | 1,179 | 1,220 | 13,785 |
| ORAC against peroxynitrite | 25 | 32 | 34 | 255 |
| ORAC against super oxide anion | 121 | 82 | 74 | 450 |
| ORAC against singlet oxygen | 348 | 279 | 263 | 2,011 |
| ORAC 5.0 (sum of above) | 2,699 | 1,830 | 1,856 | 18,837 |
| ORAC against hypochlorite | - | 94 | 107 | 620 |
| ORAC 6.0 (sum of above) | - | 1,924 | 1,963 | 19,457 |

The data in Table 19 demonstrates that the extracts of the present disclosure are efficient at scavenging 6 well-characterised and biologically relevant oxidants. The individual ORAC values against each oxidant and the combined total ORAC 6 value demonstrates that the extracts of the present disclosure are powerful antioxidants against a range of oxidant species of biological significance.

**Example 13** to **Example 16** provide illustrative and non-limiting examples of activities of the extracts derived from sugar cane, animal supplements and animal feeds of the present disclosure in improving or maintaining the health of animals (fish, chickens, cats, pigs and horses respectively) to the benefit of improved food production and food quality.

### Example 13. Fish

### Description

Pangus (*Pangasius hypophthalmus*) and tilapia (*Oreochromis niloticus*) are the most and second most cultured aquaculture finfish species throughout the world. Pangus culture holds the largest aquaculture industry throughout the world. Attention is growing in, for example, Bangladesh to promote pangus farming for supplying sustainable protein. Tilapia can easily adapt in tropical and sub-tropical regions of the world and hence it is regarded as an important fish species that can reduce the gap of increasing worldwide demand for protein sources from fish.

Prawn *(Macrobrachium rosenbergii)* is one of the freshwater species of crustacean possessing high potential and market demand. At present, there is significant decline of catch from natural stocks and harvest has diminished owing to indiscriminate fishing. Hence, freshwater prawn obtained culture is important as a source of the highly valued prawn products for international markets.

### Study objective

The study assessed the effects of an extract derived from sugar cane of the present disclosure on pangus and tilapia finfish and prawn growth performance including FCR. The proximate composition, taste, flavour, odor of fish flesh were also assessed. In addition, the study investigated the palability/acceptance of feed.

### General methodology

General water quality parameters (temperature, pH, Dissolve oxygen, turbidity) were monitored to ensure proper environment for the cultured species. The data on growth performance, survival rate, feed conversion ratio (FCR), were collected on weekly basis. Data on proximate composition, taste of fish muscle and economic analysis were collected at the last period of the research. All the data were entered into MS Excel. Data management and data analysis was undertaken using the statistics software package SPSS (IBM).

### Pangus and tilapia methodology

Pangus *(Pangasius hypophthalmus)* and tilapia (*Oreochromis niloticus*) were cultured for four months in 32 constructed for purpose cages. The size of the cages was 26 feet x 12 feet. Each cage was covered in nylon ropes to prevent birds from eating the fish. The fry of pangus and tilapia finfish were collected from the supplier Halda Fisheries Ltd., Potenga, Chittagong, Bangladesh and were examined to ensure good quality seed.

The study used four treatment groups: Tₒ - control (no sugar cane extract in feed); T₁ - sugar cane extract included in feed in an amount of 0.2 w/w % (about 60 mg of total polyphenol (TPP) per kg of feed); T₂ - sugar cane extract included in feed at 0.4 w/w % (about 120 mg TPP / kg feed); T₃ - sugar cane extract included in feed at 0.6 w/w % (about 180 mg TPP / kg feed). Each treatment group included four cages for replication of experiments. Layout of the experiments showing the distribution of pangus and tilapia in cages and the applied treatments are shown in Tables 20 and 21.

**Table 20. Layout of pangus**

| **Treatment groups** | **Treatment×Replication (Tₙ×Rₙ)** | **No. of fishes per cage** | **Total No. of fish per treatments** |
|---|---|---|---|
| T₀ | T₀R₁(Cage No.01) | 50 | 200 |
| | T₀R₂(Cage No.02) | 50 | |
| | T₀R₃(Cage No.03) | 50 | |
| | T₀R₄(Cage No.04) | 50 | |
| T₁ | T₁R₁(Cage No.05) | 50 | 200 |
| | T₁R₂(Cage No.06) | 50 | |
| | T₁R₃(Cage No.07) | 50 | |
| | T₁R₄(Cage No.08) | 50 | |
| T₂ | T₂R₁(Cage No.09) | 50 | 200 |
| | T₂R₂(Cage No. 10) | 50 | |
| | T₂R₃(Cage No.11) | 50 | |
| | T₂R₄(Cage No.12) | 50 | |
| T₃ | T₃R₁(Cage No.13) | 50 | 200 |
| | T₃R₂(Cage No.14) | 50 | |
| | T₃R₃(Cage No.15) | 50 | |
| | T₃R₄(Cage No.16) | 50 | |
| Grand total | | | 800 |

**Table 21. Layout of tilapia**

| **Treatment groups** | **Treatment×Replication (Tₙ× Rₙ)** | **No. of fishes per cage** | **Total No. of fish per treatments** |
|---|---|---|---|
| T₀ | T₀R₁ (Cage No.17) | 80 | 320 |
| | T₀R₂ (Cage No.18) | 80 | |
| | T₀R₃(Cage No.19) | 80 | |
| | T₀R₄ (Cage No.20) | 80 | |
| T₁ | T₁R₁ (Cage No.21) | 80 | 320 |
| | T₁R₂ (Cage No.22) | 80 | |
| | T₁R₃ (Cage No.23) | 80 | |
| | T₁R₄ (Cage No.24) | 80 | |
| T₂ | T₂R₁ (Cage No.25) | 80 | 320 |
| | T₂R₂ (Cage No.26) | 80 | |
| | T₂R₃ (Cage No.27) | 80 | |
| | T₂R₄ (Cage No.28) | 80 | |
| T₃ | T₃R₁ (Cage No.29) | 80 | 320 |
| | T₃R₂ (Cage No.30) | 80 | |
| | T₃R₃ (Cage No.31) | 80 | |
| | T₃R₄ (Cage No.32) | 80 | |
| Grand total | | | 1280 |

### Prawn methodology

Prawn/Golda chingri (*Macrobrachium rosenbergii*) were cultured in 12 tanks (each treatment requires 3 tanks for replication). The tanks were rectangular in shape with proper aeration system and water exchanging capacity. The layout of the experiment showing the distribution of prawns in the tanks and the applied treatments is shown in Table 22.

**Table 22. Prawn layout**

| **Treatment groups** | **Treatment×Replication (Tₙ× Rₙ)** | **No. of prawn per tank** | **Total No. of prawn per treatment** |
|---|---|---|---|
| T₀ | T₀R₁(tank no.1) | 50 | 150 |
| | T₀R₂ (tank no.2) | 50 | |
| | T₀R₃(tank no.3) | 50 | |
| T₁ | T₀R₁(tank no.4) | 50 | 150 |
| | T₀R₂(tank no.5) | 50 | |
| | T₀R₃(tank no.6) | 50 | |
| T₂ | T₀R₁ (tankno.7) | 50 | 150 |
| | T₀R₂(tank no.8) | 50 | |
| | T₀R₃(tank no.9) | 50 | |
| T₃ | T₀R₁ (tankno.10) | 50 | 150 |
| | T₀R₂ (tank no.11) | 50 | |
| | T₀R₃(tank no.12) | 50 | |
| Grand Total | | | 600 |

### Feed formulation

The animal feed was prepared in a feed mill following standard feed formulation practice for fish in Bangladesh. The feed formulation, including ingredients which are used for preparing feed and their inclusion level, that was used in the studies is shown below in Table 23. The feed was free of hormones and antibiotics.

**Table 23. Animal feed formulation for pangus and tilapia finfish and prawn**

| **Ingredient** | **Inclusion (%)** |
|---|---|
| Fish Meal 40% | 18.75 |
| Fish Meal 60% | 18.75 |
| Soya bean Meal | 10 |
| Meat & Bone Meal | 15.625 |
| Rice Bran | 10.625 |
| Wheat Bran | 11.25 |
| Mustard Oil Cake | 6.25 |
| Maize | 5 |
| Wheat Flour | 3.75 |
| **Total** | **100** |

| **Additives** | |
|---|---|
| Dicalcium phosphate (DCP) | 0.5 |
| Pellet binder | 0.5 |
| Soybean oil | 0.5 |

Proximate composition analysis demonstrated that the formulated feed contained: moisture (14.91 %); crude protein (35.41 %); crude lipid (8.82 %); and ash (22.4 %).

An extract derived from sugar cane of the present disclosure was then added to the animal feed formulation in an amount of 0.2 w/w %, 0.4 w/w %, 0.6 w/w % of feed. The resulting feed was then given to pangus and tilpia finfish and prawn. The extract supplied in the trial had the following composition and properties as displayed in Table 24.

**Table 24. Composition and properties of sugar cane extract used in fish studies**

| **Physical and bioactive properties** | **Observation/Measurement** |
|---|---|
| Appearance | Dark brownish liquid |
| Aroma | Fermented salty taste |
| Density | 1.28 g/mL (± 0.05) @ 20°C |
| Brix | 55° (± 2) @ 20°C |
| pH | 4.6 (± 0.2) @ 20°C |
| Conductivity | 250,000 uS/cm |
| Total polyphenols | 30,400 mg/kg (as gallic acid equivalents) |
| Colour absorbance readings | |
| @270 nm | 1900 - 2300 |
| @420 nm | 90 - 120 |
| A270/A420 | 15 - 30 |
| Total Flavonoids | 7,800 mg / kg |
| Anti-oxidants (ABTS) | 9,600 mg / kg |

| **Mineral Content (per 100 g)** | |
|---|---|
| Sodium | 60 - 180 mg |
| Potassium | 2000 - 4000 mg |
| Calcium | 80 - 160 mg |
| Iron | 2 - 8 mg |
| Magnesium | 300 - 600 mg |
| Zinc | 1.5 - 3.0 mg |

| **Essential trace elements** | |
|---|---|
| Selenium | 0.04 - 0.09 mg |
| Chromium | 0.015 - 0.5 mg |

The extract also contained the following amino acids: aspartic acid, glutamic acid, asparagine, alanine, serine, valine and leucine.

Sampling of the experimental fish was undertaken in regular interval of one week by using scoop net in order to check the growth performance of fish and also adjust the feeding rate. Growth performance of fish in each sampling was measured by weight (g) and by length (cm).

### Results

### Results for pangus

Sampling over 16 weeks was completed and the results are listed below in Tables 25 to 32. Abbreviations used in pangus (and tilapia) sampling are: L* - length (cm); W* = weight (kg); LG* = length gain (cm); WG* = weight gain (kg); T* = treatment group; R* = replicate).

**Table 25. Pangus sampling results from Week 0 to Week 2**

| | | **Week 0** | | **Week 1** | | | | **Week 2** | | | |
|---|---|---|---|---|---|---|---|---|---|---|---|
| T* | R* | W* | L* | W* | L* | WG* | LG* | W* | L* | WG* | LG* |
| T0 | R1 | 4.74 | 5 | 8.33 | 8.5 | 3.59 | 3.5 | 10.76 | 11 | 2.43 | 2.5 |
| T0 | R2 | 4.74 | 5 | 8.33 | 8 | 3.59 | 3 | 11.83 | 10.75 | 3.5 | 2.75 |
| T0 | R3 | 4.74 | 5 | 7.56 | 8 | 2.82 | 3 | 10.6 | 10.75 | 3.04 | 2.75 |
| T0 | R4 | 4.74 | 5 | 5.67 | 7.5 | 0.93 | 2.5 | 11.84 | 10.75 | 6.17 | 3.25 |
| T1 | R1 | 4.74 | 5 | 9.09 | 8.8 | 4.35 | 3.8 | 12.06 | 11.5 | 2.97 | 2.7 |
| T1 | R2 | 4.74 | 5 | 6.67 | 7 | 1.93 | 2 | 10.54 | 10.75 | 3.87 | 3.75 |
| T1 | R3 | 4.74 | 5 | 8.33 | 8 | 3.59 | 3 | 12.29 | 10.75 | 3.96 | 2.75 |
| T1 | R4 | 4.74 | 5 | 7.27 | 7.5 | 2.53 | 2.5 | 11.91 | 11 | 4.64 | 3.5 |
| T2 | R1 | 4.74 | 5 | 6.22 | 7 | 1.48 | 2 | 9.27 | 11 | 3.05 | 4 |
| T2 | R2 | 4.74 | 5 | 8 | 7.9 | 3.26 | 2.9 | 10.9 | 11.5 | 2.9 | 3.6 |
| T2 | R3 | 4.74 | 5 | 6.92 | 7.2 | 2.18 | 2.2 | 11.6 | 10.75 | 4.68 | 3.55 |
| T2 | R4 | 4.74 | 5 | 7.25 | 7 | 2.51 | 2 | 10.83 | 10.75 | 3.58 | 3.75 |
| T3 | R1 | 4.74 | 5 | 7 | 7.2 | 2.26 | 2.2 | 11.66 | 11 | 4.66 | 3.8 |
| T3 | R2 | 4.74 | 5 | 8.33 | 8 | 3.59 | 3 | 11.91 | 11 | 3.58 | 3 |
| T3 | R3 | 4.74 | 5 | 8 | 8.1 | 3.26 | 3.1 | 11.83 | 10.75 | 3.83 | 2.65 |
| T3 | R4 | 4.74 | 5 | 8.18 | 8.5 | 3.44 | 3.5 | 11.64 | 11 | 3.46 | 2.5 |

**Table 26. Pangus sampling results Week 3 and Week 4**

| | | **Week 3** | | | | **Week 4** | | | |
|---|---|---|---|---|---|---|---|---|---|
| T* | R* | W* | L* | WG* | LG* | W* | L* | WG* | LG* |
| T0 | R1 | 15 | 12 | 4.24 | 1 | 15.33 | 13 | 0.33 | 1 |
| T0 | R2 | 13.75 | 11.5 | 1.92 | 0.75 | 15.95 | 12.3 | 2.2 | 0.8 |
| T0 | R3 | 11.66 | 11.5 | 1.06 | 0.75 | 15.29 | 13 | 3.63 | 1.5 |
| T0 | R4 | 12.4 | 12.5 | 0.56 | 1.75 | 15.88 | 13 | 3.48 | 0.5 |
| T1 | R1 | 13 | 12.5 | 0.94 | 1 | 15.4 | 13.5 | 2.4 | 1 |
| T1 | R2 | 14 | 12 | 3.46 | 1.25 | 18.18 | 14 | 4.18 | 2 |
| T1 | R3 | 14.72 | 12 | 2.43 | 1.25 | 17.6 | 14 | 2.88 | 2 |
| T1 | R4 | 15.71 | 12 | 3.8 | 1 | 15.95 | 13.5 | 0.24 | 1.5 |
| T2 | R1 | 11.76 | 11.5 | 2.49 | 0.5 | 13.83 | 13.5 | 2.07 | 2 |
| T2 | R2 | 15 | 12 | 4.1 | 0.5 | 18.26 | 12.5 | 3.26 | 0.5 |
| T2 | R3 | 15.77 | 12 | 4.17 | 1.25 | 16 | 13 | 0.23 | 1 |
| T2 | R4 | 16.5 | 12 | 5.67 | 1.25 | 17.25 | 13.5 | 0.75 | 1.5 |
| T3 | R1 | 12 | 11.5 | 0.34 | 0.5 | 17.03 | 13.5 | 5.03 | 2 |
| T3 | R2 | 13.67 | 13 | 1.76 | 2 | 17.18 | 14 | 3.51 | 1 |
| T3 | R3 | 12 | 11.5 | 0.17 | 0.75 | 17.25 | 13 | 5.25 | 1.5 |
| T3 | R4 | 15.83 | 12.5 | 4.19 | 1.5 | 16.19 | 13.5 | 0.36 | 1 |

**Table 27. Pangus sampling results Week 5 and Week 6**

| | | **Week 5** | | | | **Week 6** | | | |
|---|---|---|---|---|---|---|---|---|---|
| T* | R* | W* | L* | WG* | LG* | W* | L* | WG* | LG* |
| T0 | R1 | 18 | 13.5 | 2.67 | 0.5 | 22 | 14 | 4 | 0.5 |
| T0 | R2 | 22 | 13.5 | 6.05 | 1.2 | 22.87 | 14 | 0.87 | 0.5 |
| T0 | R3 | 21.33 | 13.2 | 6.04 | 0.2 | 22.62 | 13.5 | 1.29 | 0.3 |
| T0 | R4 | 20.72 | 13 | 4.84 | 0 | 23 | 13.5 | 2.28 | 0.5 |
| T1 | R1 | 20.6 | 13.5 | 5.2 | 0 | 25.5 | 14 | 4.9 | 0.5 |
| T1 | R2 | 19 | 14 | 0.82 | 0 | 25 | 14.7 | 6 | 0.7 |
| T1 | R3 | 23.5 | 14 | 5.9 | 0 | 29.48 | 14.5 | 5.98 | 0.5 |
| T1 | R4 | 22.4 | 13.5 | 6.45 | 0 | 28.67 | 14 | 6.27 | 0.5 |
| T2 | R1 | 16.5 | 13.5 | 2.67 | 0 | 19.93 | 13.8 | 3.43 | 0.3 |
| T2 | R2 | 19.25 | 13.5 | 0.99 | 1 | 23.24 | 14 | 3.99 | 0.5 |
| T2 | R3 | 21 | 13 | 5 | 0 | 23.8 | 13.5 | 2.8 | 0.5 |
| T2 | R4 | 17.5 | 13.5 | 0.25 | 0 | 21.63 | 13.9 | 4.13 | 0.4 |
| T3 | R1 | 21.2 | 13.5 | 4.17 | 0 | 24.08 | 13.7 | 2.88 | 0.2 |
| T3 | R2 | 21 | 14.2 | 3.82 | 0.2 | 23.85 | 14.5 | 2.85 | 0.3 |
| T3 | R3 | 19.5 | 13.5 | 2.25 | 0.5 | 22.46 | 13.7 | 2.96 | 0.2 |
| T3 | R4 | 21 | 13.5 | 4.81 | 0 | 23.15 | 13.5 | 2.15 | 0 |

**Table 28. Pangus sampling results Week 7 and Week 8**

| | | **Week 7** | | | | **Week 8** | | | |
|---|---|---|---|---|---|---|---|---|---|
| T* | R* | W* | L* | WG* | LG* | W* | L* | WG* | LG* |
| T0 | R1 | 25.9 | 14.4 | 3.9 | 0.4 | 28 | 14.9 | 2.1 | 0.5 |
| T0 | R2 | 25.75 | 14.3 | 2.88 | 0.3 | 26.58 | 14.5 | 0.83 | 0.2 |
| T0 | R3 | 24 | 13.9 | 1.38 | 0.4 | 26 | 14.4 | 2 | 0.5 |
| T0 | R4 | 25.33 | 14 | 2.33 | 0.5 | 26.67 | 14.5 | 1.34 | 0.5 |
| T1 | R1 | 29.25 | 14.5 | 3.75 | 0.5 | 38.7 | 15 | 9.45 | 0.5 |
| T1 | R2 | 30.75 | 15 | 5.75 | 0.3 | 36.82 | 15.5 | 6.07 | 0.5 |
| T1 | R3 | 32.66 | 15 | 3.18 | 0.5 | 34.67 | 15.5 | 2.01 | 0.5 |
| T1 | R4 | 31.88 | 14.4 | 3.21 | 0.4 | 34.73 | 14.7 | 2.85 | 0.3 |
| T2 | R1 | 22.71 | 14 | 2.78 | 0.2 | 26.76 | 14.5 | 4.05 | 0.5 |
| T2 | R2 | 26.5 | 14.4 | 3.26 | 0.4 | 28 | 14.8 | 1.5 | 0.4 |
| T2 | R3 | 25.5 | 14 | 1.7 | 0.5 | 26.47 | 14.6 | 0.97 | 0.6 |
| T2 | R4 | 24.42 | 14 | 2.79 | 0.1 | 26.65 | 14.3 | 2.23 | 0.3 |
| T3 | R1 | 26 | 14.2 | 1.92 | 0.5 | 28.57 | 14.5 | 2.57 | 0.3 |
| T3 | R2 | 25.33 | 14.5 | 1.48 | 0 | 28.94 | 15 | 3.61 | 0.5 |
| T3 | R3 | 27.36 | 14.1 | 4.9 | 0.4 | 28.66 | 14.5 | 1.3 | 0.4 |
| T3 | R4 | 24.75 | 14 | 1.6 | 0.5 | 25.48 | 14.2 | 0.73 | 0.2 |

**Table 29. Pangus sampling results Week 9 and Week 10**

| | | **Week 9** | | | | **Week 10** | | | |
|---|---|---|---|---|---|---|---|---|---|
| T* | R* | W* | L* | WG* | LG* | W* | L* | WG* | LG* |
| T0 | R1 | 30 | 15 | 2 | 0.1 | 31.24 | 15.5 | 1.24 | 0.5 |
| T0 | R2 | 28.8 | 15 | 2.22 | 0.5 | 30.59 | 15.5 | 1.79 | 0.5 |
| T0 | R3 | 27.9 | 14.5 | 1.9 | 0.1 | 29.5 | 15 | 1.6 | 0.5 |
| T0 | R4 | 29.14 | 15 | 2.47 | 0.5 | 30.03 | 15.5 | 0.89 | 0.5 |
| T1 | R1 | 42.67 | 16 | 3.97 | 1 | 44.19 | 16.5 | 1.52 | 0.5 |
| T1 | R2 | 40 | 16 | 3.18 | 0.5 | 43.75 | 16.5 | 3.75 | 0.5 |
| T1 | R3 | 36.57 | 15.9 | 1.9 | 0.4 | 39.49 | 16.5 | 2.92 | 0.6 |
| T1 | R4 | 36.33 | 15 | 1.6 | 0.3 | 38.78 | 16 | 2.45 | 1 |
| T2 | R1 | 30 | 15 | 3.24 | 0.5 | 32.49 | 15.5 | 2.49 | 0.5 |
| T2 | R2 | 32.6 | 15 | 4.6 | 0.2 | 34.38 | 15.4 | 1.78 | 0.4 |
| T2 | R3 | 28.75 | 15 | 2.28 | 0.4 | 30.48 | 15.5 | 1.73 | 0.5 |
| T2 | R4 | 28 | 14.5 | 1.35 | 0.2 | 30.17 | 15 | 2.17 | 0.5 |
| T3 | R1 | 31.67 | 15 | 3.1 | 0.5 | 32.73 | 15.5 | 1.06 | 0.5 |
| T3 | R2 | 30 | 15 | 1.06 | 0 | 31.08 | 15.4 | 1.08 | 0.4 |
| T3 | R3 | 29.5 | 15 | 0.84 | 0.5 | 30.67 | 15.5 | 1.17 | 0.5 |
| T3 | R4 | 27.78 | 15 | 2.3 | 0.8 | 29.55 | 15.5 | 1.77 | 0.5 |

**Table 30. Pangus sampling results Week 11 and Week 12**

| | | **Week 11** | | | | **Week 12** | | | |
|---|---|---|---|---|---|---|---|---|---|
| T* | R* | W* | L* | WG* | LG* | W* | L* | WG* | LG* |
| T0 | R1 | 33.14 | 16 | 1.9 | 0.5 | 35.06 | 16.5 | 1.92 | 0.5 |
| T0 | R2 | 32.75 | 16 | 2.16 | 0.5 | 33.94 | 16.3 | 1.19 | 0.3 |
| T0 | R3 | 32.29 | 16 | 2.79 | 1 | 33.72 | 16.2 | 1.43 | 0.2 |
| T0 | R4 | 32 | 15.5 | 1.97 | 0 | 33.33 | 16 | 1.33 | 0.5 |
| T1 | R1 | 46.33 | 17 | 2.14 | 0.5 | 47.44 | 17.3 | 1.11 | 0.3 |
| T1 | R2 | 44.5 | 16.8 | 0.75 | 0.3 | 45.42 | 17 | 0.92 | 0.2 |
| T1 | R3 | 41 | 16.6 | 1.51 | 0.1 | 42.78 | 16.9 | 1.78 | 0.3 |
| T1 | R4 | 40 | 16.5 | 1.22 | 0.5 | 41.33 | 16.7 | 1.33 | 0.2 |
| T2 | R1 | 35.6 | 16 | 3.11 | 0.5 | 37.04 | 16.5 | 1.44 | 0.5 |
| T2 | R2 | 35.33 | 16 | 0.95 | 0.6 | 35.47 | 16.4 | 0.14 | 0.4 |
| T2 | R3 | 32 | 15.8 | 1.52 | 0.3 | 33.33 | 16 | 1.33 | 0.2 |
| T2 | R4 | 33 | 16 | 2.83 | 1 | 38.71 | 16.5 | 5.71 | 0.5 |
| T3 | R1 | 33.8 | 16 | 1.07 | 0.5 | 36 | 16.5 | 2.2 | 0.5 |
| T3 | R2 | 31.6 | 16 | 0.52 | 0.6 | 33.33 | 16.5 | 1.73 | 0.5 |
| T3 | R3 | 34 | 16 | 3.33 | 0.5 | 35.96 | 16.3 | 1.96 | 0.3 |
| T3 | R4 | 30.4 | 15.8 | 0.85 | 0.3 | 33.04 | 16 | 2.64 | |

**Table 31. Pangus sampling results Week 13 and Week 14**

| | | **Week 13** | | | | **Week 14** | | | |
|---|---|---|---|---|---|---|---|---|---|
| T* | R* | W* | L* | WG* | LG* | W* | L* | WG* | LG* |
| T0 | R1 | 36 | 16.9 | 0.94 | 0.4 | 36.85 | 17 | 0.85 | 0.1 |
| T0 | R2 | 35 | 16.5 | 1.06 | 0.2 | 36.18 | 16.8 | 1.18 | 0.3 |
| T0 | R3 | 34.5 | 16.5 | 0.78 | 0.3 | 36.04 | 16.7 | 1.54 | 0.2 |
| T0 | R4 | 34.75 | 16.5 | 1.42 | 0.5 | 36 | 16.5 | 1.25 | 0 |
| T1 | R1 | 49 | 17.5 | 1.56 | 0.2 | 52.78 | 18 | 3.78 | 0.5 |
| T1 | R2 | 46 | 17 | 0.58 | 0 | 46.5 | 17.2 | 0.5 | 0.2 |
| T1 | R3 | 43.5 | 17 | 0.72 | 0.1 | 45.78 | 17 | 2.28 | 0 |
| T1 | R4 | 42.25 | 17 | 0.92 | 0.3 | 43 | 17 | 0.75 | 0 |
| T2 | R1 | 40 | 17 | 2.96 | 0.5 | 43.83 | 17.5 | 3.83 | 0.5 |
| T2 | R2 | 36 | 16.5 | 0.53 | 0.1 | 36.21 | 16.6 | 0.21 | 0.1 |
| T2 | R3 | 36.67 | 16.5 | 3.34 | 0.5 | 38.38 | 17 | 1.71 | 0.5 |
| T2 | R4 | 40 | 16.9 | 1.29 | 0.4 | 43.67 | 17.2 | 3.67 | 0.3 |
| T3 | R1 | 40 | 17 | 4 | 0.5 | 43.75 | 17.5 | 3.75 | 0.5 |
| T3 | R2 | 35.4 | 16.7 | 2.07 | 0.2 | 40 | 17 | 4.6 | 0.3 |
| T3 | R3 | 37 | 16.5 | 1.04 | 0.2 | 38.26 | 17 | 1.26 | 0.5 |
| T3 | R4 | 36 | 16.5 | 2.96 | 0.5 | 38.09 | 17 | 2.09 | 0.5 |

**Table 32. Pangus sampling results Week 15 and Week 16**

| | | **Week 15** | | | | **Week 16** | | | |
|---|---|---|---|---|---|---|---|---|---|
| T* | R* | W* | L* | WG* | LG* | W* | L* | WG* | LG* |
| T0 | R1 | 38.51 | 17 | 1.66 | 0 | 40.96 | 17.2 | 2.45 | 0.2 |
| T0 | R2 | 38.25 | 17 | 2.07 | 0.2 | 39.78 | 17.1 | 1.53 | 0.1 |
| T0 | R3 | 37.33 | 17 | 1.29 | 0.3 | 39.82 | 17.1 | 2.49 | 0.1 |
| T0 | R4 | 37.09 | 17 | 1.09 | 0.5 | 39.17 | 17 | 2.08 | 0 |
| T1 | R1 | 58 | 19 | 5.22 | 1 | 64.32 | 20 | 6.32 | 1 |
| T1 | R2 | 49.9 | 18 | 3.4 | 0.8 | 54.29 | 18.5 | 4.39 | 0.5 |
| T1 | R3 | 47.37 | 17.5 | 1.59 | 0.5 | 49.63 | 18 | 2.26 | 0.5 |
| T1 | R4 | 44.12 | 17.5 | 1.12 | 0.5 | 46.18 | 18 | 2.06 | 0.5 |
| T2 | R1 | 44.95 | 17.8 | 1.12 | 0.3 | 46.38 | 18.2 | 1.43 | 0.4 |
| T2 | R2 | 38.58 | 17 | 2.37 | 0.4 | 40.89 | 17.5 | 2.31 | 0.5 |
| T2 | R3 | 40 | 17.2 | 1.62 | 0.2 | 42.14 | 17.5 | 2.14 | 0.3 |
| T2 | R4 | 44.32 | 17.5 | 0.65 | 0.3 | 45.65 | 18 | 1.33 | 0.5 |
| T3 | R1 | 44.45 | 17.9 | 0.7 | 0.4 | 46.43 | 18.5 | 1.98 | 0.6 |
| T3 | R2 | 43.89 | 17.5 | 3.89 | 0.5 | 48.78 | 18 | 4.89 | 0.5 |
| T3 | R3 | 40.67 | 17.3 | 2.41 | 0.3 | 43.17 | 17.8 | 2.5 | 0.5 |
| T3 | R4 | 40 | 17.3 | 1.91 | 0.3 | 42.16 | 17.6 | 2.16 | 0.3 |

Initial average weight of the pangus was 4.74g and the initial average length was 5 cm. From the last sampling at week 16, the average weight was found to be 39.93 g in T₀, 53.61 g in T₁, 43.77 g in T₂ and 45.14 g in T₃. The average length was found as 17.1 cm in T₀, 18.63 cm in T₁, 17.8 g in T₂ and 17.98 cm in T₃. In summary, the T₁ treatment showed higher and even growth (by weight and length) in comparison with the other treatment groups. The growth performance of pangus in terms of length and weight; average weight gain and average length gain chart are shown in Figures 14 to 17. Figures 18 and 19 exhibit photographs of pangus showing the size comparison across the treatment groups.

### Results for tilapia

Sampling over sixteen weeks has been conducted and the results are listed below in Tables 33 to 40.

**Table 33. Tilapia sampling results from Week 0 to Week 2**

| | | **Week 0** | | **Week 1** | | | | **Week 2** | | | |
|---|---|---|---|---|---|---|---|---|---|---|---|
| T* | R* | W* | L* | W* | L* | WG* | LG* | W* | L* | WG* | LG* |
| T0 | R1 | 2.24 | 3.21 | 5.11 | 5.5 | 5.11 | 2.29 | 8.83 | 8 | 3.72 | 2.5 |
| T0 | R2 | 2.24 | 3.21 | 4.76 | 5.5 | 2.52 | 2.29 | 9.91 | 9 | 5.15 | 3.5 |
| T0 | R3 | 2.24 | 3.21 | 5 | 5 | 2.76 | 1.79 | 8.33 | 8 | 3.33 | 3 |
| T0 | R4 | 2.24 | 3.21 | 4.76 | 5 | 2.52 | 1.79 | 8.5 | 7.5 | 3.74 | 2.5 |
| T1 | R1 | 2.24 | 3.21 | 4.54 | 6.5 | 2.3 | 3.29 | 9 | 8.5 | 4.46 | 2 |
| T1 | R2 | 2.24 | 3.21 | 5 | 7 | 2.76 | 3.79 | 10.12 | 7 | 5.12 | 0 |
| T1 | R3 | 2.24 | 3.21 | 5 | *5* | 2.76 | 1.79 | 8 | 7.5 | 3 | 2.5 |
| T1 | R4 | 2.24 | 3.21 | 4.33 | 5 | 2.09 | 1.79 | 9.26 | 8 | 4.93 | 3 |
| T2 | R1 | 2.24 | 3.21 | 5.92 | 6.5 | 3.68 | 3.29 | 10.89 | 7.75 | 4.97 | 1.25 |
| T2 | R2 | 2.24 | 3.21 | 4.76 | 6 | 2.52 | 2.79 | 8.31 | 7.5 | 3.55 | 1.5 |
| T2 | R3 | 2.24 | 3.21 | 5.26 | 4.5 | 3.02 | 1.29 | 8.41 | 8 | 3.15 | 3.5 |
| T2 | R4 | 2.24 | 3.21 | 5.56 | 6 | 3.32 | 2.79 | 8.18 | 7.5 | 2.62 | 1.5 |
| T3 | R1 | 2.24 | 3.21 | 4.76 | 6 | 2.52 | 2.79 | 8.06 | 8.5 | 3.3 | 2.5 |
| T3 | R2 | 2.24 | 3.21 | 3.33 | 4.5 | 1.09 | 1.29 | 6.44 | 6.75 | 3.11 | 2.25 |
| T3 | R3 | 2.24 | 3.21 | 5.56 | 6 | 3.32 | 2.79 | 8.75 | 9 | 3.19 | 3 |
| T3 | R4 | 2.24 | 3.21 | 5 | 6 | 2.76 | 2.79 | 8.06 | 8.5 | 3.06 | 2.5 |

**Table 34. Tilapia sampling results Week 3 and Week 4**

| | | **Week 3** | | | | **Week 4** | | | |
|---|---|---|---|---|---|---|---|---|---|
| T* | R* | W* | L* | WG* | LG* | W* | L* | WG* | LG* |
| T0 | R1 | 12.5 | 10.5 | 3.67 | 2.5 | 15.24 | 11 | 2.74 | 0.5 |
| T0 | R2 | 14 | 9.5 | 4.09 | 0.5 | 19.25 | 11.17 | 5.25 | 1.67 |
| T0 | R3 | 12.14 | 9.5 | 3.81 | 1.5 | 14.67 | 10 | 2.53 | 0.5 |
| T0 | R4 | 12.5 | 10.5 | 4 | 3 | 15.44 | 10.83 | 2.94 | 0.33 |
| T1 | R1 | 10.95 | 10 | 1.95 | 1.5 | 16.52 | 11 | 5.57 | 1 |
| T1 | R2 | 16 | 10.5 | 5.88 | 3.5 | 17.5 | 11 | 1.5 | 0.5 |
| T1 | R3 | 12.94 | 9.5 | 4.94 | 2 | 14.89 | 11.3 | 1.95 | 1.8 |
| T1 | R4 | 12.5 | 9 | 3.24 | 1 | 16.66 | 10.5 | 4.16 | 1.5 |
| T2 | R1 | 15 | 9.5 | 4.11 | 1.75 | 18.83 | 11 | 3.83 | 1.5 |
| T2 | R2 | 12.5 | 11 | 4.19 | 3.5 | 15.95 | 12 | 3.45 | 1 |
| T2 | R3 | 14.35 | 10 | 5.94 | 2 | 15 | 11 | 0.65 | 1 |
| T2 | R4 | 10.91 | 10 | 2.73 | 2.5 | 16.2 | 11.33 | 5.29 | 1.33 |
| T3 | R1 | 12 | 11 | 3.94 | 2.5 | 16.25 | 11.5 | 4.25 | 0.5 |
| T3 | R2 | 14 | 11.5 | 7.56 | 4.75 | 16.43 | 11.5 | 2.43 | 0 |
| T3 | R3 | 12 | 10 | 3.25 | 1 | 17.45 | 11.67 | 5.45 | 1.67 |
| T3 | R4 | 11.95 | 10 | 3.89 | 1.5 | 17.43 | 10.5 | 5.48 | 0.5 |

**Table 35. Tilapia sampling results Week 5 and Week 6**

| | | **Week 5** | | | | **Week 6** | | | |
|---|---|---|---|---|---|---|---|---|---|
| T* | R* | W* | L* | WG* | LG* | W* | L* | WG* | LG* |
| T0 | R1 | 26.67 | 12 | 11.43 | 1 | 30.69 | 12.2 | 4.02 | 0.2 |
| T0 | R2 | 23.29 | 12.2 | 4.04 | 1.03 | 36.82 | 12.4 | 13.53 | 0.2 |
| T0 | R3 | 21.62 | 11.5 | 6.95 | 1.5 | 23.53 | 11.5 | 1.91 | 0 |
| T0 | R4 | 22.31 | 12.5 | 6.87 | 1.67 | 28.89 | 13 | 6.58 | 0.5 |
| T1 | R1 | 25 | 12.5 | 8.48 | 1.5 | 32.33 | 12.6 | 7.33 | 0.1 |
| T1 | R2 | 27.33 | 13 | 9.83 | 2 | 38.79 | 13.6 | 11.46 | 0.6 |
| T1 | R3 | 24.54 | 12 | 9.65 | 0.7 | 30.4 | 13 | 5.86 | 1 |
| T1 | R4 | 23 | 11 | 6.34 | 0.5 | 29.07 | 11.5 | 6.07 | 0.5 |
| T2 | R1 | 34 | 13 | 15.17 | 2 | 35.5 | 13.9 | 1.5 | 0.9 |
| T2 | R2 | 25.64 | 12 | 9.69 | 0 | 29.12 | 12.3 | 3.48 | 0.3 |
| T2 | R3 | 22.29 | 11.5 | 7.29 | 0.5 | 30.54 | 13 | 8.25 | 1.5 |
| T2 | R4 | 21.43 | 12 | 5.23 | 0.67 | 33.25 | 13.2 | 11.82 | 1.2 |
| T3 | R1 | 29 | 13.5 | 12.75 | 2 | 29.84 | 13.7 | 0.84 | 0.2 |
| T3 | R2 | 29 | 13 | 12.57 | 1.5 | 33.08 | 14 | 4.08 | 1 |
| T3 | R3 | 27 | 12.5 | 9.55 | 0.83 | 31.78 | 13.5 | 4.78 | 1 |
| T3 | R4 | 18 | 11.5 | 0.57 | 1 | 29.14 | 12.3 | 11.14 | 0.8 |

**Table 36. Tilapia sampling results Week 7 and Week 8**

| | | **Week 7** | | | | **Week 8** | | | |
|---|---|---|---|---|---|---|---|---|---|
| T* | R* | W* | L* | WG* | LG* | W* | L* | WG* | LG* |
| T0 | R1 | 40.84 | 13.5 | 10.15 | 1.3 | 43.38 | 14.5 | 2.54 | 1 |
| T0 | R2 | 50 | 13.5 | 13.18 | 1.1 | 50.31 | 14.5 | 0.31 | 1 |
| T0 | R3 | 31.94 | 13.5 | 8.41 | 2 | 38.05 | 14 | 6.11 | 0.5 |
| T0 | R4 | 33.17 | 13.5 | 4.28 | 0.5 | 41.58 | 14 | 8.41 | 0.5 |
| T1 | R1 | 40 | 14.5 | 7.67 | 1.9 | 49.44 | 15 | 9.44 | 0.5 |
| T1 | R2 | 50.75 | 14 | 11.96 | 0.4 | 52.54 | 14.5 | 1.79 | 0.5 |
| T1 | R3 | 48.75 | 14 | 18.35 | 1 | 48.94 | 14.5 | 0.19 | 0.5 |
| T1 | R4 | 37.69 | 13.5 | 8.62 | 2 | 46.89 | 15 | 9.2 | 1.5 |
| T2 | R1 | 47 | 14.5 | 11.5 | 0.6 | 47.62 | 15 | 0.62 | 0.5 |
| T2 | R2 | 37.36 | 13.5 | 8.24 | 1.2 | 40.96 | 13.5 | 3.6 | 0 |
| T2 | R3 | 45.58 | 14 | 15.04 | 1 | 48.15 | 15 | 2.57 | 1 |
| T2 | R4 | 37.69 | 14 | 4.44 | 0.8 | 49.06 | 14 | 11.37 | 0 |
| T3 | R1 | 46.25 | 14 | 16.41 | 0.3 | 49.52 | 15 | 3.27 | 1 |
| T3 | R2 | 43.4 | 14 | 10.32 | 0 | 48.51 | 15 | 5.11 | 1 |
| T3 | R3 | 40 | 14 | 8.22 | 0.5 | 46.15 | 15 | 6.15 | 1 |
| T3 | R4 | 41.53 | 14 | 12.39 | 1.7 | 42.43 | 15 | 0.9 | 1 |

**Table 37. Tilapia sampling results Week 9 and Week 10**

| | | **Week 9** | | | | **Week 10** | | | |
|---|---|---|---|---|---|---|---|---|---|
| T* | R* | W* | L* | WG* | LG* | W* | L* | WG* | LG* |
| T0 | R1 | 45.71 | 15 | 2.33 | 0.5 | 56.34 | 16.5 | 10.63 | 1.5 |
| T0 | R2 | 60 | 14.5 | 9.69 | 0 | 70.53 | 17 | 10.53 | 2.5 |
| T0 | R3 | 40 | 14.5 | 1.95 | 0.5 | 51.67 | 15.5 | 11.67 | 1 |
| T0 | R4 | 52.5 | 15 | 10.92 | 1 | 58.14 | 16.5 | 5.64 | 1.5 |
| T1 | R1 | 52.5 | 15 | 3.06 | 0 | 68.75 | 15.5 | 16.25 | 0.5 |
| T1 | R2 | 67.17 | 15 | 14.63 | 0.5 | 73.77 | 16 | 6.6 | 1 |
| T1 | R3 | 50 | 15 | 1.06 | 0.5 | 65.33 | 16 | 15.33 | 1 |
| T1 | R4 | 57 | 15 | 10.11 | 0 | 58.99 | 15 | 1.99 | 0 |
| T2 | R1 | 55 | 15.5 | 7.38 | 0.5 | 81.35 | 16 | 26.35 | 0.5 |
| T2 | R2 | 46 | 15 | 5.04 | 1.5 | 55.56 | 16 | 9.56 | 1 |
| T2 | R3 | 56 | 15 | 7.85 | 0 | 65 | 16 | 9 | 1 |
| T2 | R4 | 62.5 | 15 | 13.44 | 1 | 71.83 | 15.5 | 9.33 | 0.5 |
| T3 | R1 | 52.5 | 15 | 2.98 | 0 | 54.17 | 16 | 1.67 | 1 |
| T3 | R2 | 50.71 | 15 | 2.2 | 0 | 61.19 | 15.5 | 10.48 | 0.5 |
| T3 | R3 | 54 | 15 | 7.85 | 0 | 58.62 | 16 | 4.62 | 1 |
| T3 | R4 | 50 | 15.5 | 7.57 | 0.5 | 54.26 | 16 | 4.26 | 0.5 |

**Table 38. Tilapia sampling results Week 11 and Week 12**

| | | **Week 11** | | | | **Week 12** | | | |
|---|---|---|---|---|---|---|---|---|---|
| T* | R* | W* | L* | WG* | LG* | W* | L* | WG* | LG* |
| T0 | R1 | 64 | 16.5 | 7.66 | 0 | 68.85 | 16.5 | 4.85 | 0 |
| T0 | R2 | 70.87 | 17 | 0.34 | 0 | 72.08 | 17 | 1.21 | 0 |
| T0 | R3 | 58.75 | 15.5 | 7.08 | 0 | 60.44 | 16 | 1.69 | 0.5 |
| T0 | R4 | 64 | 16.5 | 5.86 | 0 | 72.79 | 16.5 | 8.79 | 0 |
| T1 | R1 | 70.56 | 16.5 | 1.81 | 1 | 72.85 | 17 | 2.29 | 0.5 |
| T1 | R2 | 82.22 | 17 | 8.45 | 1 | 88.24 | 17 | 6.02 | 0 |
| T1 | R3 | 68.75 | 16.5 | 3.42 | 0.5 | 70 | 16.5 | 1.25 | 0 |
| T1 | R4 | 65 | 15.5 | 6.01 | 0.5 | 66.13 | 16.5 | 1.13 | 1 |
| T2 | R1 | 85.5 | 17 | 4.15 | 1 | 91.67 | 17 | 6.17 | 0 |
| T2 | R2 | 70 | 16.4 | 14.44 | 0.4 | 70.89 | 17 | 0.89 | 0.6 |
| T2 | R3 | 70 | 16.5 | 5 | 0.5 | 73.17 | 17 | 3.17 | 0.5 |
| T2 | R4 | 80 | 16 | 8.17 | 0.5 | 86.03 | 17 | 6.03 | 1 |
| T3 | R1 | 65 | 16 | 10.83 | 0 | 70.41 | 17 | 5.41 | 1 |
| T3 | R2 | 68.8 | 16 | 7.61 | 0.5 | 70.46 | 17 | 1.66 | 1 |
| T3 | R3 | 69.67 | 16 | 11.05 | 0 | 75.95 | 16.5 | 6.28 | 0.5 |
| T3 | R4 | 68.17 | 16 | 13.91 | 0 | 70.89 | 16.5 | 2.72 | 0.5 |

**Table 39. Tilapia sampling results Week 13 and Week 14**

| | | **Week 13** | | | | **Week 14** | | | |
|---|---|---|---|---|---|---|---|---|---|
| T* | R* | W* | L* | WG* | LG* | W* | L* | WG* | LG* |
| T0 | R1 | 70 | 16.5 | 1.15 | 0 | 74.39 | 17.5 | 4.39 | 1 |
| T0 | R2 | 75 | 17 | 2.92 | 0 | 80 | 17.5 | *5* | 0.5 |
| T0 | R3 | 62.5 | 16.5 | 2.06 | 0.5 | 65.15 | 17 | 2.65 | 0.5 |
| T0 | R4 | 75.25 | 16.5 | 2.46 | 0 | 77.78 | 17.5 | 2.53 | 1 |
| T1 | R1 | 74.5 | 17 | 1.65 | 0 | 84.29 | 17.5 | 9.79 | 0.5 |
| T1 | R2 | 90 | 17 | 1.76 | 0 | 98 | 17 | 8 | 0 |
| T1 | R3 | 82 | 17 | 12 | 0.5 | 92.31 | 18 | 10.31 | 1 |
| T1 | R4 | 72.5 | 17 | 6.37 | 0.5 | 79.17 | 17 | 6.67 | 0 |
| T2 | R1 | 96 | 17.5 | 4.33 | 0.5 | 105 | 18 | 9 | 0.5 |
| T2 | R2 | 72 | 17 | 1.11 | 0 | 79.49 | 18 | 7.49 | 1 |
| T2 | R3 | 75 | 17 | 1.83 | 0 | 90.57 | 17.5 | 15.57 | 0.5 |
| T2 | R4 | 92 | 17.5 | 5.97 | 0.5 | 111.54 | 18 | 19.54 | 0.5 |
| T3 | R1 | 73 | 17 | 2.59 | 0 | 78 | 17 | 5 | 0 |
| T3 | R2 | 74 | 17 | 3.54 | 0 | 77.78 | 17.5 | 3.78 | 0.5 |
| T3 | R3 | 77 | 16.5 | 1.05 | 0 | 81.95 | 17.5 | 4.95 | 1 |
| T3 | R4 | 80 | 17 | 9.11 | 0.5 | 80 | 17 | 0 | 0 |

**Table 40. Tilapia sampling results Week 15 and Week 16**

| | | **Week 15** | | | | **Week 16** | | | |
|---|---|---|---|---|---|---|---|---|---|
| T* | R* | W* | L* | WG* | LG* | W* | L* | WG* | LG* |
| T0 | R1 | 78 | 17.5 | 3.61 | 0 | 88.11 | 17.5 | 10.11 | 0 |
| T0 | R2 | 80 | 17.5 | 0 | 0 | 92.28 | 17.5 | 12.28 | 0 |
| T0 | R3 | 76 | 17 | 10.85 | 0 | 76.46 | 17 | 0.46 | 0 |
| T0 | R4 | 82 | 17.5 | 4.22 | 0 | 89.7 | 17.5 | 7.7 | 0 |
| T1 | R1 | 86 | 17.5 | 1.71 | 0 | 87.16 | 17.5 | 1.16 | 0 |
| T1 | R2 | 99 | 18 | 1 | 1 | 103.05 | 18.5 | 4.05 | 0.5 |
| T1 | R3 | 92.67 | 18 | 0.36 | 0 | 95 | 18 | 2.33 | 0 |
| T1 | R4 | 82.5 | 17.5 | 3.33 | 0.5 | 88 | 18 | 5.5 | 0.5 |
| T2 | R1 | 120 | 18 | 15 | 0 | 123.27 | 18 | 3.27 | 0 |
| T2 | R2 | 80 | 18 | 0.51 | 0 | 82.68 | 18 | 2.68 | 0 |
| T2 | R3 | 100 | 17.5 | 9.43 | 0 | 103.02 | 18.5 | 3.02 | 1 |
| T2 | R4 | 114.5 | 18 | 2.96 | 0 | 116.68 | 18.5 | 2.18 | 0.5 |
| T3 | R1 | 79.5 | 17.5 | 1.5 | 0.5 | 80 | 18 | 0.5 | 0.5 |
| T3 | R2 | 84 | 18 | 6.22 | 0.5 | 102.08 | 18 | 18.08 | 0 |
| T3 | R3 | 90 | 18 | 8.05 | 0.5 | 95.58 | 18.5 | 5.58 | 0.5 |
| T3 | R4 | 80 | 17.5 | 0 | 0.5 | 81.46 | 18 | 1.46 | 0.5 |

At stocking of tilapia and the beginning of the study, the average weight of fish was 2.24 g. The first sampling showed that the average weight of each treatment group T₀, T₁, T₂ and T₃ was 4.9075g, 4.7175 g, 5.375g and 4.665g respectively. In week 16 at the final sampling, the average weight of each treatment group T₀, T₁, T₂ and T₃ was 86.6375 g, 93.3025 g, 106.4125 g and 89.78 g respectively. It indicates that, the average weight was increased at T₂ (0.4%) treated feed. During stocking of fish, the average length of fish was 3.21 cm. After 16 week interval, the average length of fish was increased which fed with T₂ (0.4%) treated feed. After 16 times sampling, it indicates that the average weight gain were increased which fed with T₂ (0.4%) and length gain were increased which fed with T₂ (0.4%) treated feed. The growth performance of tilapia in terms of length and weight; average weight gain and average length gain chart are shown in Figures 20 to 23. Figures 24 and 25 exhibit photographs of tilapia showing the size comparison across the treatment groups.

### Results for prawn

Sampling results for prawn are shown in Table 41A to Table 41D (L - length (cm); LG = length gain (cm); T* = treatment group; R* = replicate).

**Table 41A. Length gain for prawn - sampling 1 to 3**

| **T*** | **R*** | **Initial** | **Sampling 1** | | **Sampling 2** | | **Sampling 3** | |
|---|---|---|---|---|---|---|---|---|
| T | R | L | L | LG | L | LG | L | LG |
| T0 | R1 | 2 | 2.4 | 0.4 | 2.55 | 0.15 | 3.15 | 0.6 |
| T0 | R2 | 2 | 2.65 | 0.65 | 2.6 | -0.05 | 2.6 | 0 |
| T0 | R3 | 2 | 2.26 | 0.26 | 3.15 | 0.89 | 3.28 | 0.13 |
| T1 | R1 | 2 | 2.2 | 0.2 | 2.45 | 0.25 | 2.8 | 0.35 |
| T1 | R2 | 2 | 2.8 | 0.8 | 3 | 0.2 | 3.4 | 0.4 |
| T1 | R3 | 2 | 2.95 | 0.95 | 3.15 | 0.2 | 3.1 | -0.05 |
| T2 | R1 | 2 | 2.75 | 0.75 | 2.76 | 0.01 | 3.3 | 0.54 |
| T2 | R2 | 2 | 3.05 | 1.05 | 3.15 | 0.1 | 3.1 | -0.05 |
| T2 | R3 | 2 | 3 | 1 | 3.05 | 0.05 | 3.73 | 0.68 |
| T3 | R1 | 2 | 3.25 | 1.25 | 3.25 | 0 | 3.38 | 0.13 |
| T3 | R2 | 2 | 2.4 | 0.4 | 3.15 | 0.75 | 3.23 | 0.08 |
| T3 | R3 | 2 | 3.55 | 1.55 | 3.7 | 0.15 | 3.7 | 0 |

**Table 41B. Length gain for prawn - sampling 4 and 5**

| **T*** | **R*** | **Sampling 4** | | **Sampling 5** | |
|---|---|---|---|---|---|
| T | R | L | LG | L | LG |
| T0 | R1 | 3.73 | 0.58 | 3.99 | 0.26 |
| T0 | R2 | 3.43 | 0.83 | 3.75 | 0.32 |
| T0 | R3 | 3.43 | 0.15 | 4.4 | 0.97 |
| T1 | R1 | 3.33 | 0.53 | 4.2 | 0.87 |
| T1 | R2 | 3.77 | 0.37 | 3.9 | 0.13 |
| T1 | R3 | 4.17 | 1.07 | 4.8 | 0.63 |
| T2 | R1 | 3.6 | 0.3 | 4.15 | 0.55 |
| T2 | R2 | 3.93 | 0.83 | 4.1 | 0.17 |
| T2 | R3 | 4.37 | 0.64 | 4.8 | 0.43 |
| T3 | R1 | 3.83 | 0.45 | 4.7 | 0.87 |
| T3 | R2 | 4.25 | 1.02 | 4.75 | 0.5 |
| T3 | R3 | 4.47 | 0.77 | 4.57 | 0.1 |

**Table 41C. Length gain for prawn - sampling 6 to 8 weight gain for prawn - sampling 8**

| **T*** | **R*** | **Sampling 6** | | **Sampling 7** | | **Sampling 8** | | |
|---|---|---|---|---|---|---|---|---|
| T | R | L | LG | L | LG | L | LG | W |
| T0 | R1 | 4.23 | 0.24 | 4.33 | 0.1 | 4.43 | 0.1 | 0.5 |
| T0 | R2 | 3.96 | 0.21 | 4.5 | 0.54 | 4.7 | 0.2 | 0.76 |
| T0 | R3 | 4.8 | 0.4 | 4.85 | 0.05 | 4.89 | 0.04 | 0.44 |
| T1 | R1 | 4.25 | 0.05 | 4.45 | 0.2 | 4.5 | 0.05 | 0.5 |
| T1 | R2 | 4.16 | 0.26 | 4.43 | 0.27 | 4.85 | 0.42 | 0.77 |
| T1 | R3 | 4.9 | 0.1 | 5.03 | 0.13 | 5.05 | 0.02 | 0.81 |
| T2 | R1 | 4.35 | 0.2 | 4.38 | 0.03 | 4.43 | 0.05 | 0.53 |
| T2 | R2 | 4.6 | 0.5 | 4.85 | 0.25 | 5 | 0.15 | 0.66 |
| T2 | R3 | 5.15 | 0.35 | 5.17 | 0.02 | 5.2 | 0.03 | 1 |
| T3 | R1 | 4.8 | 0.1 | 4.9 | 0.1 | 5 | 0.1 | 0.66 |
| T3 | R2 | 5 | 0.25 | 5.11 | 0.11 | 5.19 | 0.08 | 0.59 |
| T3 | R3 | 5 | 0.43 | 5.53 | 0.53 | 5.63 | 0.1 | 0.7 |

**Table 41D. Length and weight gain for prawn - sampling 9 and 10**

| **T*** | **R*** | **Sampling 9** | | | | **Sampling 10** | | | |
|---|---|---|---|---|---|---|---|---|---|
| T | R | L | LG | W | WG | L | LG | W | WG |
| T0 | R1 | 4.5 | 0.07 | 1.1 | 0.6 | 4.7 | 0.2 | 1.15 | 0.05 |
| T0 | R2 | 4.83 | 0.13 | 1.05 | 0.29 | 4.9 | 0.07 | 1.18 | 0.13 |
| T0 | R3 | 4.95 | 0.06 | 0.92 | 0.48 | 4.95 | 0 | 1.4 | 0.48 |
| T1 | R1 | 4.6 | 0.1 | 0.65 | 0.15 | 4.7 | 0.1 | 0.82 | 0.17 |
| T1 | R2 | 5 | 0.15 | 0.98 | 0.21 | 5.06 | 0.06 | 1.17 | 0.19 |
| T1 | R3 | 5.15 | 0.1 | 1.09 | 0.28 | 5.2 | 0.05 | 1.21 | 0.12 |
| T2 | R1 | 4.6 | 0.17 | 0.63 | 0.1 | 4.7 | 0.1 | 0.84 | 0.21 |
| T2 | R2 | 5.5 | 0.5 | 1.79 | 1.13 | 5.65 | 0.15 | 1.89 | 0.1 |
| T2 | R3 | 5.3 | 0.1 | 1.21 | 0.21 | 5.3 | 0 | 1.26 | 0.05 |
| T3 | R1 | 5.2 | 0.2 | 1.4 | 0.74 | 6.86 | 1.66 | 2.29 | 0.89 |
| T3 | R2 | 5.25 | 0.06 | 1.59 | 1 | 6 | 0.75 | 2.05 | 0.46 |
| T3 | R3 | 5.7 | 0.07 | 1.36 | 0.66 | 5.95 | 0.25 | 2.13 | 0.77 |

The study indicated that the growth of prawn is highest in the T₃ treatment group. During stocking of prawn the average length was 2 cm. At the 10^{th} sampling, average length was 6.27 cm in the T₃ treatment group (0.6 % dose); 5.22 cm in the T₂ treatment group (0.4% dose); 4.99 cm in the T₂ treatment group (0.2% dose) and 4.85 cm in the T₀ control group. The average weight was 2.16 g at 0.6%; 1.33g at 0.4%, 1.07g at 0.2% and 1.24g in the control. The growth performance of prawn in terms of weight and length; charts for average weight gain and average length gain chart are shown in Figures 26 to 29. Figures 30 and 31 exhibit photographs of prawn showing the size comparison across the treatment groups.

### Summary

### Improved growth performance

In pangus, the best weight gain performance was found in the 0.2% extract treated fish - 48.87 g compared to the control fish (35.45 g). Not only the 0.2% treated fish, but all treated fish had higher growth performance than the control.

In tilapia, the best weight gain performance was found in 0.4% extract treated fish - 104.1725 g compared to the control fish (84.3975 g) where average weight gain of 0.4% treated fish was greater than control fish. Not only 0.4% treated fish, but all treated fish had higher growth performance than the control. A summary of the weight gain performance of pangus and tilapia is shown in Table 42.

**Table 42. Growth performance of treated pangus and tilapia finfish by weight**

| **Fish** | **Initial wt. (g)** | **Final wt. (g)** | | **Wt. gain** |
|---|---|---|---|---|
| Pangus | 4.74 | Control | 39.93(±0.75) | 35.45 |
| | | 0.2% | 53.61(±7.9) | 48.87 |
| | | 0.4% | 43.77(±2.7) | 38.97 |
| | | 0.6% | 45.14(±3.1) | 40.4 |
| Tilapia | 2.24 | Control | 86.6375(±6.99) | 84.3975 |
| | | 0.2% | 93.205(±7.4) | 90.965 |
| | | 0.4% | 106.4125(±17.9) | 104.1725 |
| | | 0.6% | 89.78(±10.7) | 87.54 |

In prawn, the best length gain performance was found in 0.6% extract treated fish (4.27 cm) than control fish (2.85 cm) where average length gain of 0.6% extract treated fish was greater than control fish. Not only 0.6% extract treated fish but also all treated fish has higher growth performance than control. A summary of the weight gain performance of prawn is shown in Table 43.

**Table 43. Growth performance of treated prawn by length**

| **Initial length (cm)** | **Final length (cm)** | | **Length gain** |
|---|---|---|---|
| 2 | Control | 4.85(±18.7) | 2.85 |
| | 0.2% | 4.99(±0.28) | 2.99 |
| | 0.4% | 5.22(±0.47) | 3.22 |
| | 0.6% | 6.27(±0.78) | 4.27 |

### Improved FCR

**Table 44. FCR performance of treated fish**

| **Fish** | **Treatment Group** | | | |
|---|---|---|---|---|
| | T₀ - Control | T₁ - 0.2% | T₂ - 0.4% | T₃ - 0.6% |
| Pangus | 2.00 | 1.49 | 1.83 | 1.77 |
| Tilapia | 2.00 | 1.85 | 1.63 | 1.93 |
| Prawn | 2.00 | 1.85 | 1.86 | 1.15 |

In pangus, the best FCR (1.49) was found in the 0.2% treatment group (T₁), whereas the FCR value of 0.4% and 0.6% treated fish (T₂ and T₃) was respectively1.83 and 1.77. All treated fish showed better FCR performance than control fish (2.004).

In tilapia, the best FCR (1.63) was found in 0.4% treated feed, whereas the FCR value of 0.2% and 0.6% treated fish was respectively 1.854 and 1.93. All treated fish showed better FCR performance than the fish in the control group (FCR was calculated to be 2.00).

In prawn, the best FCR (1.15) was found in 0.6% treated feed, whereas the FCR value of 0.2% and 0.4% treated prawn was respectively1.85 and 1.83. All treated prawn showed better FCR value than the prawn in the control group.

### Taste

Odour, flavour and taste experiments were performed by some panelists immediately after completing field research. Panelists declared that the treated fish were tastier than the control fish and the treated fish had less odour.

### Palatability /acceptance of feed

The feed containing an extract derived from sugar cane of the present disclosure was found to have little odour and attracted fish enhancing feed intake. All fish were attracted to the feed fighting to consume the feed of the treatment groups T₁, T₂ and T₃ than the control group (Tₒ).

### Improved protein content

Proximate composition analysis was undertaken for the various treatment groups in the pangus, tilapia and prawn experiments and the results are shown in Table 45. All treated fish showed greater protein content than the control.

**Table 45. Proximate composition analysis**

| **Protein percentage** | Control (%) | 0.2 %(%) | 0.4 %(%) | 0.6 %(%) |
|---|---|---|---|---|
| Pangus | 9.84 | 15.46 | 19.64 | 11.34 |
| Tilapia | 10.37 | 21.37 | 19.65 | 17.5 |
| Prawn | 8.88 | 15.54 | 17.71 | 14.45 |

| **Moisture content** | | | | |
|---|---|---|---|---|
| Pangus | 88.07 | 81.64 | 79.61 | 78.77 |
| Tilapia | 77.85 | 79.57 | 78.95 | 80.33 |

| **Ash content** | | | | |
|---|---|---|---|---|
| Pangus | 0.87 | 1.28 | 1.31 | 1.44 |
| Tilapia | 1.07 | 1.57 | 1.64 | 1.88 |

| **Lipid content** | | | | |
|---|---|---|---|---|
| Pangus | 1.33 | 1.28 | | |
| Tilapia | 1.63 | 1.25 | | |

### Example 14. Chicken study

### Rationale

Corn is one of the most common cereal grains to include in the diets for broiler chickens. It has been reported to have rapidly digestible starch (Giuberti *et al.,* 2012. Liu and Selle, 2015 suggest that feed conversion efficiency may be improved by slowly digestible starch and rapidly digestible protein.

### Objective

The purpose of the study was to investigate the effect of an extract derived from sugar cane of the present disclosure (extract of Example 4) in the diets of broiler chickens. The extract was included in the diets in amounts of 0, 0.5, 2, and 4 % i.e. inclusion rates of 0, 0.5, 2, and 4 %. The effects of including a sugar cane extract of Example 4 on growth performance was examined. In terms of growth performance, body weight gain in view of feed intake of broiler chickens was examined.

### Experimental Design

This study was conceived as with corn as an example of a dietary cereal type and 4 inclusion rates of the polyphenolic sugar cane extract of Example 4 (0, 0.5, 2 and 4%) giving a total of 8 experimental groups. The statistical unit for growth performance and was the cage, with each cage containing 5 birds. Six replicate cages per treatment group were used, giving a total of 240 birds (8 treatments x 6 cages x 5 birds). Each of the four dietary treatments was offered from 1-35 days post-hatch as "starter" (1-14 days) and "finisher" diets (15-35 days). The starter and finisher basal diets were formulated to commercial specifications.

### Statistics

The experimental units were pooled cage means and differences were considered significant at P < 0.05 by Students' t-test. The experimental groups are shown in Table 45 and dietary specifications in Table 46.

**Table 45. Experimental groups**

| **Diet** | **Stage*** | **Cereal Type** | **Inclusion Rate** |
|---|---|---|---|
| A | Starter | Corn-based diet | 0% |
| | Finisher | | |
| B | Starter | Corn-based diet | 0.5% |
| | Finisher | | |
| C | Starter | Corn-based diet | 2% |
| D | Starter | Corn-based diet | 4% |

| | | | |
|---|---|---|---|
| *Starter (day of hatch to 14 days old); Finisher (14 days to 42 days) | | | |

**Table 46. Typical dietary compositions and nutrient specifications for the starter phases**

| **Ingredient (kg/t)** | **Starter diet** | **Finisher Diet** |
|---|---|---|
| | (1-14 days post-hatch) | (15-37 days post-hatch) |
| | Per 1000kg | Per 1000kg |
| Wheat / Corn | 599 | 705 |
| Soybean meal | 290 | 159 |
| Canola meal | 31.5 | 48 |
| Vegetable oil | 36 | 45 |
| Limestone | 2.5 | 3.7 |
| Dicalcium phosphate | 27 | 25 |
| Salt | 1.5 | 1.3 |
| Sodium bicarbonate | 5.25 | 4.6 |
| Lysine HCl | 2.5 | 3.25 |
| Methionine | 2.75 | 2.4 |
| Threonine | 0.75 | 1.5 |
| TMV premix | 1.25 | 1.25 |
| Extract level (0 - 1.2%) | TBC | TBC |
| Total | 1000 | 1000 |

As would be recognised, final dietary composition may change, but will be generally representative of a commercial broiler starter and finisher diet. In diets, the sugar cane extract was included at the expense of corn starch.

### Results and comments

The study incorporated experimental diets comprised of corn and 4 levels of a sugar cane extract of Example 4; 0, 0.5, 2 and 4% giving a total of 4 experimental groups. The data were analysed has been presented to show the effect of the extract of Example 4 in a corn/maize diet.

### Growth performance

Growth performance over the duration of the study was found to be equal to or in excess of industry targets suggested for the Ross 308 broiler chicken (http://en.aviagen.com/assets/Tech Center/Ross Broiler/Ross-308-Broiler-PO-2014-EN.pdf ). Although there appeared to be little effect on average daily gain during the initial exposure period (day 10-17) and then days 17-24 (Figure 30 A and Figure 30 B) a significant increase in average daily gain was observed in the experimental group C (2% of extract in diet, Figure 31 A) over days 24-38. Overall, a significant weight gain at day 38 was observed for group C (Figure 31 B). These results came without a significant change in intake as shown in Figure 31 C (days 24-38).

### Mortality

**Table 47. Effect of sugar cane extract (and cereal type) on broiler mortality**

| **Treatment group** | **Mortality (%)** |
|---|---|
| Maize diet 0% extract | 1.7 |
| Maize diet 0.5% extract | 1.7 |
| Maize diet 2% extract | 1.6 |
| Maize diet 4% extract | 1.7 |
| SEM | 0.5 |
| Significance (P) | P>0.1 |

There was no adverse effect of the extract on broiler mortality over the entire growth period even up to a 4% inclusion rate. Mortality level was in line with industry targets.

### Summary

The effect of the sugarcane extract of Example 4 at 0.5, 2 and 4% in a corn/maize diet was studied as this diet types reflects a common grain used in intensive broiler meat production. The feeding trial was completed without any deviations from projected performance objectives. The birds grew at or exceeded industry targets recommended by the Ross 308 hybrid company. Mortality was on average 2% which is in line with production targets.

There was little effect of sugarcane extract inclusion rate on body weight gain during the initial exposure period (day 10-17) and then days 17-24. However, there were promising improvements in body weight gain at days 24-38 with the experimental group containing 2% extract. This suggests either that the birds initially required an adaptation to the presence of sugarcane extract in the diet (as would be expected) and that when the gastrointestinal tract matured, birds responded to the benefits of the sugar cane extract. The improvement in body weight gain came without a significant change in intake.

### Example 15. Cat study

### Summary

A study was conducted to test the effects of feeding an extract derived from sugar cane of the present disclosure (extract of Example 3) on body fat and health parameters in the domestic cat (*Felis catus*)*.* Sixteen cats (4 years of age) were fed with a control diet (a commercially available dry diet plus carrier (chicken stock)) or the control diet coated in 2% (w/w) of the extract. The cats were fed the diets for a total of 30 weeks in a cross over design (Period 1; 1-18 weeks and Period 2: 19-30 weeks). Feeding an extract derived from sugar cane of the present disclosure has no negative effects on blood or urinary health measures. Cats fed the sugar cane extract had a lower (P=0.02) metabolisable energy intake compared to cats for the Control diet. Additionally carbohydrate digestibility was reduced (P<0.001) in cats fed on the extract. This may explain the reduction in bodyweight and body fats that were observed in cats fed an extract derived from sugar cane of the present disclosure.

### Study objective

The objective of the study was to determine whether an extract derived from sugar cane of the present disclosure (extract of Example 3) prevents body fat gain associated with the consumption of high carbohydrate diets in domestic cats.

### Methodology

### Animals

The protocol for this study was approved by the Massey University Animal Ethics Committee (MUEAC # 15/(amended 07/10)). All cats were housed at the Centre for Feline Nutrition (Massey University, Palmerston North; New Zealand) according to the Animal Welfare (Companion Cats) Code of Welfare (2007). Prior to the study, all cats had complete blood counts and thyroid assessment to ensure each cat was clinically and physiologically normal.

Two groups (n=8) of adult (4 years of age) mixed-sex neutered cats (4 neutered male, 4 neutered female) (balanced for sex, initial bodyweight (BW), and body condition score) were housed and ad-lib fed dry isocaloric diets with (2%; sugar cane extract + carrier) or without (carrier alone; Control) the sugar cane extract of Example 3 for 18 weeks. After the week 18 sample, some cats were switched to the other diet (see Figure 32) for a further 12 weeks so that the effects of the sugar cane extract on reducing body fat could be ascertained.

### Diets

A commercially available adult formula dry diet was used as the basal diet. The macronutrient content of the diet is outlined in Table 48. The diet came in batches of 9000 g. For each batch of diet, half the batch was used to make the test diet and the remaining half was fed as the control diet. This ensured that any batch to batch variation in macronutrient content was minimised across the two treatment groups over the 30-week study.

**Table 48. Macronutrient content of the Control diet**

| **Macronutrient** | **Content** |
|---|---|
| ME (metabolisable energy) protein | 26 % |
| ME fat | 44 % |
| ME carbohydrate | 455 mg/kJ |
| Calcium | 28 % |
| Phosphorus | 455 mg/kJ |
| Potassium | 359 mg/kJ |
| Sodium | 172 mg/kJ |
| Chloride | - |
| Magnesium | 36 mg/kJ |
| Energy as fed | 16.9 kJ/gram |
| Energy as fed | 4052 kcal/g |

To make the Control diet, 121 g of warm (25°C) chicken stock (as a carrier, see Table 49) was added to 4535 g of the basal diet and mixed using a feed mixer. To make the 2% sugar cane extract diet, 91 g of an extract derived from sugar cane of the present disclosure was dissolved in 121 g of warm (25°C) chicken stock (as a carrier, see Table 33). The solution was added to 4535 g of the basal diet and mixed using a feed mixer. The diets were dried at room temperature (25°C) for 24 hr (until dry to touch) and stored in airtight containers until fed to the cats.

**Table 49. Macronutrient profile of the carrier**

| **Nutrient** | **Quantity per 100 mL** |
|---|---|
| Energy | 4.3 kJ |
| Protein | 1.1 g |
| Fat (total) | 0.1 g |
| Fat (saturated) | 0.1 g |
| Carbohydrate (total) | 1.4 g |
| Carbohydrate (sugars) | 1.4 g |
| Sodium | 477 mg |

### Health parameters

Before the study commenced (week 0), background blood samples (2 mmL) were taken for Complete Blood Count analysis (CBC; haematocrit, haemoglobin, red corpuscle content, mean corpuscle volume, mean corpuscular haemoglobin, mean corpuscular haemoglobin concentration, platelets, white cell count), to ascertain basal levels of animal health markers. CBC analysis was repeated on weeks 4, 9, 18, 24 and 30 of the study.

### Feed intake

During the 30-week study the animals were group-housed according to treatment diet. Daily food offered and refusals were measured for the group, and individual cat BW determined weekly.

At five time points during the study (weeks 0, 4, 9, 18, 24 and 30), daily feed intake was determined for each individual animal for one week, with faeces and urine collected daily. Apparent digestibility of the macronutrients (carbohydrate, protein, fat and energy) for each diet were determined using standard digestibility protocols. Briefly, individual food intake and refusals were recorded daily and total urine and faecal output collected over a 5-day period. Faecal samples were frozen (-20°C), freeze dried and ground for analysis.

The diets were analysed for percentage moisture using a convection oven at 105°C (AOAC 930.15, 925.10), percentage ash using a furnace at 550°C (AOAC 942.05), percentage protein using the leco total combustion method (AOAC 968.06), percentage fat using acid hydrolysis/Mojonnier extraction (AOAC 954.02), gross energy (kJ/g) using bomb calorimetry, percentage crude fibre using gravimetric method (AOAC 978.10 animal feed) and percentage carbohydrate by difference.

### Metabolite profiling

Urine samples (250 µL) were collected from the tray within 1 hr of excretion, snap frozen in liquid N and stored at -85°C until analysis. Urine samples (4 µL urine in 200 µL 0.1% Formic Acid) were analysed by reverse-phase UHPLC-MS methods, and mass spectral ions indicative of changes in metabolic processes were selected and monitored. These candidate ions were then further characterised/identified within a limited number of samples by standard LC with targeted MS-MSn. Candidate selection was also based on the elimination of adduct ions, isotopologues, weak intensity peaks or difficult candidates (multiple charged and/or high mass candidates).

### Body composition

### Deuterium oxide (D₂O)

On weeks 0, 4, 9, 18, 24 and 30, body composition was determined using an injection of deuterium oxide (D2O) into the jugular vein of the animal (Backus *et al.,* 2000). Briefly, the cats were injected with 0.4g/kg BW dose of D2O (Backus *et al.,* 2000). Food and water was withheld 24 hr beforehand. A baseline blood sample (day 0) was taken by jugular venepuncture prior to the intravenous injection with the labelled water. Blood samples (2 mL) were collected 4 hr post injection, and plasma was separated and frozen at -20°C until analysis. The enrichment of ²H in water was determined by transfer of the hydrogen in water to acetylene and subsequent analysis of the acetylene isotopes by IRMS (Van Kreel *et al.,* 1996). This method was adapted and modified by converting the acetylene eluting from the gas chromatograph (GC) to hydrogen and analysing the resulting hydrogen. Briefly, 350 mg granulated calcium carbide (Sigma) was transferred into a 12 mL exetainer (Labco, UK), sealed with a cap and septa, and evacuated. Plasma (20 µL) was injected through the septa onto the bed of calcium carbide and allowed to react at room temperature for a minimum of 30 min before analysis on a GC-TC-IRMS. The determination of ²H enrichment for the headspace acetylene derived from plasma was carried out with a Thermo Finnigan Delta V Plus continuous flow isotope ratio mass spectrometer (Thermo-Finnigan, Bremen, Germany) coupled online with a Thermo Trace GC via a Thermo Conflow III combustion interface (a high temperature pyrolysis furnace at 1450°C). Acetylene eluting from the GC column was pyrolysed to H₂. After drying the gas stream by a nafion membrane, gases were introduced into the IRMS ion source. A capillary column (Agilent Poraplot Q, 30 m x 0.32 mm ID) with helium as carrier gas (1.5 mL/min) was used for the separation of acetylene from air components. 15 µL was injected in the split mode (1:20 spilt ratio) by an autosampler (CTC A200S; CTC analytics, Zwingen, Switzerland) fitted with a 100 µL headspace syringe. The column head pressure was 150 kPa and injector temperature 110°C. The GC oven temperature was maintained isothermally at 110°C for the duration of the run. Data processing was performed by the vendor provided software, ISODAT. For purposes of IRMS calibration, mixtures of D₂O and unlabelled H₂O, between 0 and 5 mg of D₂O per mL H₂O, were prepared and analysed in analogous fashion to the plasma samples.

### Dual energy X-ray absorptiometry methodology

On weeks 0, 4, 9, 18, 24 and 30, body composition was also determined using dual energy X-ray absorptiometry (DEXA; Speakman *et al.,* 2001). Following the 4 hr blood sample for the D2O study, the cats were sedated using Medetomidine (10 µg/kg BW; 1 mg/mL) by a qualified veterinarian. The cats were placed on their side, and scanned using 'whole body' and 'infant whole body' settings on a Discovery A (S/N 82414) DEXA (HOLOGIC^{®}, Denver, Colorado, USA). Immediately after scanning, the cats were given Butorphanol (100 µg/kg BW; 10 mg/mL) to reverse the effects of the sedative, and monitored for 2 hr. Once the cat was alert, it was offered food and water according to its dietary allocation.

### Calculations

The digestibility co-efficients of dry matter, energy, fat, carbohydrate and protein were determined using: % digestibility = [(content in diet - content in faeces)/content in diet] × 100 (Wichert *et al.,* 2009). Metabolisable energy intake (MEI) was calculated by correcting gross energy (determined via bomb calorimetry) content of the diet by energy digestibility and crude protein content. Body fat was calculated using an isotope wash out method (Backus *et al.,* 2001).

### Statistics

All parameters were analysed using a Linear Mixed Model of a REML variance components analysis (GenStat v12). Period was defined as diet consumed in Period 1 (weeks 4, 9 and 18) or Period 2 (weeks 24 and 30). The model included Period Treatment*Gender as fixed parameters and Cat*Period as random effects. Week 0 was used as a covariate for all analyses. Data are reported as mean and standard error of difference (SED). Probabilities lower than 0.05 were considered significant, and values between 0.05 and 0.10 a trend. Two cats were omitted from the week 30 analysis due to abnormally low intakes (less than 40% of the previous measurement).

### Results and Discussion

### Effects of sugar cane extract on blood and urinary health parameters

Blood health parameters were assessed in the cats at weeks 0, 4, 9, 18, 24 and 30 weeks of the study using a CBC. A CBC test measures the following: the number of red blood cells (RBC), the number of white blood cells (WBC), the total amount of haemoglobin in the blood, the fraction of the blood composed of red blood cells (haematocrit). The CBC test also provides information about the following measurements: average red blood cell size (MCV), haemoglobin amount per red blood cell (MCH), the amount of haemoglobin relative to the size of the cell (haemoglobin concentration) per red blood cell (MCHC). The test can reveal problems with RBC production and destruction, or help diagnose malnutrition, kidney disease, dehydration, infection, allergies, and problems with blood clotting. MCV, MCH, and MCHC values reflect the size and haemoglobin concentration of individual cells, and are useful in diagnosing different types of anaemia.

All health parameters at all points during the study were within normal range. As indicated in Figure 33 and Figure 34, there was no negative effect of feeding the compound on health parameters. While the levels of both MCH (P=0.03) and MCHC (P=0.05) were increased in the cats fed the sugar cane extract, the values were well within normal ranges (13-18 pg and 290-360 g/L, for MCH and MCHC, respectively). Therefore, feeding an extract derived from sugar cane of the present disclosure does not negatively affect cat health.

The effects of feeding the sugar cane extract on urinary markers of cat health are shown in Figure 35 A and B. The extract increased urinary pH in cats (P=0.002). Cats need acidic urine for urinary tract health. Although the higher range may vary under certain circumstances, the expert consensus of a healthy range seems to be from 6.0 to 6.5. A pH higher than 6.5 can lead to the growth of struvites (magnesium ammonium phosphate crystals). A pH lower than 6.0 can cause the formation of calcium oxalate crystals. In the current study, urine pH values were within normal range for both diets.

There was no effect of feeding sugar cane extract on urine specific gravity. Specific gravity is an important measure of how well the urine is being concentrated by the cat's kidneys, and therefore, how well the kidneys are actually functioning as filters. The normal range for specific gravity is between 1.015 and 1.060, but only concentrations higher than about 1.030 can be considered solid evidence of normal kidney function. Therefore, it appears that feeding an extract derived from sugar cane of the present disclosure does not negatively affect urinary health in the domestic cat.

### Effects of sugar cane extract on food intake and digestibility

The macronutrient profiles of the control and test diet were determined from proximal analysis and are shown in Table 50. The following abbreviations are used: dry matter (DM); gross energy (GE); crude fibre (CF).

**Table 50. Macronutrient content of control and sugar cane extract diets fed to the domestic cat subject**

| **Diet** | **DM (%)** | **Ash (%)** | **Protein** | **GE** | **Fat (%)** | **CF** |
|---|---|---|---|---|---|---|
| Control | 91.9 | 4.9 | 31.5 | 21.4 | 21.0 | 1.3 |
| Sugar cane | 91.1 | 4.8 | 31.0 | 21.2 | 20.4 | 1.2 |

Total daily metabolisable energy intake (kcal/day) was reduced (P<0.001) in cats fed the sugar cane extract (Table 51). When metabolisable energy (ME) intake was expressed as a function of body weight (BW), which is commonly how energy requirements of cats are reported, it was reduced (P-value = 0.02) in the cats fed an extract derived from sugar cane of the present disclosure.

**Table 51. The effects of feeding of an extract derived from sugar cane of the present disclosure on daily dry matter and energy intake in the domestic cat**

| | **Control** | **Extract** | **SED** | **P-value** |
|---|---|---|---|---|
| ME intake (kcal/day) | 308.1 | 238.4 | 18.03 | <0.001 |
| ME intake (kcal/kg BW/day) | 68.97 | 59.41 | 3.96 | 0.02 |

The digestibility of macronutrients is shown in Figure 36. Sugar cane extract had no effect on the digestibility of protein (P=0.55) and fat (P=0.48). Energy digestibility tended (P=0.06) to be reduced in cats fed the extract as were the digestibility of dry matter (P=0.01) and carbohydrate (P<0.001).

### Effects of sugar cane on bodyweight and body composition

Bodyweight was maintained in the cats fed an extract derived from sugar cane of the present disclosure (extract of Example 3) while those fed the control diet gained weight (Figure 37B). Hence, there was a reduction in bodyweight in cats fed the extract of Example 3 relative to the control. In this regard, Figure 38 shows the results of the crossover in cats fed the control diet to a sugar cane extract diet based on the extract of Example 3. It was observed that cats that start on the control diet and then cross over to the sugar cane extract diet lose all the weight they gained in the first 18 weeks (the line marked "C-X" of Figure 38).

Body composition was assessed using both DEXA and deuterated water to assess lean mass and body fat levels. Cats fed the extract of Example 3 had a reduction in body fat level with respect to both fat % and fat mass (Figure 39 A and 39 B) and lean mass (Figure 37 A). Further, while there was little difference in energy intake observed between the sugar cane extract diet and the control diet (Figure 40) there is a significant reduction in body fat level as seen in Figure 41 which shows that cats fed the sugar cane extract diet had 29% less fat than cats fed the control diet.

### Conclusions

Cats fed an extract derived from sugar cane of the present disclosure had a reduction in body fat level with respect to both fat % and fat mass and lean mass. Bodyweight was reduced in the cats fed an extract derived from sugar cane of the present disclosure (Figure 39 B). The extract derived from sugar cane of the present disclosure had no negative impacts on blood or urinary markers of cat health. The digestibility of energy and carbohydrate were reduced by feeding the extract. Overall intake and metabolisable energy intakes were also reduced in the cats fed the extract, but within normal limits for adult cats indicating that the extract acts to increase satiety in the domestic cat.

### Example 16. Horse study

### Study objective

To evaluate an extract derived from sugar cane of the present disclosure in horses. The study design was multifaceted and involved evaluation of the following effects: nutrient digestibility and hindgut digestive function; stimulation or sustenance of appetite and weight gain or weight alterations; muscle condition such as muscle build, shape and the overall appearance of the horse subject.

### Methodology

The study had three components:

In a stable study, the study group comprised a group of horses not administered any anti-ulcer medication. These horses were feed daily with an extract derived from sugar cane of the present disclosure mixed into their feeds. Photographs of the horses before and after treatment were taken (this component is referred to below as the stable study).

A number of horse trainers, horsemen/horsewomen were asked to answer a questionnaire after 8 to 12 weeks of feeding horses with an extract derived from sugar cane of the present disclosure. The questionnaire dealt with the following issues: appetite; weight change; nutrient digestion and hindgut function (this component is referred to below as the questionnaire component).

A further case study was undertaken through testimony by a horse owner as to the effects of an extract derived from sugar cane of the present disclosure on horse health (this component is referred to below as the testimony component).

The sugar cane extract used in the trial was produced according to Example 5 and is set out below in Table 52.

**Table 52. Properties of sugar cane extract used in the horse trial**

| **Property** | **Hybrid sugar cane extract** |
|---|---|
| Brix | 70° (+/- 2) @ 20°C |
| pH | 4.6 (+/- 0.2) @ 20°C |
| Density | 1.35 (+/- 0.05 @ 20°C) |
| Colour Absorbance 420 | 90 - 120 |
| Absorbance 270 | 1900 - 2300 |
| Ratio A270/A420 | 15 - 30 |
| Total Polyphenol (mg/L as gallic acid equivalent) | Min 30,000 milligrams per litre (as gallic acid equivalents) |
| Total Flavonoid (mg/L as gallic acid equivalent) | Minimum 10,000 |
| Conductivity (uS/m) | 180,000 - 200,000 |
| Calcium (mg/kg) | 80 - 160 |
| Iron (mg/kg) | 2-8 |
| Magnesium (mg/kg) | 300 - 600 |
| Potassium (mg/kg) | 2000 - 4000 |
| Sodium (mg/kg) | 60 - 180 |
| Zinc (mg/100 g) | 1.5 - 3.0 |
| Selenium (mg/100 g) | 0.04 - 0.09 |
| Chromium (mg/100 g) | 0.015 - 0.50 |

### Results

### Stable Study

Results from the stable study are shown below in Table 53 Before and after photographs of appearance for horses A to E are shown in Figures 42 to 46.

**Table 53. Before and after weight measurements**

| **Horse** | **Weight before treatment (kg)** | **Weight after treatment (kg)** |
|---|---|---|
| A (Angel Eight) | 498.5 | 489 |
| B (Dashing Savanna) | 452 | 473 |
| C (Bullet Train) | 518 | 518 |
| D (Bon Rocket) | 506.5 | 487 |
| E (Best Hoffa) | 544 | 536 |
| F (Majestic Halo) | 446 | 479 |
| G (Mortified) | 520 | 502.5 |
| H (Admiral Michelle) | 497 | 496.5 |
| I (Teoflyte) | 450 | 465 |
| J (Haybale) | 549 | 551 |
| K (Capanello) | 497.5 | 469 |
| L (Classic Diva) | 490 | 453 |
| M (Electron) | 535 | 465 |
| N (Single Note) | 473 | 445 |
| O (Saveyourenthusiasm) | 486 | 497 |
| P (Terindah) | 509 | 504 |
| Q (Barleycove) | 436 | 467 |
| R (Believing) | 525 | 537 |
| S (Critical Rhythm) | 460 | 525 |
| T (Roman Fizz) | 548 | 530 |
| U (Milady Cabriot) | 499 | 490 |
| V (Perfect Command) | 520.5 | 511 |
| W (Streetshavenoname) | 507 | 495 |
| X (Sepoy/Bang On) | 412.5 | 481 |
| Y (BeneIla) | 475 | 43 |
| Z (Separee) | 505 | 443 |
| AA (So You Dream) | 515 | 520 |
| AB (Fast Stryke) | 556.5 | 502 |
| AC (Charlton) | 501.5 | 518 |

The photographs displayed in Figures 42 to 46 indicate that there is a beneficial improvement in horse appearance.

### Questionnaires

Questionnaire responses with respect to appetite; weight gain/loss; nutrient digestion and hindgut function received are shown below. Responses and comments are listed below in Table 54A and 54B.

**Table 54A. Questionnaire responses**

| | **Respondent** | | | |
|---|---|---|---|---|
| | A | B | C | D |
| Appetite | All improved | Improved appetite | All horses eating well before and during treatment | Greatly improved |
| Weight Gain/Loss | All maintained good weight gain work [*sic*] | Definitely gained weight | Normal weight fluctuations | Increased by approximately 20 kg |
| Digestion | No undigested grain; droppings improved | Normal | Very good no undigested grain | Definitely better; no undigested grain |

**Table 54B. Questionnaire responses**

| | **Respondent** | | | |
|---|---|---|---|---|
| | E | F | G | H |
| Appetite | Much improved after 10-12 days | Much improved | Good improvement in every horse within two days | Good improvement - cleaned up feed |
| Weight Gain/Loss | Increased 10 - 12 kg | All increasing between 9 - 40 kg | No [*sic*] measured but all held good condition through preparation | Variable but has put on 8-12 kg due to increased appetite |
| Digestion | No undigested grain | No undigested grain | No grain in dropping | No undigested grain |

The comments indicate that an extract derived from sugar cane of the present disclosure has a beneficial and significant effect on appetite. Virtually all of the respondents noted that appetite was stimulated or sustained in the horses. Further, a healthy weight was maintained. While one response indicated that normal weight fluctuations were observed, a number of responses indicated a significant weight gain was observed. Importantly, there was a positive effect on digestion/hindgut function. Most respondents remarked that undigested grain was not observed in droppings/faeces.

### Testimony

On treatment with an extract derived from sugar cane of the present disclosure the subject horse showed an increased top line due to improved muscle condition. Described as a fussy eater, the subject horse was found to eat all hard feed each night, despite having has a history of stomach ulceration. The horse was also observed to exhibit more energy.

### Summary

An extract derived from sugar of the present disclosure is able to improve or maintain horse health. Appetite is stimulated or sustained. Interestingly this effect on appetite was maintained in the presence of gastric ulcer syndrome ("GUS"). This is particularly relevant as one of the main clinical effects of GUS is diminished appetite. This then has a 'roll on' effect in that horse trainers generally modify and reduce training regimes when horses stop eating properly. In this study, treated horses maintained their appetite in spite of GUS and therefore there is not the need to modify husbandry practices.

### Example 17. Supplement comprising fiber

An extract derived from sugar cane of the present disclosure was developed into supplements containing fiber. The fiber was ground chia seeds which was coated onto the extract in an amount of 2% or 5% of the total weight of the supplement.

The chia fiber containing supplements are set out below in Table 55.

**Table 55. Chia fiber containing supplements**

| **Properties** | **Sugar cane extract** |
|---|---|
| Brix | 55° (+/- 2) @ 20°C |
| pH | 4.6 (+/- 0.2) @ 20°C |
| Density | 1.28 g/mL (+/- 0.05) @ 20°C |
| Colour Absorbance 420 | 190 - 280 |
| Absorbance 270 | 2300 - 3000 |
| Ratio A270/A420 | 10 - 15 |
| Total Polyphenol (mg/L as gallic acid equivalent) | Minimum 45,000 |
| Total Flavonoid (mg/L as gallic acid equivalent) | Minimum 10,000 |
| Conductivity (uS/m) | 250,000 - 350,000 |
| Calcium (mg/kg) | 3,000 - 4,000 |
| Iron (mg/kg) | 100 - 150 |
| Magnesium (mg/kg) | 3,000 - 5,000 |
| Potassium (mg/kg) | 30,000 - 40,000 |
| Sodium (mg/kg) | 2,000 - 3,000 |
| Zinc (mg/100 g) | 0.5 - 1.5 |
| Selenium (mg/100 g) | 0.02 - 0.05 |
| Chromium (mg/100 g) | 0.20 - 0.5 |
| Chia fiber coating | 2 or 5% w/w |

### Example 18. - Chicken study

### Study Objective

To evaluate an extract derived from sugar cane of the present disclosure in Ross308 broiler chickens. The study design was multifaceted and involved evaluation of the following effects under both normal growing conditions ("thermoneutral", TN) and under "heat stress" (HS) conditions:
- Growth performance (weight gain, feed consumption and feed conversion ratio)
- Meat quality (Warner Bratzler Shear Force, colour, drip loss, moisture content, lipid peroxidation, myofibrillar fragmentation index)

### Methodology

The broilers were received as one day old chickens from the hatchery. Half of the chickens were grown under TN conditions and the other under HS conditions (Figure 47). The broilers were fed standard industry based rations for starters, growers and finishers supplemented with 0, 2, 4, 6 and 10 g/kg extract derived from sugar cane of the present disclosure and 1 g/kg Betaine. The chickens were grown to a maximum of 42 days then electrically stunned and euthanized to determine product quality.

The broilers were fed wheat based diets matched to standard "starter" (0-14d), "grower" (15-28d) and finisher (29-end) formulations commonly used in the Australian poultry industry.

The respiration rate and rectal temperature were measured weekly from day 14 until the end of the experiment. On the penultimate day of the experiment a blood sample was collected and blood gas analysis performed.

Meat quality was determined by Warner Bratzler Shear Force (WBSF, instrumental measure of tenderness), myofibrillar fragmentation index (MFI, indirect measure of calpain mediated degradation of skeletal muscle fibres during meat tenderization), colour, lipid peroxidation Thiobarbituric acid reactive substances, TBARS) and water holding capacity (dry wt, drip and cook loss %).

### Results

### Growth Parameters

There was a linear increase in final body weight at day 35 in the extract derived from sugar cane of the present disclosure supplemented broilers (P=0.002, Figure 48) in both the TN and HS groups, with the inclusion level of 10 g/kg being the most effective (P=0.038). There was a tendency for broilers grown under HS conditions to be lighter (P=0.069), however no interaction between the sugar cane derived extract and HS was observed (Table 56).

No effects of sugar cane derived extract on feed intake were observed. However as the sugar cane derived extract increased body weight this resulted in a trend for an improved (lowered) feed conversion ratio (FCR, P=0.057 Figure 49) in both the TN and HS groups.

### Meat quality

The sugar cane derived extract improved meat tenderness, as assessed by the reduction in Warner Bratzler Shear Force (WBSF, Figure 50), with the 10 g/kg inclusion rate providing the best benefit. Heat stress increased WBSF, indicating tougher meat (21.0 vs 24.1 for TN and HS, P<0.001), however no interaction with the sugar cane derived extract was observed (P=0.26, Table 57). Proteolytic degradation of the myofibril post-mortem leads to the generation of myofibrillar fragments and is an important precursor to the meat tenderisation process. The myofibrillar fragmentation index (MFI) was not influenced by the sugar cane derived extract of the present disclosure. (P=0.17), however was lower in HS broilers which indicates reduced proteolytic activity post mortem (P=0.007, Table 57).

Lipid oxidation was quantified by the TBARS assay (Figure 51a (TN group) and Figure 51b (HS group)) and was significantly higher at 72 h in control meat samples than 24 h. Conversely, the TBARS levels did not increase between 24 and 72 h in the sugar cane derived extract supplemented muscle samples. This result indicates that the extract derived from sugar cane presently disclosed improved lipid stability, particularly at 72 h which is typically when consumers will be purchasing chicken meat.

### Summary

The sugar cane derived extract of the current disclosure was supplemented at 0 (control), 2, 4, 6 and 10 g/kg into Ross308 broilers from 1d to 35d. The principal findings were that the sugar cane derived extract increased the final body weight and tended to improve feed conversion ratio. Furthermore it improved product quality, resulting in more tender breast meat. Furthermore meat from the sugar cane derived extract of the present disclosure supplemented broilers showed improved lipid stability, which is an important determinant of shelf life. The most effective dose of the sugar cane derived extract was 10 g/kg.

| **Thermoneutral** | | | | | | **Heat Stress** | | | | | **SED** | **P-Value** | | | |
|---|---|---|---|---|---|---|---|---|---|---|---|---|---|---|---|
| | | | | | | | | | | | | **Diet** | **Temp.** | **D*T** | **Diet (lin)** |
| Level of extract (g/kg) | 0 | 2 | 4 | 6 | 10 | 0 | 2 | 4 | 6 | 10 | | | | | |
| Body wt (g) | 1928 | 1918 | 1994 | 1978 | 2157 | 1828 | 1915 | 1955 | 1943 | 1976 | 87.3 | 0.038 | 0.069 | 0.62 | 0.002 |
| Feed intake (g) | 3075 | 2994 | 3126 | 3062 | 3094 | 2747 | 3158 | 2977 | 2788 | 2914 | 178 | 0.61 | 0.066 | 0.36 | 0.9 |
| FCR | 1.61 | 1.56 | 1.58 | 1.55 | 1.43 | 1.51 | 1.66 | 1.58 | 1.43 | 1.5 | 0.099 | 0.24 | 0.75 | 0.44 | 0.057 |

**Table 57. Effects of heat stress ans sugar cane derived extract on meat quality parameters.**

| **Thermoneutral** | | | | | | | **Heat Stress** | | | | | **SED** | **P-Value** | | |
|---|---|---|---|---|---|---|---|---|---|---|---|---|---|---|---|
| | | | | | | | | | | | | | **Diet** | **Temp** | **D*T** |
| Level of extract (g/kg) | | 0 | 2 | 4 | 6 | 10 | 0 | 2 | 4 | 6 | 10 | | | | |
| WBSF (kg/cm²) | | 24.1 | 20.1 | 20.9 | 19.9 | 19.4 | 24.8 | 26.3 | 23.3 | 23.1 | 23.1 | 1.76 | 0.034 | <0.001 | 0.26 |
| MFI | | 82 | 113 | 70 | 106 | 98 | 66 | 69 | 60 | 86 | 69 | 19.5 | 0.17 | 0.007 | 0.75 |
| TBARS | 24 h | 1.38 | 1.66 | 2 | 2.13 | 1.49 | 2.11 | 1.57 | 4.19 | 2.08 | 2.88 | 0.813 | 0.029 | <0.001 | 0.102 |
| | 72 h | 3.7 | 2.37 | 1.68 | 1.96 | 1.73 | 5.04 | 4.07 | 4.34 | 2.19 | 2.98 | | | | |

### References

The Merck Veterinary Manual, 11th Edition, 2016, Merck (The Merck Veterinary Manual).
CRC Handbook of Food Drug and Cosmetic Excipients, 1st Ed., 1992, S. C. Smolinske, CRC Press (CRC Handbook of Food Drug and Cosmetic Excipients, 1992).
Maes, Michael, et al. "New drug targets in depression: inflammatory, cell-mediated immune, oxidative and nitrosative stress, mitochondrial, antioxidant, and neuroprogressive pathways. And new drug candidates-Nrf2 activators and GSK-3 inhibitors." Inflammopharmacology 20.3 (2012): 127-150 (Maes, M. et al. (2012)).
Tan, Sih Min, and Judy B. de Haan. "Combating oxidative stress in diabetic complications with Nrf2 activators: how much is too much?." Redox Report 19.3 (2014): 107-117 (Tan, S. M. and de Haan, J. B, 2014).
Hawkey, C. J. "COX-1 and COX-2 inhibitors." Best Practice & Research Clinical Gastroenterology 15.5 (2001): 801-820 (Hawkey, C. J, 2001).
Boxin Ou, Maureen Hampsch-Woodill, and Ronald L. Prior. "Development and validation of an improved oxygen radical absorbance capacity assay using fluorescein as the fluorescent probe." Journal of Agricultural and Food chemistry 49.10 (2001): 4619-4626 (Ou et al. 2001).
Dejian Huang, Boxin Ou, Maureen Hampsch-Woodill, Judith A. Flanagan, and Elizabeth K. Deemer "Development and validation of oxygen radical absorbance capacity assay for lipophilic antioxidants using randomly methylated β-cyclodextrin as the solubility enhancer." Journal of Agricultural and Food Chemistry 50.7 (2002): 1815-1821 (Huang et al. 2002).
Boxin Ou, Maureen Hampsch-Woodill, Judith Flanagan, Elizabeth K. Deemer, Ronald L. Prior, and Dejian Huang "Novel fluorometric assay for hydroxyl radical prevention capacity using fluorescein as the probe" Journal of Agricultural and Food Chemistry 50.10 (2002): 2772-2777 (Ou et al. 2002).
N. Joy Dubost, Boxin Ou, Robert B. Beelman "Quantification of polyphenols and ergothioneine in cultivated mushrooms and correlation to total antioxidant capacity." Food Chemistry 105.2 (2007): 727-735 (Dubost et al. 2007).
Liliang Zhang, Dejian Huang, Miwako Kondon, Ellen Fan, Hongping Ji, Yan Kou and Boxin Ou "Novel high-throughput assay for antioxidant capacity against superoxide anion." Journal of Agricultural and Food chemistry 57.7 (2009): 2661-2667 (Zhang et al. 2009).
Boxin Ou, Dejian Huang, and Maureen H. Woodill (Inventors) "Method for assaying the antioxidant capacity of a sample" U.S. Patent No. 7,132,296. (US 7,132,296).
Giuberti G, Gallo A, Cerioli C, Masoero F (2012) Animal Feed Science and Technology 174, 163-173 (Giuberti et al., 2012).
Liu SY, Selle PH (2015) Worlds Poultry Science Journal 71, 297-310 (Liu and Selle, 2015).
Backus RC, Havel PJ, Gingerich RL & Rogers QR (2000) Relationship between serum leptin immunoreactivity and body fat mass as estimated by use of a novel gas-phase Fourier transform infrared spectroscopy deuterium dilution method in cats. American Journal of Veterinary Research 61, 796-801 (Backus et al., 2000)*.*
Van Kreel BK, Van der Vegt F, Meers M, Wagenrnakers T, Westerterp K & Coward A (1996) (Van Kreel *et al.* 1996).
Speakman JR, Booles D & Butterwick R (2001) Validation of dual energy X-ray absorptiometry (Speakman et al., 2001)*.*
Wichert B, Schade L, Gebert S, Bucher B, Zottmaier B, Wenk C & Wanner M (2009) Energy and protein needs of cats for maintenance, gestation and lactation. Journal of Feline Medicine & Surgery 11, 808-815 (Wichert et al., 2009).
Backus RC, Ginzinger DG, Ashbourne Excoffon KJ, Clee SM, Hayden MR, Eckel RH, Hickman, MA & Rogers QR (2001) Maternal expression of functional lipoprotein lipase and effects on body fat mass and body condition scores of mature cats with lipoprotein lipase deficiency. American Journal of Veterinary Research 62, 264-269 (Backus et al., 2001)*.*
Brand, J., Cherikoff, V. Lee, A., & Truswell, A. S. (1982), An outstanding food source of vitamin C. The Lancet, 320(8303), 873 (Brand et al. 1982).
Tan, A. C., Konczak, I., Ramzan, I., Zabaras, D., & Sze, D. M. Y. (2011), Potential antioxidant, antiinflammatory, and proapoptotic anticancer activities of Kakadu plum and Illawarra plum polyphenolic fractions. Nutrition and cancer, 63(7), 1074-1084 (Tan et al. 2011).

## Claims

1. A non-human animal formulated feed supplement comprising an extract derived from sugar cane, the extract comprising from about 20 catechin equivalent (CE) g/L to about 50 CE g/L of polyphenols and about 1 CE g/L to about 15 CE g/L of flavonoids or from about 200 CE mg/g to about 500 CE mg/g of polyphenols and about 10 CE mg/g to about 150 CE mg/g of flavonoids.

2. The supplement according to claim 1, wherein the extract comprises from: about 3 CE g/L to about 10 CE g/L of flavonoids or from about 30 CE mg/g to about 100 CE mg/g of flavonoids.

3. The supplement according to claim 1 or claim 2, wherein the polyphenols comprise one or more of syringic acid, chlorogenic acid, caffeic acid, vanillin, sinaptic acid, p-coumaric acid, ferulic acid, gallic acid, vanillic acid, diosmin, diosmetin, apigenin, vitexin, orientin, homoorientin, swertisin, tricin, (+)catechin, (-)catechin gallate, (-)epicatechin, quercetin, kaempherol, myricetin, rutin, schaftoside, isoschaftoside, luteolin and scoparin.

4. The supplement according to any one of claims 1 to 3, wherein the polyphenols comprise syringic acid, chlorogenic acid and diosmin.

5. The supplement according to any one of claims 1 to 4, wherein the extract is in a form selected from the group comprising a mash, crumble, pellet, syrup, liquid and a powder which is optionally spray-dried, freeze-dried or drum-dried.

6. The supplement according to any one of claims 1 to 5, wherein the extract is derived from dunder and/or molasses.

7. A non-human animal feed comprising the supplement according to any one of claims 1 to 6 in an amount of between about 0.5 wt % and 20 wt% of the total weight of the feed.

8. The feed according to claim 7, wherein the supplement is present in the feed in an amount of between about 1 and 5 wt%.

9. The supplement according to any one of claims 1 to 6, or feed according to claim 7 or claim 8 for use in improving or maintaining gastrointestinal health in a non-human animal subject.

10. The supplement or the feed for use according to claim 9, wherein: the supplement or feed thereof has a beneficial immunomodulatory effect; the supplement or feed thereof has an anti-inflammatory effect; the supplement or feed thereof has an anti-oxidant effect; the supplement or feed thereof has a cytoprotective effect; or the supplement or feed thereof has an anti-microbial effect.

11. Non-therapeutic use of the supplement according to any one of claims 1 to 6, or feed according to claim 7 or claim 8 in
(i) improving the appeal and/or palatability of food;
(ii) improving growth performance in a non-human animal subject;
(iii) reducing body fat content in a non-human animal subject;
(iv) improving nutrient digestibility in a non-human animal subject;
(v) reducing feed conversion ratio (FCR) in a non-human animal subject;
(vi) improving meat quality in a non-human animal subject;
(vii) improving or maintaining muscle condition in a non-human animal subject.; or
(viii) stimulating or sustaining appetite in a non-human animal subject.

12. The supplement or the feed for use according to claim 9 or claim 10, or the use of the supplement or feed according to claim 11, wherein the non-human animal subject is selected from an aquatic animal, an insect, an amphibian, a reptile, a gastropod, a bird, a monogastric non-human animal, a ruminant and a pseudo-ruminant.

13. The supplement or the feed for use according to claim 12 or the use of the supplement or feed according to claim 12, wherein:
the aquatic animal is selected from the group comprising fish, finfish, pangus, tilapia, shellfish, a crustacean, crabs, crayfish, lobsters, prawns, shrimp, a mollusc, clams, mussels, oysters, scallops and winkles;
the bird is selected from the group comprising chickens, ducks, geese and turkeys;
the monogastric non-human animal is selected from the group comprising a rodent, cat, dog and pig;
the ruminant is selected from the group comprising cattle, sheep, goats and deer; or
the pseudo-ruminant is selected from the group comprising horses, rabbits and guinea pigs.

## Patentansprüche

1. Nichtmenschliches formuliertes Tierfutterergänzungsmittel, das einen Extrakt umfasst, der von Zuckerrohr abgeleitet ist, wobei der Extrakt etwa 20 g Catechinäquivalente (CE) / l bis etwa 50 g CE / l Polyphenole und etwa 1 g CE / l bis etwa 15 g / l Flavonoide oder etwa 200 mg CE / g bis etwa 500 mg CE / g Polyphenole und etwa 10 mg CE / g bis etwa 150 mg CE / g Flavonoide umfasst.

2. Ergänzungsmittel nach Anspruch 1, wobei der Extrakt etwa 3 g CE / l bis etwa 10 g CE / l Flavonoide oder etwa 30 mg CE / g bis etwa 100 mg CE / g Flavonoide umfasst.

3. Ergänzungsmittel nach Anspruch 1 oder Anspruch 2, wobei die Polyphenole eine oder mehrere unter Syringasäure, Chlorogensäure, Kaffeesäure, Vanillin, sinaptischer Säure, p-Cumarsäure, Ferulasäure, Gallussäure, Vanillinsäure, Diosmin, Diosmetin, Apigenin, Vitexin, Orientin, Homoorientin, Swertisin, Tricin, (+)Catechin, (-)Catechingallat, (-)Epichatechin, Quercetin, Kaempherol, Myricetin, Rutin, Schaftosid, Isoschaftosid, Luteolin und Scoparin umfassen.

4. Ergänzungsmittel nach einem der Ansprüche 1 bis 3, wobei die Polyphenole Syringasäure, Chlorogensäure und Diosmin umfassen.

5. Ergänzungsmittel nach einem der Ansprüche 1 bis 4, wobei der Extrakt in einer Form vorliegt, die aus der Gruppe ausgewählt ist umfassend einen Brei, Streusel, Kügelchen, Sirup, Flüssigkeit und ein Pulver, das wahlweise sprühgetrocknet, gefriergetrocknet oder trommelgetrocknet ist.

6. Ergänzungsmittel nach einem der Ansprüche 1 bis 5, wobei der Extrakt von Dunder und/oder Melasse abgeleitet ist.

7. Nichtmenschliches Tierfutter umfassend das Ergänzungsmittel nach einem der Ansprüche 1 bis 6 in einer Menge zwischen etwa 0,5 Gew.-% und 20 Gew.-% des Gesamtgewichts des Futters.

8. Futter nach Anspruch 7, wobei das Ergänzungsmittel in dem Futter in einer Menge zwischen etwa 1 und 5 Gew.-% vorliegt.

9. Ergänzungsmittel nach einem der Ansprüche 1 bis 6 oder Futter nach Anspruch 7 oder Anspruch 8 zur Verwendung zum Verbessern oder Aufrechterhalten gastrointestinaler Gesundheit bei einem nichtmenschlichen Tiersubjekt.

10. Ergänzungsmittel oder Futter zur Verwendung nach Anspruch 9, wobei das Ergänzungsmittel oder Futter davon eine günstige immunmodulatorische Wirkung aufweist, das Ergänzungsmittel oder Futter davon eine antiinflammatorische Wirkung aufweist, das Ergänzungsmittel oder Futter davon eine Antioxidanswirkung aufweist, das Ergänzungsmittel oder Futter davon eine zytoschützende Wirkung aufweist oder das Ergänzungsmittel oder Futter davon eine antimikrobielle Wirkung aufweist.

11. Nichttherapeutische Verwendung des Ergänzungsmittels nach einem der Ansprüche 1 bis 6 oder Futters nach Anspruch 7 oder Anspruch 8 zum
(i) Verbessern des Anreizes und/oder der Schmackhaftigkeit des Futters;
(ii) Verbessern der Wachstumsleistung bei einem nichtmenschlichen Tiersubjekt;
(iii) Reduzieren des Körperfettgehalts bei einem nichtmenschlichen Tiersubjekt;
(iv) Verbessern der Nährstoffverdaulichkeit bei einem nichtmenschlichen Tiersubjekt;
(v) Reduzieren des Futterumwandlungsverhältnisses (FUV) bei einem nichtmenschlichen Tiersubjekt;
(vi) Verbessern der Fleischqualität bei einem nichtmenschlichen Tiersubjekt;
(vii) Verbessern oder Aufrechterhalten des Muskelzustands bei einem nichtmenschlichen Tiersubjekt oder
(viii) Anregen oder Unterstützen des Appetits bei einem nichtmenschlichen Tiersubjekt.

12. Ergänzungsmittel oder Futter zur Verwendung nach Anspruch 9 oder Anspruch 10 oder Verwendung des Ergänzungsmittels oder Futters nach Anspruch 11, wobei das nichtmenschliche Tiersubjekt unter einem Wassertier, einem Insekt, einem Amphibium, einem Reptil, einem Bauchfüßler, einem Vogel, einem monogastrischen nichtmenschlichen Tier, einem Wiederkäuer und einem Pseudowiederkäuer ausgewählt ist.

13. Ergänzungsmittel oder Futter zur Verwendung nach Anspruch 12 oder Verwendung des Ergänzungsmittels oder des Futters nach Anspruch 12, wobei
das Wassertier aus der Gruppe ausgewählt ist umfassend Fische, Flossenfische, Pangus, Buntbarsche, Schalentiere, ein Krebstier, Krebse, Langusten, Hummer, Garnelen, Krabben, eine Molluske, Muscheln, Miesmuscheln, Austern, Jakobmuscheln und Strandschnecken;
der Vogel aus der Gruppe ausgewählt ist umfassend Hühner, Enten, Gänse und Truthähne;
das monogastrische nichtmenschliche Tier aus der Gruppe ausgewählt ist umfassend ein Nagetier, eine Katze, einen Hund und ein Schwein;
der Wiederkäuer aus der Gruppe ausgewählt ist umfassend Vieh, Schafe, Ziegen und Wild; oder
der Pseudowiederkäuer aus der Gruppe ausgewählt ist umfassend Pferde, Kaninchen und Meerschweinchen.

## Revendications

1. Complément alimentaire formulé pour animaux non humains comprenant un extrait dérivé de la canne à sucre, l'extrait comprenant d'environ 20 g d'équivalents de catéchine (EC)/L à environ 50 g EC/L de polyphénols et d'environ 1 g EC/L à environ 15 g EC/L de flavonoïdes ou d'environ 200 mg EC/g à environ 500 mg EC/g de polyphénols et d'environ 10 mg EC/g à environ 150 mg EC/g de flavonoïdes.

2. Complément selon la revendication 1, dans lequel l'extrait comprend de : environ 3 g EC/L à environ 10 g EC/L de flavonoïdes ou d'environ 30 mg EC/g à environ 100 mg EC/g de flavonoïdes.

3. Complément selon la revendication 1 ou la revendication 2, dans lequel les polyphénols comprennent un ou plusieurs parmi l'acide syringique, l'acide chlorogénique, l'acide caféique, la vanilline, l'acide sinaptique, l'acide p-coumarique, l'acide férulique, l'acide gallique, l'acide vanillique, la diosmine, la diosmétine, l'apigénine, la vitexine, l'orientine, l'homoorientine, la swertisine, la tricine, la (+)catéchine, le (-)gallate de catéchine, la (-)épicatéchine, la quercétine, le kaemphérol, la myricétine, la rutine, le schaftoside, l'isochaftoside, la lutéoline et la scoparine.

4. Complément selon l'une quelconque des revendications 1 à 3, dans lequel les polyphénols comprennent l'acide syringique, l'acide chlorogénique et la diosmine.

5. Complément selon l'une quelconque des revendications 1 à 4, dans lequel l'extrait est sous une forme choisie dans le groupe comprenant une purée, des miettes, un granulé, un sirop, un liquide et une poudre qui est éventuellement séchée par atomisation, lyophilisée ou séchée sur tambour.

6. Complément selon l'une quelconque des revendications 1 à 5, dans lequel l'extrait est dérivé de dunder et/ou de mélasses.

7. Aliment pour animaux non humains comprenant le complément selon l'une quelconque des revendications 1 à 6 en une quantité comprise entre environ 0,5 % en poids et 20 % en poids du poids total de l'aliment.

8. Aliment selon la revendication 7, dans lequel le complément est présent dans l'aliment en une quantité comprise entre environ 1 et 5 % en poids.

9. Complément selon l'une quelconque des revendications 1 à 6, ou aliment selon la revendication 7 ou la revendication 8 pour une utilisation pour améliorer ou maintenir la santé gastro-intestinale chez un sujet animal non humain.

10. Complément ou aliment pour une utilisation selon la revendication 9, dans lequel : le complément ou l'aliment de celui-ci a un effet immunomodulateur bénéfique ; le complément ou l'aliment de celui-ci a un effet anti-inflammatoire ; le complément ou l'aliment de celui-ci a un effet antioxydant ; le complément ou l'aliment de celui-ci a un effet cytoprotecteur ; ou le complément ou l'aliment de celui-ci a un effet antimicrobien.

11. Utilisation non thérapeutique du complément selon l'une quelconque des revendications 1 à 6, ou de l'aliment selon la revendication 7 ou la revendication 8 pour
(i) améliorer l'attrait et/ou l'appétence de la nourriture ;
(ii) améliorer les performances de croissance d'un sujet animal non humain ;
(iii) réduire la teneur en graisse corporelle d'un sujet animal non humain ;
(iv) améliorer la digestibilité des nutriments chez un sujet animal non humain ;
(v) réduire le taux de conversion alimentaire (FCR) chez un sujet animal non humain ;
(vi) améliorer la qualité de la viande d'un sujet animal non humain ;
(vii) améliorer ou maintenir la condition musculaire chez un sujet animal non humain ; ou
(viii) stimuler ou préserver l'appétit d'un sujet animal non humain.

12. Complément ou aliment pour une utilisation selon la revendication 9 ou la revendication 10, ou utilisation du complément ou de l'aliment selon la revendication 11, dans lequel/laquelle le sujet animal non humain est choisi parmi un animal aquatique, un insecte, un amphibien, un reptile, un gastéropode, un oiseau, un animal non humain monogastrique, un ruminant et un pseudo-ruminant.

13. Complément ou aliment pour une utilisation selon la revendication 12 ou utilisation du complément ou de l'aliment selon la revendication 12, dans lequel/laquelle :
l'animal aquatique est choisi dans le groupe comprenant les poissons, les poissons à nageoires, les pangasius, les tilapias, les coquillages, les crustacés, les crabes, les écrevisses, les homards, les crevettes, les crevettes grises, les mollusques, les palourdes, les moules, les huîtres, les coquilles Saint-Jacques et les bigorneaux ;
l'oiseau est choisi dans le groupe comprenant les poulets, les canards, les oies et les dindes ;
l'animal non humain monogastrique est choisi dans le groupe comprenant un rongeur, un chat, un chien et un porc ;
le ruminant est choisi dans le groupe comprenant les bovins, les ovins, les caprins et les cervidés ; ou
le pseudo-ruminant est choisi dans le groupe comprenant les chevaux, les lapins et les cobayes.
